(19) Europäisches Patentamt
European Patent Office
Office européen des brevets

(11) EP 0 424 248 B1

(12) **FASCICULE DE BREVET EUROPEEN**

(45) Date de publication et mention de la délivrance du brevet: **10.01.1996 Bulletin 1996/02**

(51) Int. Cl.[6]: **C07D 461/00**, C07D 491/22, A61K 31/435

(21) Numéro de dépôt: **90402898.2**

(22) Date de dépôt: **16.10.1990**

(54) **Nouveaux dérivés substitués en 15 de la 20,21-dinoréburnaménine, leur procédé de préparation et les nouveaux intermédiaires ainsi obtenus, leur application comme médicaments et les compositions les renfermant**

20,21-Dinoreburnaminderivate, substituiert in Position 15, Verfahren zu deren Herstellung und die auf diese Weise erhaltenen Zwischenverbindungen, deren Anwendung als Arzneimittel und diese enthaltende Zusammensetzungen

20,21-Dinoreburnamine derivatives, substituted in position 15, process for their preparation and novel intermediates so obtained, their use as medicines and compositions containing them

(84) Etats contractants désignés:
**AT BE CH DE DK ES FR GB GR IT LI LU NL SE**

(30) Priorité: **17.10.1989 FR 8913544**

(43) Date de publication de la demande:
**24.04.1991 Bulletin 1991/17**

(73) Titulaire: **ROUSSEL UCLAF**
**F-93230 Romainville (FR)**

(72) Inventeurs:
- **Clemence, François**
  **F-75009 Paris (FR)**
- **Haesslein, Jean-Luc**
  **F-93110 Rosny Sous Bois (FR)**
- **Oberlander, Claude**
  **F-75018 Paris (FR)**

(74) Mandataire: **Vieillefosse, Jean-Claude et al**
**F-93235 Romainville Cédex (FR)**

(56) Documents cités:

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.9.2/3.4

## Description

La présente invention concerne de nouveaux dérivés substitués en 15 de 20,21-dinoréburnaménine, leur procédé de préparation et les nouveaux intermédiaires ainsi obtenus, leur application code médicaments et les compositions les renfermant.

Le document EP-A-317427 décrit des dérivés substitués de 20,21-dinoréburnaménine possédant des activités nootropes, anti-dépressives, protectrices neuronales, anti-anoxiques, anti-ischémiques.

La présente invention a pour objet les produits de formule (I) :

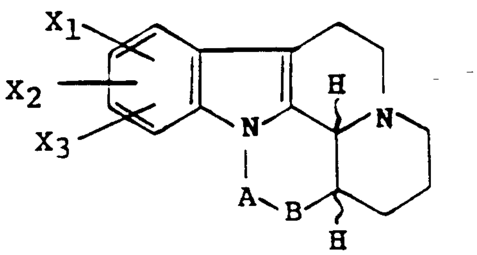

(I)

dans laquelle $X_1$, $X_2$ et $X_3$, identiques ou différents, représentent un atome d'hydrogène, un atome d'halogène, un radical alkyle, alkényle ou alkynyle, ces radicaux étant linéaires ou ramifiés renfermant au plus 18 atomes de carbone et éventuellement substitués, un radical alkyloxy linéaire ou ramifié renfermant au plus 7 atomes de carbone éventuellement substitué, un radical hydroxy, trifluorométhyle ou nitro, un radical amino, mono ou dialkylamino, ou un radical phényle éventuellement substitué, et dans laquelle

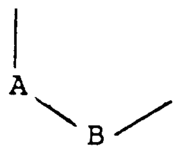

représente

A) soit le groupement

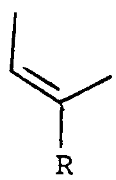

dans lequel R représente :

a) un radical alkyle, alkényle ou alkynyle linéaire ou ramifié renfermant au plus 7 atomes de carbone, ce radical étant éventuellement substitue,
b) un radical phényle éventuellement substitué,
c) un groupement carboxy libre, salifié ou estérifié,

B) soit le groupement

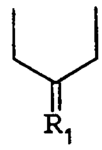

dans lequel $R_1$ représente l'un des deux groupements

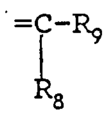 ou 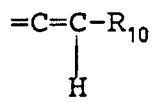

dans lesquels :
$R_8$ et $R_9$, identiques ou différents, sont tels que :

- l'un est choisi dans le groupe formé par :

  a) un atome d'hydrogène,
  b) un radical alkyle, alkényle ou alkynyle linéaire ou ramifié renfermant au plus 6 atomes de carbone, ce radical étant éventuellement substitué,
  c) un radical phényle éventuellement substitué,
  d) un radical carboxy estérifié,
  e) un radical cyano,
  f) un radical acyle ayant de 2 à 6 atomes de carbone,

- et l'autre est choisi dans le groupe formé par :

  a) un atome d'hydrogène,
  b) un radical alkyle, alkényle ou alkynyle linéaire ou ramifié renfermant au plus 6 atomes de carbone, ce radical étant éventuellement substitué,
  c) un radical phényle éventuellement substitué,

et $R_{10}$ représente :

a) un atome d'hydrogène,
b) un radical alkyle, alkényle ou alkynyle linéaire ou ramifié renfermant au plus 5 atomes de carbone, ce radical étant éventuellement substitué,
c) un radical phényle éventuellement substitué,

lesdits produits de formule (I) étant sous toutes les formes isomères possibles racémiques ou optiquement actives, ainsi que les sels d'addition avec les acides minéraux ou organiques ou avec les bases desdits produits de formule (I).

Dans les produits de formule (I), l'atome d'hydrogène en position 3 et l'atome d'hydrogène en position 16 peuvent chacun occuper l'une ou l'autre des orientations alpha et béta, ce qui détermine l'existence de diastéréoisomères cis et trans.

Dans les produits de formule (I) et dans ce qui suit :

- le terme atome d'halogène désigne de préférence l'atome de chlore ou de fluor, mais peut aussi représenter un atome de brome ou d'iode,
- le terme radical alkyle linéaire ou ramifié désigne de préférence les radicaux méthyle, éthyle, n-propyle ou isopropyle, mais peut également représenter un radical n-butyle, isobutyle, sec-butyle, t-butyle ou n-pentyle,
- le terme radical alkyloxy linéaire ou ramifié désigne de préférence les radicaux méthoxy ou éthoxy, mais peut aussi représenter un radical propoxy, isopropoxy, butoxy linéaire, secondaire ou tertiaire,

- le terme radical monoalkyl- ou dialkylamino désigne de préférence les radicaux monoalkyl- ou dialkylamino dans lesquels les radicaux alkyles linéaires ou ramifiés renferment de 1 à 5 atomes de carbone et en particulier des radicaux méthyle, éthyle ou isopropyle,
- le terme radical alkényle linéaire ou ramifié désigne de préférence un radical vinyle, allyle, 1-propényle, butényle ou pentényle,
- le terme radical alkynyle linéaire ou ramifié désigne de préférence un radical éthynyle, propargyle, butynyle ou pentynyle,
- le terme radical acyle ayant de 2 à 6 atomes de carbone désigne de préférence un radical acétyle, propionyle, butyryle ou benzoyle, mais également un radical valéryle, hexanoyle, acryloyle, crotonoyle ou carbamoyle,
- le terme carboxy estérifié désigne de préférence un groupe alkyloxy inférieur carbonyle tel que méthoxycarbonyle, éthoxycarbonyle ou benzyloxycarbonyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alkylmonosulfoniques tels que par exemple l'acide méthanesulfonique, l'acide éthanesulfonique, l'acide propanesulfonique, les acides alkyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

Lorsque R représente une fonction carboxy, elle peut être salifiée par un reste de base. On peut alors avoir un sel de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris (hydroxyméthyl) aminométhane, l'éthanolamine, la pyridine, la picoline, la dicyclohexylamine, la morpholine, la benzylamine, la procaïne, la lysine, l'arginine, l'histidine, la N-méthylglucamine.

Les radicaux alkyle, alkényle ou alkynyle peuvent ne pas être substitués ou porter un ou plusieurs substituants tels que par exemple les radicaux suivants : hydroxy ; aryle, par exemple phényle ou naphtyle ; arylalkyle, par exemple benzyle ou phénéthyle ; cycloalkyle, par exemple cyclopropyle, cyclopentyle ou cyclohexyle ; alkyloxy, par exemple méthoxy, éthoxy, propoxy ou isopropoxy comme dans par exemple les groupes méthoxyméthyle ou 1-éthoxyéthyle ; aryloxy, par exemple phénoxy ; (arylalkyl) oxy, par exemple benzyloxy ; mercapto ; alkylthio, par exemple méthylthio ou éthylthio ; arylthio ; aralkylthio ; amino comme dans, par exemple, le groupe 2-aminoéthyle ; amino substitué, par exemple méthylamino, éthylamino ou diméthylamino ; halogène, par exemple chloro ou bromo, comme dans, par exemple, le groupe 2-bromoéthyle ; nitro ; azido ; carbamoyle ; carbamoyle substitué par exemple un groupe N-monoalkyl inférieur carbamoyle, tel que N-méthylcarbamoyle, N-éthylcarbamoyle, un groupe N,N-dialkyl inférieur carbamoyle, tel que N,N-diméthylcarbamoyle, N,N-diéthylcarbamoyle ; un groupe N-(hydroxyalkyl inférieur) carbamoyle, tel que N-(hydroxyméthyl) carbamoyle, N-(hydroxyéthyl) carbamoyle, un groupe carbamoylalkyle inférieur, tel que carbamoylméthyle, carbamoyléthyle ; carboxy ; carboxy estérifié, par exemple méthoxycarbonyle ou éthoxycarbonyle ; formyle ; acyle, par exemple acétyle, propionyle ou benzoyle ; acyloxy, par exemple acétoxy ou propionyloxy ; cyano ; phtalimido ; acylamido, par exemple acétamido ou benzamido ; alkyloxycarbonylamino, par exemple méthoxycarbonylamino ou éthoxycarbonylamino ; ou (arylalkyl)oxycarbonylamino, par exemple benzyloxycarbonylamino.

Les radicaux aryles et arylalkyles peuvent ne pas être substitués ou porter un ou plusieurs substituants tels que par exemple les radicaux suivants : hydroxy ; halogène ; alkyle, par exemple méthyle, éthyle, isopropyle ou tert-butyle ; alkyloxy, par exemple méthoxy, éthoxy ou isopropoxy ; alkylthio, par exemple méthylthio ou éthylthio ; nitro ; amino ; amino substitué, tel que monoalkylamino et dialkylamino, par exemple méthylamino, éthylamino ou diméthylamino.

Lorsque $X_1$, $X_2$ ou $X_3$ représentent un radical alkyle ou alkyloxy substitué, ce radical peut être substitué de préférence par un ou plusieurs radicaux hydroxyle, carboxy libre ou estérifié, par exemple méthoxycarbonyle ou éthoxycarbonyle, et le radical alkyle peut également être substitué par un radical alkyloxy linéaire ou ramifié renfermant de 1 à 5 atomes de carbone, par exemple méthoxy, éthoxy ou isopropoxy.

L'invention a notamment pour objet les produits de formule (I) tels que définis ci-dessus, caractérisés en ce que $X_1$, $X_2$ et $X_3$ représentent un atome d'hydrogène, lesdits produits de formule (I) étant sous toutes les formes isomériques possibles racémiques ou optiquement actives, ainsi que les sels d'addition avec les acides minéraux ou organiques ou avec les bases desdits produits de formule (I).

L'invention a particulièrement pour objet les produits de formule (I) tels que définis ci-dessus, caractérisés en ce que le ou les substituants que peuvent porter les radicaux alkyle, alkyloxy, alkényle ou alkynyle sont choisis dans le groupe formé par :

- le radical hydroxy,
- les atomes d'halogène,
- les radicaux alkyloxy ayant de 1 à 6 atomes de carbone,
- les radicaux formyle et acyle ayant de 2 à 6 atomes de carbone et le radical benzoyle,

- les radicaux carboxy libres et estérifiés par un radical alkyle linéaire ou ramifié renfermant au plus 5 atomes de carbone,
- le radical cyano,
- le radical carbamoyle éventuellement substitué,
- le radical phényle éventuellement substitué,
- le radical

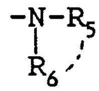

dans lequel :

**soit** $R_5$ et $R_6$, identiques ou différents, représentent :

- un atome d'hydrogène,
- un radical alkyle linéaire ou ramifié renfermant au plus 7 atomes de carbone éventuellement substitué par un ou plusieurs radicaux choisis dans le groupe formé par le radical hydroxy, les radicaux alkyloxy linéaires et ramifiés renfermant au plus 5 atomes de carbone et les radicaux carboxy libres ou estérifiés par un radical alkyle linéaire ou ramifié renfermant au plus 5 atomes de carbone,
- un radical aryle ou arylalkyle renfermant de 7 à 12 atomes de carbone, ces radicaux étant éventuellement substitués,

**soit** $R_5$ et $R_6$ forment ensemble avec l'atome d'azote auquel ils sont liés un hétérocycle de cinq à six chainons, renfermant éventuellement un second hétéroatome choisi parmi l'oxygène, le soufre ou l'azote, éventuellement substitué sur l'atome d'azote non lié à $R_5$ et $R_6$ par :

- un radical alkyle linéaire ou ramifié renfermant au plus 5 atomes de carbone éventuellement substitué par un radical hydroxy, un atome d'halogène ou un radical alkyloxy renfermant de 1 à 4 atomes de carbone,
- un radical aryle ou un radical arylalkyle renfermant de 7 à 12 atomes de carbone, ces radicaux étant éventuellement substitués, lesdits produits de formule (I) étant sous toutes les formes isomères possibles racémiques ou optiquement actives, ainsi que les sels d'addition avec les acides minéraux ou organiques desdits produits de formule (I).

L'invention a plus particulièrement pour objet les produits de formule (I) tels que définis ci-dessus, caractérisés en ce que le ou les substituants que peuvent porter les radicaux phényle, aryle et arylalkyle sont choisis dans le groupe formé par les atomes d'halogène, le radical hydroxy, les radicaux alkyle et alkyloxy linéaires et ramifiés renfermant au plus 5 atomes de carbone, les radicaux méthylthio, nitro, amino, monoalkyl- et dialkylamino, lesdits produits de formule (I) étant sous toutes les formes isomères possibles racémiques ou optiquement actives, ainsi que les sels d'addition avec les acides minéraux ou organiques desdits produits de formule (I).

Dans les produits de formule (I) et dans ce qui suit :

- le terme radical arylalkyle renfermant de 7 à 12 atomes de carbone désigne de préférence un radical benzyle ou phénéthyle et parmi les substituants éventuels des radicaux phényle, aryle et arylalkyle se trouvent de préférence les radicaux méthyle, éthyle, isopropyle et tert-butyle, méthoxy, éthoxy et propoxy,
- lorsque $R_5$ et $R_6$ forment ensemble avec l'atome d'azote auquel ils sont liés un hétérocycle, il s'agit, par exemple, d'un cycle pyrrolidino, pipéridino, morpholino, pipérazinyle, méthylpipérazinyle, éthylpipérazinyle, propylpipérazinyle, phénylpipérazinyle ou benzylpipérazinyle ; lorsque $R_5$ et $R_6$ représentent ces deux derniers radicaux, les radicaux phényle et benzyle peuvent être éventuellement substitués par les substituants déjà mentionnés précédemment pour aryle et arylalkyle.

Parmi les produits objet de l'invention, peuvent être cités tout particulièrement les produits décrits ci-après dans les exemples et notamment :

- l'oxalate de [16alpha,(±)]-15-(1-propynyl) 20,21-dinoréburnaménine,
- le [16alpha,(±)]-15-méthyl 20,21-dinoréburnaménine,
- le maléate acide de [16alpha,(±)]-14,15-dihydro 15-méthylène 20,21-dinoréburnaménine,
- le maléate de [16alpha,(±)] 20,21-dinoréburnaménine-15-acétate d'éthyle,
- le maléate acide de [16alpha,(±)] 20,21-dinoréburnaménine-15-méthanol,
- le maléate acide de [16alpha,(±)]-N,N-diméthyl 20,21-dinoréburnaménine-15-méthanamine.

L'invention a également pour objet un procédé de préparation des produits de formule (I) telle que définie ci-dessus, caractérisé en ce que l'on fait réagir un produit de formule (II) :

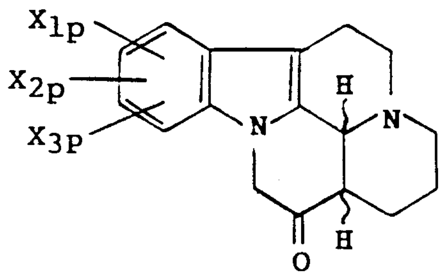

(II)

dans laquelle $X_{1p}$, $X_{2p}$ et $X_{3p}$ représentent $X_1$, $X_2$ et $X_3$ qui ont les significations indiquées ci-dessus, dans lesquelles les fonctions réactives sont éventuellement protégées,

A) soit avec un halogénure de formule (III) :

R'-V-Hal (III)

dans laquelle V représente un atome de magnésium ou de zinc et Hal représente un atome d'halogène, ou un organométallique de formule (IV) :

R'-W (IV)

dans laquelle W représente un atome de lithium, sodium ou potassium, formules (III) et (IV) dans lesquelles R' représente ou bien les valeurs indiquées ci-dessus pour R, sauf la valeur carboxy libre, salifié ou estérifié, ou bien lesdites valeurs de R dans lesquelles les fonctions réactives sont protégées, pour obtenir un composé de formule (V) :

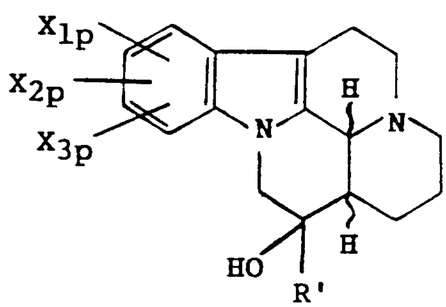

(V)

dans laquelle R' a la signification indiquée ci-dessus, que l'on soumet à une réaction de déshydratation et, si nécessaire, à une réaction d'élimination des groupements protecteurs des fonctions réactives que peuvent porter $X_{1p}$,

$X_{2p}$, $X_{3p}$ et R', pour obtenir un composé de formule ($Ia_1$) :

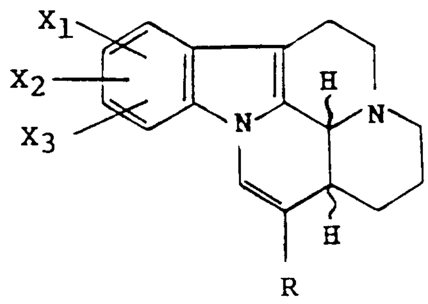

($Ia_1$)

dans laquelle $X_1$, $X_2$, $X_3$ et R ont les significations indiquées ci-dessus, produit de formule ($Ia_1$) que l'on soumet le cas échéant lorsque R représente un radical $-CH_2OH$, à l'action d'un agent d'oxydation pour obtenir un produit de formule ($Ia_2$) :

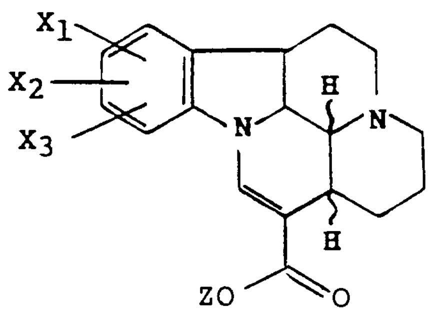

($Ia_2$)

dans laquelle $X_1$, $X_2$, $X_3$ ont les significations indiquées ci-dessus et Z représente un atome d'hydrogène ou le reste d'un groupement ester que l'on soumet si nécessaire ou si désiré à l'une ou aux deux réactions suivantes :

- hydrolyse du groupement ester,
- estérification ou salification par une base de la fonction carboxylique.

B) <u>soit</u> avec un dérivé de triphénylphosphorane de formule (VI) :

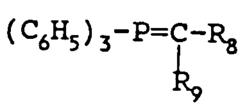

(VI)

ou un phosphonate de formule (VI') :

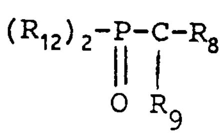

(VI')

dans laquelle $R_{12}$ représente un radical alkyloxy, formules (VI) et (VI') dans lesquelles $R_8$ et $R_9$ ont les significations indiquées ci-dessus, pour donner selon les conditions opératoires utilisées et éventuellement aprés élimination des

groupements protecteurs des fonctions réactives que peuvent porter $X_{1p}$, $X_{2p}$ et $X_{3p}$, un composé de formule ($Ib_1$) :

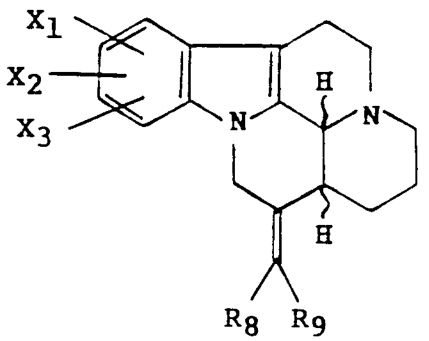

($Ib_1$)

ou un composé de formule ($Ia_3$) :

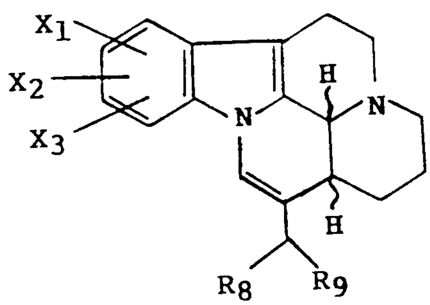

($Ia_3$)

dans lesquelles $X_1$, $X_2$, $X_3$, $R_8$ et $R_9$ ont les significations indiquées ci-dessus,
C) soit avec un dérivé activé du produit de formule (VII) :

$$R_{10}\text{-}C{\equiv}CH \qquad (VII)$$

dans laquelle $R_{10}$ a la signification donnée ci-dessus, pour obtenir un composé de formule (VIII) :

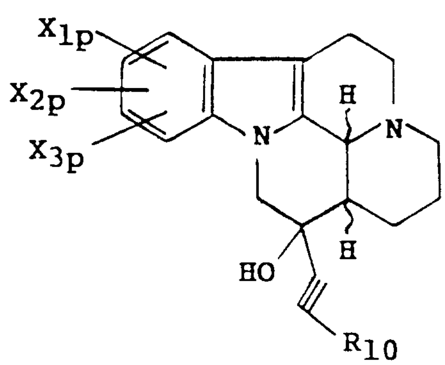

(VIII)

dans laquelle $X_{1p}$, $X_{2p}$, $X_{3p}$ et $R_{10}$ ont les significations indiquées ci-dessus, que l'on soumet, aprés activation du radical hydroxyle et éventuellement protection des fonctions réactives que peut comporter $R_{10}$, à une réaction de réduction, éventuellement d'une réaction d'élimination des groupements protecteurs des fonctions réactives protégées, pour obtenir un composé de formule ($Ib_2$) :

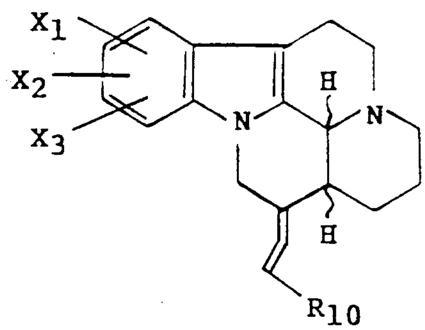

($Ib_2$)

dans laquelle $X_1$, $X_2$, $X_3$ et $R_{10}$ ont les significations indiquées ci-dessus, et traite, si désiré les produits de formules ($Ia_1$), ($Ia_3$), ($Ib_1$) et ($Ib_2$) par un acide minéral ou organique pour obtenir le sel correspondant, lesdits produits de formule (I) étant sous toutes les formes isoméres possibles racémiques ou énantiomères.

Dans des conditions préférentielles de mise en oeuvre de l'invention, le procédé ci-dessus est réalisé de la manière suivante :

- dans la réaction, telle que définie ci-dessus en A), la préparation des organomagnésiens ou zinciques de formule (III) et leur réaction avec un produit de formule (II) peuvent être réalisées dans les conditions habituelles connues de l'homme de métier.

L'atome d'halogène de l'halogénure de magnésium ou de zinc de formule (III) peut être un atome de brome comme par exemple dans le bromure de phénylmagnésium mais également un atome d'iode ou encore de chlore.

La préparation de l'halogénure de magnésium peut être réalisée, par exemple, par réaction du magnésium avec un halogénure organique dans un milieu inerte légèrement polaire tel que l'éther selon le procédé de préparation des halogénures organomagnésiens ou réactifs de Grignard.

La réaction du produit de formule (II) avec l'halogénure de magnésium de formule (III) pour obtenir des produits de formule (V), s'effectue préférentiellement dans un solvant organique tel que l'éther ou le tétrahydrofuranne à la température ambiante ou au reflux.

La préparation d'organométalliques de formule (IV) dans laquelle W représente un atome de lithium, de sodium ou de potassium et leur réaction avec un produit de formule (II) peuvent être réalisées dans les conditions habituelles connues de l'homme de métier.

La préparation de l'organolithien de formule (IV) peut être réalisée, par exemple, par réaction d'un halogénure organique avec une base telle que préférentiellement l'amidure de diisopropyllithium ou le butyllithium par exemple dans de l'éther éthylique ou du tétrahydrofuranne, à basse température.

La réaction du produit de formule (II) avec l'organolithien de formule (IV) peut s'effectuer, par exemple, dans du tétrahydrofuranne ou de l'éther à basse température de -70°C à -10°C ou encore dans le diméthoxyméthane.

La déshydratation du produit de formule (V) pour obtenir un produit de formule ($Ia_1$) s'opère dans un solvant organique qui est préférentiellement le toluène ou le xylène mais qui peut être aussi le tétrahydrofuranne en présence par exemple de $P_2O_5$ ou encore de sels de Burgess. Cette réaction de déshydratation peut également être réalisée par activation de l'alcool préférentiellement en mésylate par le chlorure de méthanesulfonyle suivie d'un traitement par une base forte telle que préférentiellement le diazabicycloundécène ou le diazabicyclononène.

L'oxydation du produit de formule ($Ia_1$) conduisant au produit de formule ($Ia_2$) est effectuée selon les méthodes usuelles en utilisant par exemple des sels de chrome, l'oxyde de sélénium ou encore en opérant selon la réaction de Swern.

L'hydrolyse du groupement ester, l'estérification ou la salification éventuelle du produit de formule ($Ia_2$) est effectuée selon les méthodes usuelles connues de l'homme de métier.

- Dans la réaction, telle que définie ci-dessus en B) 1), le triphénylphosphorane de formule (VI) est formé par réaction de triphénylphosphine avec l'halogénure correspondant dans lequel l'atome d'halogène est préférentiellement un atome de brome et conduisant à un sel de phosphonium sur lequel on fait agir une base telle que par exemple le terbutylate de potassium ou encore le butyllithium pour obtenir le produit de formule (VI) attendu. La réaction est effectuée dans un solvant organique tel que le tétrahydrofuranne ou encore l'éther à une température comprise entre 0°C et le reflux.

La réaction du produit de formule (II) avec le triphénylphosphorane de formule (VI) pour obtenir un produit de formule ($Ib_1$) est réalisée à une température comprise entre -70°C et 0°C.

La préparation du réactif de formule (VI) et sa réaction avec le produit de formule (II) sont réalisées selon les méthodes classiques.

La réaction du produit de formule (II) et du phosphonate de formule (VI') pour donner le produit de formule ($Ib_1$) est réalisée en présence d'hydrure de sodium ou encore d'une base faible telle que par exemple le carbonate de sodium ou de potassium dans un solvant tel que le tétrahydrofuranne.

Lorsqu'un mélange mole à mole du produit de formule (II) et du phosphonate de formule (VI') est utilisé, les composés recherchés de formule ($Ib_1$) seront majoritairement obtenus tandis qu'un excés d'ylure et un milieu basique conduisent majoritairement aux composés de formule ($Ia_3$).

Dans des conditions préférentielles de mise en oeuvre de la réaction, le dérivé phosphoré utilisé pour l'obtention de composés de formule ($Ib_1$) dans laquelle $R_8$ et $R_9$ représentent un radical alkyle ou phényle éventuellement substitué est un phosphorane.

Dans les cas où $R_8$ et $R_9$ comportent un ou plusieurs groupements ester, cyano ou acyle, le dérivé phosphoré utilisé est préférentiellement un phosphonate.

- Dans la réaction, telle que définie ci-dessus en C), du produit de formule (II) avec un dérivé acétylénique de formule (VII) pour obtenir un composé de formule (VIII), le dérivé acétylénique tel que par exemple l'acétylène ou le propyne est activé sous forme d'anion dans un milieu basique par exemple d'alcanoate de sodium ou de potassium tel que le terbutanoate de potassium ou d'une base lithiée telle que par exemple le butyllithium. La réaction s'effectue dans un solvant organique tel que par exemple le tétrahydrofuranne ou l'éther.
- Dans la réaction, telle que définie ci-dessus en C), la réduction du produit de formule (VIII) est réalisée avec un hydrure, notamment un hydrure mixte, tel que par exemple, l'hydrure mixte de lithium et d'aluminium ou le diéthylhydrure de sodium et d'aluminium. On peut également utiliser comme réactif le borohydrure de sodium ou de potassium ou encore le cyanoborohydrure de sodium en présence d'un alcool tel que par exemple le méthanol ou l'éthanol.

L'activation de la fonction hydroxyle du composé de formule (VIII) peut être réalisée par exemple par un halogénure tel que préférentiellement le chlorure de méthanesulfonyle dans un milieu basique par exemple de triéthylamine ou de pyridine dans un solvant organique tel que le tétrahydrofuranne ou le dichlorométhane.

La réaction de réduction du composé de formule (VIII) activé pour obtenir des composés de formule ($Ib_2$) est réalisée préférentiellement dans des conditions anhydres, par exemple avec un hydrogénure de lithium et d'aluminium dans du tétrahydrofuranne ou de l'éther.

Dans le cas où $R_{10}$ comporte une ou plusieurs fonctions carboxy estérifié, celles-ci peuvent, par la réduction du composé de formule (VIII) indiquée ci-dessus, être transformées en fonctions alcool, et dans ce cas ces fonctions alcools ainsi obtenues peuvent être, si désiré, éventuellement réoxydées pour redonner les fonctions carboxy estérifiées initiales : cette réoxydation peut être réalisée par exemple par l'oxyde chromique ou des sels de chrome tels que le pyridinium dichromate ou le pyridinium chlorochromate dans un solvant tel que par exemple le dichlorométhane ou la diméthylformamide.

L'invention a aussi pour objet un procédé de préparation des produits de formule (I) telle que définie ci-dessus et dans laquelle :

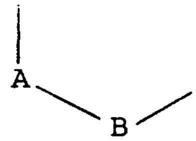

représente le groupement

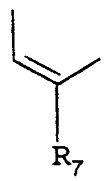

dans lequel $R_7$ représente un radical méthyle éventuellement substitué par un radical hydroxy par un atome d'halogène ou par un radical

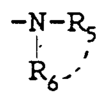

dans lequel $R_5$ et $R_6$ ont la signification indiquée ci-dessus ou $R_7$ représente un radical carboxy libre, salifié ou estérifié, caractérisé en ce que l'on fait réagir un produit de formule (II) :

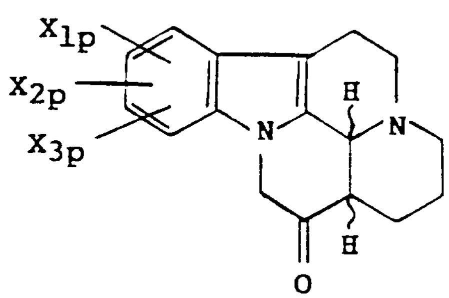

(II)

dans laquelle $X_{1p}$, $X_{2p}$ et $X_{3p}$ ont la signification indiquée ci-dessus, avec un dérivé halogéné de méthyloxosulfonium de formule (IX) :

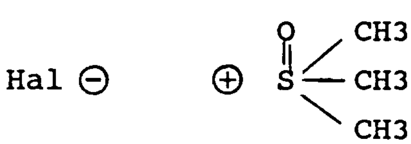

(IX)

en milieu basique, pour obtenir l'époxyde réactionnel correspondant de formule (X) :

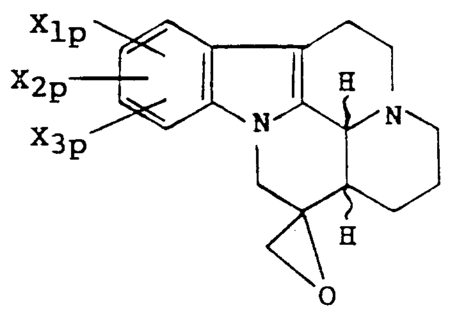

(X)

dans laquelle $X_{1p}$, $X_{2p}$ et $X_{3p}$ ont les significations indiquées ci-dessus, que l'on traite :
**ou bien** par une base pour donner l'alcool correspondant de formule (X') :

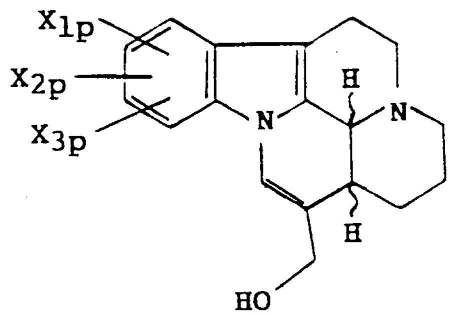

(X')

dans laquelle $X_{1p}$, $X_{2p}$ et $X_{3p}$ ont les significations indiquées ci-dessus, et, si nécessaire, que l'on soumet à une réaction d'élimination des groupements protecteurs des fonctions réactives que peuvent porter $X_{1p}$, $X_{2p}$ et $X_{3p}$, pour donner un produit de formule ($Ia_4$) :

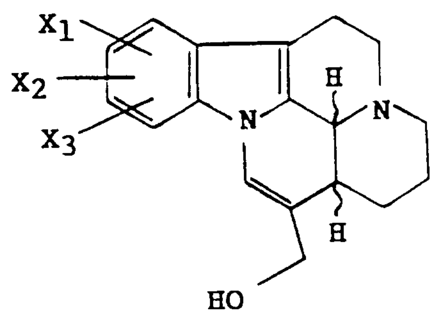

($Ia_4$)

dans laquelle $X_1$, $X_2$, $X_3$ ont les significations indiquées ci-dessus, puis si nécessaire et si désiré, traite le produit ($Ia_4$) obtenu à l'aide d'un agent d'oxydation pour obtenir un produit de formule ($Ia_2$) :

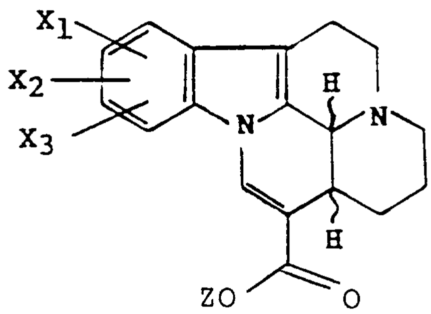

($Ia_2$)

dans laquelle $X_1$, $X_2$, $X_3$ et Z ont les significations indiquées ci-dessus, que l'on soumet si nécessaire et si désiré à l'une ou aux deux réactions suivantes :

- hydrolyse du groupement ester,
- estérification ou salification par une base de la fonction carboxylique,

**ou bien** par une amine de formule (XI) :

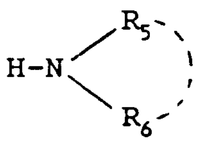

(XI)

dans laquelle $R_5$ et $R_6$ ont la signification indiquée ci-dessus, pour obtenir le composé de formule (XII) :

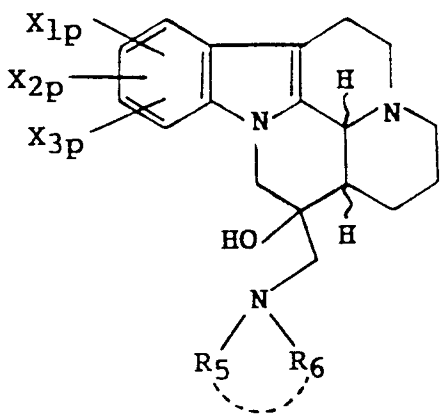

(XII)

dans laquelle $X_{1p}$, $X_{2p}$ et $X_{3p}$ ont les significations indiquées ci-dessus, lesdits composés de formules (X') et (XII) étant soumis aprés activation du radical hydroxyle :

- soit, si désiré dans le cas du composé (X'), à une réaction avec l'amine de formule (XI) telle que définie ci-dessus,
- soit, dans le cas du composé de formule (XII), à une réaction de déshydratation, pour obtenir, dans ces deux cas et aprés élimination, si nécessaire, des groupements protecteurs des fonctions réactives que peuvent porter $X_{1p}$,

$X_{2p}$ et $X_{3p}$, un composé de formule ($Ia_5$) :

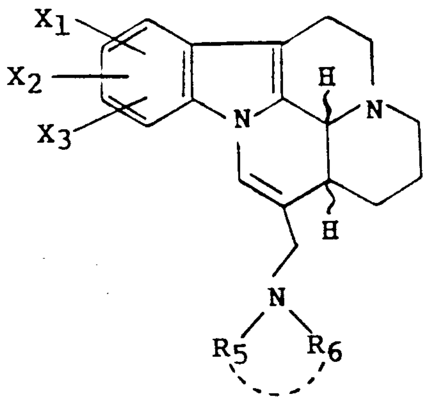

($Ia_5$)

dans laquelle $X_1$, $X_2$, $X_3$, $R_5$ et $R_6$ ont les significations indiquées ci-dessus,

- ou bien par un halogénure de tétrabutylammonium pour obtenir un produit de formule (XIII) :

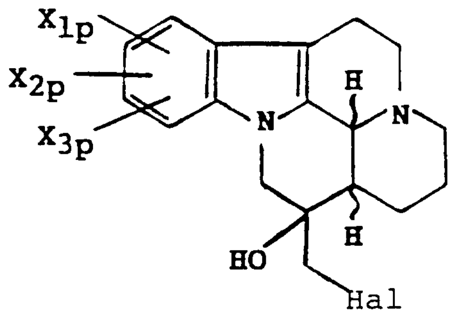

(XIII)

dans laquelle Hal représente un atome d'halogène, puis soumet à une réaction de déshydratation pour obtenir après élimination, si nécessaire, des groupements protecteurs des fonctions réactives que peuvent porter $X_{1p}$, $X_{2p}$ et $X_{3p}$ un composé de formule ($Ia_6$) :

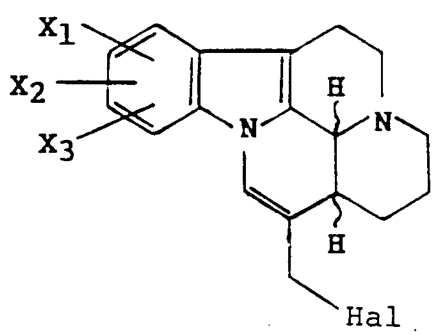

($Ia_6$)

dans lequel $X_1$, $X_2$, $X_3$ et Hal ont la signification indiquée ci-dessus et traite, si désiré le produit de formule ($Ia_3$), ($Ia_5$) et ($Ia_6$) par un acide minéral ou organique, pour obtenir le sel correspondant, lesdits produits de formule (I) étant sous toutes les formes isoméres possibles racémiques ou énantiomères.

Dans des conditions préférentielles de mise en oeuvre de l'invention, le procédé ci-dessus est réalisé de la manière suivante :

- la réaction, telle que définie ci-dessus du produit de formule (II) avec le dérivé halogéné de méthyloxosulfonium de formule (IX), tel que par exemple l'iodure de triméthyloxosulfonium, pour obtenir des composés de formule (X), est réalisée dans un milieu basique par exemple de terbutylate de potassium ou de butyllithium en présence d'un solvant organique tel que le tétrahydrofuranne ou l'éther.

L'ouverture de l'époxyde réactionnel de formule (X) conduisant au produit de formule (X') est réalisée par exemple dans une solution de diisopropylamidure de lithium dans le tétrahydrofuranne à une température d'environ -70°C que l'on laisse ensuite remonter à la température ambiante.

L'oxydation du produit de formule ($Ia_4$) conduisant au produit de formule ($Ia_2$) est effectuée comme indiqué ci-dessus pour l'oxydation des produits ($Ia_1$).

L'hydrolyse du groupement ester, l'estérification ou la salification éventuelle est effectuée selon les méthodes connues de l'homme de métier.

La réaction d'ouverture de l'époxyde de formule (X) avec addition de l'amine de formule (XI), conduisant au composé de formule, (XII) est réalisée dans un solvant alcoolique tel que le méthanol ou l'éthanol au reflux de l'alcool utilisé.

L'hydroxyle des composés de formule (X') et (XII) peut être activé à l'aide par exemple de chlorure de mésyle ou encore de chlorure de trifluoroacétyle dans une solution de tétrahydrofuranne en présence d'une base telle que la pyridine ou la triéthylamine.

La déshydratation du composé de formule (XII) activé conduisant au produit de formule ($Ia_5$) est réalisée dans un solvant organique tel que par exemple le toluène en présence d'une base forte telle que la diazabicycloundécène ou la diazabicyclononène au reflux.

Le produit de formule ($Ia_5$) peut également être obtenu par addition de l'amine de formule (XI) sur le composé de formule (X') activé : la réaction se produit dans un solvant tel que le toluène ou le xylène ou un alcool tel que par exemple le méthanol ou l'éthanol, le mélange étant porté au reflux.

L'halogénure que l'on utilise pour obtenir un produit de formule (XIII) est par exemple le fluorure de tétrabutylammonium au sein d'un solvant organique.

L'invention a encore pour objet un procédé de préparation des produits de formule (I) telle que définie ci-dessus et dans laquelle :

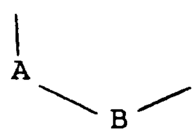

représente le groupement

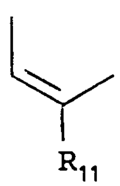

dans lequel $R_{11}$ représente le radical acétylénique de formule :

$$R_{10}\text{-C}{\equiv}\text{C-}$$

dans laquelle $R_{10}$ a la signification indiquée ci-dessus, caractérisé en ce que l'on soumet un produit de formule (VIII) :

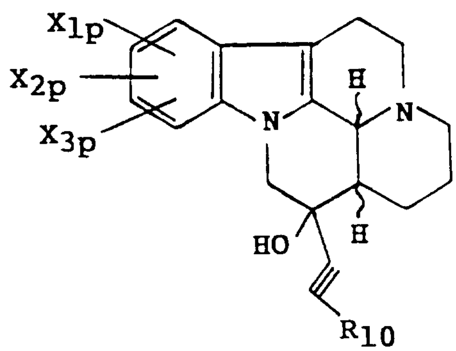

(VIII)

telle que définie ci-dessus, dans laquelle $X_{1p}$, $X_{2p}$, $X_{3p}$ et $R_{10}$ ont la signification indiquée ci-dessus, après activation du radical hydroxyle, à une réaction de déshydratation pour obtenir, aprés élimination, si nécessaire et si désiré, des groupements protecteurs des fonctions réactives que peuvent porter $X_{1p}$, $X_{2p}$ et $X_{3p}$, un composé de type ényne de formule ($Ia_7$) :

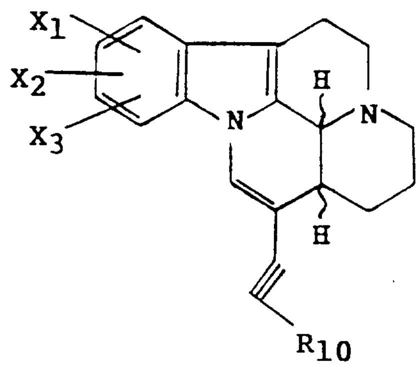

($Ia_7$)

dans laquelle $X_1$, $X_2$, $X_3$ et $R_{10}$ ont les significations indiquées ci-dessus, et traite, si désiré le produit de formule ($Ia_7$) par un acide minéral ou organique, lesdits produits de formule (I) étant sous toutes les formes isoméres possibles racémiques ou énantiomères.

La réaction de déshydratation du composé de formule (VIII) activé sur la fonction hydroxyle, par exemple, comme indiqué ci-dessus, en mésylate ou en acétate, est réalisée préférentiellement dans un solvant organique tel que par exemple le toluène en présence d'une base telle que par exemple la diazabicycloundécène ou la diazabicyclononène.

Les diverses fonctions réactives que peuvent porter certains composés ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :

- les groupements hydroxy peuvent être protégés par exemple par les radicaux triméthylsilyle, tert-butyldiméthylsilyle, dihydropyranne ou méthoxyméthyle,
- les groupements amino peuvent être protégés par exemple par les radicaux trityle, benzyle, tert-butoxycarbonyle, phtalimido ou d'autres radicaux connus dans la chimie des peptides,
- les groupements acyles tel que le groupement formyle peuvent être protégés par exemple sous forme de cétals cycliques ou non cycliques tels que le diméthyl ou diéthylcétal ou l'éthylène dioxycétal,
- les groupements carboxy peuvent être protégés par exemple sous forme d'esters formés avec les esters facilement clivables tels que les esters benzyliques ou tert-butyliques.

L'élimination de ces groupements protecteurs est effectuée dans les conditions usuelles connues de l'homme de métier notamment l'hydrolyse acide effectuée avec un acide tel que l'acide chlorhydrique, benzène sulfonique ou paratoluène sulfonique, formique ou trifluoroacétique.

Le groupement phtalimido est éliminé par l'hydrazine. On trouvera une liste de différents groupements protecteurs utilisables par exemple dans le brevet BF 2 499 995.

Les formes optiquement actives des produits de formule (I) peuvent être préparées par dédoublement des racémiques selon les méthodes usuelles ou en utilisant comme produits de départ des produits de formule (II) sous formes optiquement actives.

Les composés de formule (I) tels que définis ci-dessus ainsi que leurs sels d'addition avec les acides présentent d'intéressantes propriétés pharmacologiques.

Certains produits présentent notamment une affinité pour les récepteurs alpha2 adrénergique.

Certains produits peuvent aussi présenter d'intéressantes propriétés anti-amnésiantes, anti-dépressives protectrices neuronales, anti-anoxiques, anti-ischémiques.

Ces propriétés justifient leur application en thérapeutique et l'invention a également pour objet à titre de médicaments, les produits tels que définis par la formule (I) ci-dessus, lesdits produits de formule (I) étant sous toutes les formes isoméres possibles racémiques ou optiquement actives, ainsi que les sels d'addition avec les acides minéraux ou organiques pharmaceutiquement acceptables desdits produits de formule (I).

L'invention a plus particulièrement pour objet, à titre de médicaments, les produits de formule (I) suivants :

- l'oxalate de [16alpha,(±)]-15-(1-propynyl) 20,21-dinoréburnaménine,
- le [16 alpha,(±)]-15-méthyl 20,21-dinoréburnaménine,
- le maléate acide de [16alpha,(±)]-14,15-dihydro 15-méthylène 20,21-dinoréburnaménine,
- le maléate de [16alpha,(±)] 20,21-dinoréburnaménine-15-acétate d'éthyle,
- le maléate acide de [16alpha,(±)] 20,21-dinoréburnaménine-15-méthanol
- le maléate acide de [16alpha,(±)]-N,N-diméthyl 20,21-dinoréburnaménine-15-méthanamine,

ainsi que, le cas échéant, leurs sels d'addition avec les acides minéraux ou organiques pharmaceutiquement acceptables.

Les médicaments, objet de l'invention, peuvent être utilisés dans le traitement des insuffisances cérébrales d'origine anoxique ou ischémique dans les troubles de la mémoire et de l'attention. Ils peuvent également être utilisés comme anti-dépresseurs.

L'invention s'étend aux compositions pharmaceutiques renfermant comme principe actif les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 50 mg par jour chez l'adulte, par voie orale.

Le composé de départ de formule (IIa) dans laquelle les trois substituants $X_1$, $X_2$ et $X_3$ représentent des atomes d'hydrogène, est décrit dans le brevet européen n° 0013315.

Les composés de départ de formule (II') :

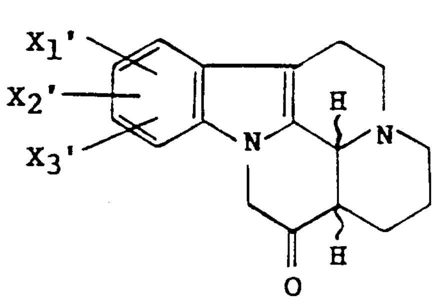

(II')

dans laquelle $X_1'$, $X_2'$ et $X_3'$, identiques ou différents, représentent $X_1$, $X_2$ et $X_3$ étant entendu que l'un au moins de $X_1'$, $X_2'$ et $X_3'$ ne représente pas un atome d'hydrogène, peuvent être préparés selon le procédé décrit dans le brevet cité précédemment à partir des tryptamines substituées correspondantes.

Un autre procédé de préparation de produits de formule (II') telle que définie ci-dessus consiste à soumettre le produit de formule (IIa) :

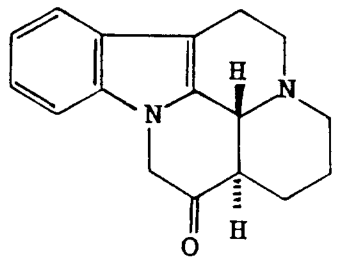

(IIa)

à une réaction de nitration pour obtenir un produit de formule (IIb) :

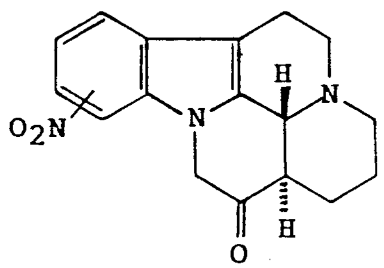

(IIb)

que l'on réduit le cas échéant pour obtenir un produit de formule (IIc) :

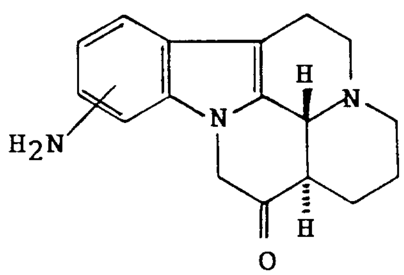

(IIc)

que, le cas échéant :

- soit l'on soumet à une réaction d'alkylation ou d'acylation,

- soit l'on transforme en sel de diazonium à partir duquel on prépare par les procédés connus les dérivés de formule (IId) :

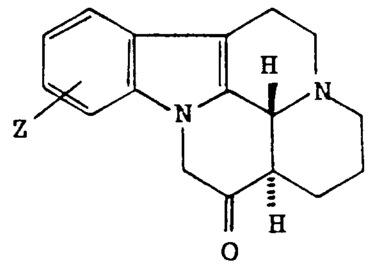

(IId)

dans laquelle Z représente un atome d'halogène ou un radical hydroxy ou phényle éventuellement substitué, que l'on transforme le cas échéant en dérivés correspondants dans lesquels Z représente un radical alkyloxy ou alkyle.

La présente invention a enfin pour objet à titre de produits industriels nouveaux, notamment à titre de produits intermédiaires nécessaires à la préparation des produits de formule (I), les composés de formules (II") :

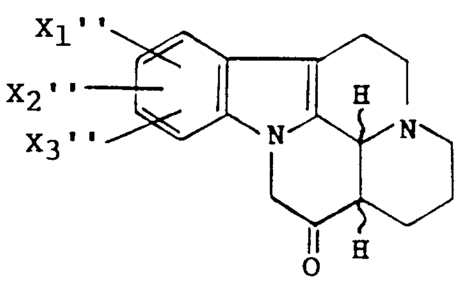

(II'')

dans laquelle $X_1$", $X_2$" et $X_3$" identiques ou différents représentent $X_1$, $X_2$ et $X_3$, à l'exception des produits dans lesquels l'un des substituants $X_1$", $X_2$" et $X_3$"représente un atome d'hydrogène et les deux autres radicaux, identiques ou différents, sont choisis parmi les atomes d'hydrogène ou d'halogène, les radicaux alkyle ou alkyloxy renfermant de 1 à 5 atomes de carbone, un radical hydroxy trifluorométhyle ou nitro et les composés de formules (V), (VIII), (X), (X'), (XII) et (XIII).

Les exemples suivants illustrent l'invention sans toute-fois la limiter.

**EXEMPLE 1 : [16alpha,(±)]-15-éthynyl-20,21-dinoréburnaménine.**

STADE A : [16alpha,(±)]-14,15-dihydro-15-éthynyl-20,21-dinoréburnaménin-15-ol.

On fait barboter de l'acétylène, pendant 30 minutes à 0°C dans 600 cm³ d'une solution molaire de terbutylate de potassium dans le tétrahydrofuranne. On ajoute, à 0°C, une solution de 6 g de [16alpha,(±)]-20,21-dinoréburnaménin-15(14H)-one, dans 140 cm³ de tétrahydrofuranne. On agite 3 heures à température ambiante. On verse dans l'eau, extrait avec du chlorure de méthylène, sèche et évapore à sec sous pression réduite, on recueille 5,4 g de produit attendu que l'on utilise tel quel pour le stade suivant.

STADE B : [16alpha,(±)]-14,15-dihydro-15-éthynyl-20,21-dinoréburnaménin-15-méthanesulfonate.

A une solution de 3,1 g du produit obtenu au stade A dans 125 cm³ de tétrahydrofuranne, on ajoute 3,7 cm³ de triéthylamine. On agite 20 minutes et ajoute lentement 0,8 cm³ de chlorure de méthane sulfonyle. On agite 1 heure 30 à température ambiante. On filtre et concentre le filtrat à sec. On recueille 3,8 g de produit attendu utilisé tel quel pour le stade suivant.

RMN : ($CDCl_3$) 250 MHz

$N-CH_2-C$ 5,18 ppm (d, j = 11 Hz)

| | |
|---|---|
| -O-Mes | 3,28 (s) |
| acétylénique | 3,06 (s) |
| aromatiques | 7,1 à 7,5 (m). |

STADE C : [16alpha,(±)]-15-éthynyl-20,21-dinoréburnaménine.

A une solution de 3,8 g du produit obtenu au stade B dans 125 $cm^3$ de toluène, on ajoute 3,2 $cm_3$ de diazabicyclo[5,4,0] undec-7-ène (DBU), agite 20 heures à température ambiante puis 8 heures à 50°C. On évapore le toluène et reprend le résidu avec 200 $cm^3$ d'acétate d'éthyle, lave à l'eau, sèche et évapore à sec. On chromatographie le résidu (4,2 g) sur silice, (éluant chlorure de méthylène-méthanol (98-2)), recueille 2,3 g de produit que l'on chromatographie à nouveau sur silice, (éluant : hexane-acétate d'éthyle (8-2)). On obtient 1,6 g de produit recherché F = 106°C.

Spectre I.R. ($CHCl_3$)

| | |
|---|---|
| -C≡C-H | 3306 $cm^{-1}$ |
| Bandes de Bohlmann | |
| Système conjugué | 1654 $cm^{-1}$ |
| + | 1615 $cm^{-1}$ |
| Aromatiques | 1594 $cm^{-1}$ (F) |
| | 1569 $cm^{-1}$ (fine). |

**EXEMPLE 2 : [16alpha,(±)]-15-(1-propynyl)-20,21-dinoréburnaménine et son oxalate.**

STADE A : [16alpha,(±)]-14,15-dihydro-15-(1-propynyl)-20,21-dinoréburnaménin-15-ol.

On opère comme au stade A de l'exemple 1, à partir de 3 g de [16alpha,(±)]-20,21-dinoréburnaménin-15(14H)-one, en utilisant du propyne à la place de l'acétylène. On obtient 2,01 g de produit F > 260°C, après empâtage dans l'acétone, utilisé tel quel pour le stade suivant.

STADE B : [16alpha,(±)]-14,15-dihydro-15-(1-propynyl)-20,21-dinoréburnaménin-15-méthane-sulfonate.

On opère comme au stade B de l'exemple 1 à partir de 0,750 g du produit obtenu au stade A, on obtient 0,6 g de produit attendu, utilisé tel quel pour le stade suivant.

STADE C : [16alpha,(±)]-15-(1-propynyl)-20,21-dinoréburnaménine et son oxalate.

A une solution de 0,6 g du produit obtenu au stade B dans 30 $cm^3$ de toluène, on ajoute 0,22 $cm^3$ de diazabicyclo[5,4,0] undec-7-ène (DBU), on agite 20 heures à 50°C. On évapore le toluène et chromatographie le résidu sur silice (éluant : acétate d'éthyle-hexane (2-8)). On obtient 0,270 g de produit recherché sous forme de base. F > 260°C.
Salification : A une solution de 0,1 g du produit obtenu ci-dessus, dans 20 $cm^3$ d'acétate d'éthyle à chaud, on ajoute 32 mg d'acide oxalique en solution dans 2 $cm^3$ d'acétate d'éthyle chaud. On agite 4 heures à température ambiante, filtre, lave avec 10 $cm^3$ d'acétate d'éthyle. On obtient 0,097 g du produit recherché. F = 220°C.

| Analyse pour $C_{20}H_{20}N_2,C_2H_2O_4$ | | | | | | |
|---|---|---|---|---|---|---|
| calculés | C% | 69,82 | H% | 5,86 | N% | 7,4 |
| trouvés | | 69,7 | | 5,7 | | 7,2 |

Spectre I.R. ($CHCl_3$)

C=C + Aromatiques, Bandes de Bohlmann: 1652 $cm^{-1}$, 1596 $cm^{-1}$, 1563 $cm^{-1}$

**<u>EXEMPLE 3</u> : [16alpha,(±)]-14,15-dihydro-15-éthénylidène-20,21-dinoréburnaménine et son maléate.**

A une suspension d'hydrure d'aluminium lithium dans 30 $cm^3$ de tétrahydrofuranne, on ajoute lentement, sans dépasser 50°C, une solution de 0,44 g de produit préparé comme au stade B de l'exemple 1 dans 70 $cm^3$ de tétrahydrofuranne. On agite 20 heures à température ambiante. On détruit l'excès d'hydrure avec 15 $cm^3$ de mélange tétrahydrofuranne-eau (2/3-1/3). On évapore le tétrahydrofuranne et reprend le résidu avec 100 $cm^3$ d'acétate d'éthyle, on filtre sur clarcel et lave le filtrat à l'eau, sèche et évapore à sec. On chromatographie le résidu (0,650 g) sur silice (éluant : chlorure de méthylène-méthanol (98-2)). On obtient 0,210 g de produit attendu sous forme de base. F = 138°C.
<u>Salification</u> : A une solution de 0,210 g de la base obtenue ci-dessus dans 100 $cm^3$ d'acétate d'éthyle, on ajoute, à chaud, 88 mg d'acide maléique en solution dans 5 $cm^3$ d'acétate d'éthyle. On agite pendant 2 heures à température ambiante. On filtre et lave avec 3 $cm^3$ d'acétate d'éthyle. On obtient le produit recherché 0,240 g. F = 196°C.

| <u>Analyse</u> pour $C_{19}H_{20}N_2,C_4H_4O_4$ | | | | | | |
|---|---|---|---|---|---|---|
| calculés | C% | 70,39 | H% | 6,16 | N% | 7,13 |
| trouvés | | 70,2 | | 6,1 | | 7,0 |

Spectre I.R. ($CHCl_3$)

1963 $cm^{-1}$ $>C=C=CH_2$

**<u>EXEMPLE 4</u> : [16alpha,(±)]-15-phényl-20,21-dinoréburnaménine et son maléate.**

<u>STADE A</u> : [16alpha,(±)]-14,15-dihydro-15-phényl-20,21-dinoréburnaménin-15-ol.

A une suspension de 5,34 g de magnésium dans 50 $cm^3$ d'éther, on ajoute 21,6 $cm^3$ de bromure de phényle en solution dans 200 $cm^3$ d'éther. On agite à température ambiante jusqu'à disparition complète du magnésium (environ 4 heures). On refroidit à 10°C et on ajoute en 50 minutes à +10°C, 10 g de (16alpha) 20,21-dinoréburnaménin-15(14H)-one (EP 0013315) en solution dans 300 $cm^3$ de tétrahydrofuranne. On agite 20 heures à température ambiante, verse dans 400 $cm^3$ d'eau contenant du chlorure d'ammonium, extrait avec de l'acétate d'éthyle, sèche et évapore à sec sous pression réduite, on chromatographie le résidu (13 g) sur silice (éluant : chlorure de méthylène-acétone (8-2)). On obtient 2,05 g de produit attendu (isomère A). F > 260°C.

<u>STADE B</u> : [16alpha,(±)]-15-phényl-20,21-dinoréburnaménine et son maléate.

A une suspension de 0,8 g du produit obtenu au stade A dans 30 $cm^3$ de toluène anhydre, on ajoute 0,66 g de pentoxyde de phosphore. On chauffe 7 heures au reflux, refroidit et verse dans 150 $cm^3$ d'eau, on alcalinise avec de l'ammoniaque concentrée et extrait avec de l'acétate d'éthyle, lave à l'eau, sèche et évapore à sec sous pression réduite. On chromatographie le résidu (0,8 g) sur silice (éluant chlorure de méthylène-acétate d'éthyle (9-1)) et obtient 0,350 g de produit sous forme de base. F = 148°C.
<u>Salification</u> : 0,6 g de base obtenue comme ci-dessus sont dissous dans 20 $cm^3$ d'acétate d'éthyle à chaud, on ajoute 0,252 g d'acide maléique et agite pendant 2 heures à température ambiante. On essore et lave avec 3 $cm^3$ d'acétate

d'éthyle. On obtient 0,785 g de produit recherché. F = 260°C.

| Analyse pour $C_{23}H_{22}N_2,C_4H_4O_4$ | | | | | | |
|---|---|---|---|---|---|---|
| calculés | C% | 73,28 | H% | 5,92 | N% | 6,33 |
| trouvés | | 73,6 | | 5,9 | | 6,1 |

Spectre I.R. ($CHCl_3$)

C=C 1650 $cm^{-1}$

Aromatique { 1650 $cm^{-1}$, 1612 $cm^{-1}$, 1566 $cm^{-1}$, 1492 $cm^{-1}$

Bande de Bohlmann.

**EXEMPLE 5 : [16alpha,(±)]-15-méthyl-20,21-dinoréburnaménine.**

STADE A : (16alpha) 14,15-dihydro-15-méthyl-20,21-dinoréburnaménin-15-ol.

On opère comme au stade A de l'exemple 4 à partir de 5,33 g de (16alpha) 20,21-dinoréburnaménin-15(14H)-one (EP 0013315) en utilisant 40 $cm^3$ d'une solution toluénique (1,5 M) de bromure de méthyl magnésium, on obtient, après chromatographie sur silice, (éluant : chlorure de méthylène-acétone (1-1)), 3,6 g de produit recherché.

STADE B : [16alpha,(±)]-15-méthyl-20,21-dinoréburnaménine.

A une solution de 3,5 g du produit obtenu au stade A dans 100 $cm^3$ de tétrahydrofuranne, on ajoute 4,3 $cm^3$ de triéthylamine, puis 1,56 g de chlorure de mésyle. On agite 3 heures à température ambiante, filtre l'insoluble et concentre le filtrat à sec sous pression réduite. On dissout le résidu dans 100 $cm^3$ de dioxane et ajoute 3,7 $cm^3$ de diazabicyclo-undécène, agite 16 heures au reflux puis refroidit et agite 48 heures à température ambiante. On évapore à sec sous pression réduite et chromatographie sur silice (éluant : chlorure de méthylène-acétone (1-1)). On obtient 0,6 g de produit attendu. F = 110°C.

En opérant comme à l'exemple 5 et en utilisant l'isomère de 20,21-dinoréburnaménine 15(14H)-one approprié, on a obtenu les produits des exemples suivants :

**EXEMPLE 6 : (16alpha)-15-méthyl-20,21-dinoréburnaménine.**

F = 140°C.
$[alpha]_D$ +313° ± 5° (c = 0,5 % $CHCl_3$).

**EXEMPLE 7 : (3alpha)-15-méthyl-20,21-dinoréburnaménine.**

F = 140°C.
$[alpha]_D$ -311°5 ± 5° (c = 0,5 % $CHCl_3$).

En opérant comme à l'exemple 5 en utilisant le bromure d'alkyl magnésium approprié, on a obtenu les produits des exemples suivants.

**EXEMPLE 8 : [16alpha,(±)]-15-éthyl-20,21-dinoréburnaménine.**

F = 112°C.

**EXEMPLE 9 : maléate acide de [16alpha,(±)]-15-(1-méthyléthyl)-20,21-dinoréburnaménine.**

F = 200°C.

**EXEMPLE 10 : [16alpha,(±)]-14,15-dihydro-15-éthylidène-20,21-dinoréburnaménine et son oxalate.**

A un mélange de 2,51 g de terbutylate de potassium et 8,35 g de bromure d'éthyltriphényl phosphonium, parfaitement anhydre, on ajoute 40 $cm^3$ de tétrahydrofuranne et agite 30 minutes. On ajoute 3 g de (16alpha) 20,21-dinoréburnaménine-15(14H)-one (EP 0013315) en solution dans 80 $cm^3$ de tétrahydrofuranne. On agite pendant 1 heure, filtre et évapore à sec le filtrat. Le résidu est repris avec de l'acétate d'éthyle et filtré, le filtrat est amené à sec, le résidu est empâté dans l'éther isopropylique, on filtre et évapore à sec le filtrat. Au résidu obtenu, on ajoute 100 $cm^3$ d'acétate d'éthyle et 1,008 g d'acide oxalique. On filtre l'oxalate obtenu. On le dissout dans l'eau et alcalinise avec de l'ammoniaque à 0°C, agite 15 minutes, essore et empâte dans 60 $cm^3$ de pentane. On obtient 1,8 g de produit recherché sous forme de base. F = 138°C.
Salification : A une solution de 1,8 g de la base obtenue ci-dessus, dans 120 $cm^3$ d'acétate d'éthyle, on ajoute à 45°C, 10 $cm^3$ d'éthanol et on porte au reflux. On ajoute 0,581 g d'acide oxalique en solution dans 15 $cm^3$ d'acétate d'éthyle chaud et 5 $cm^3$ d'éthanol. On agite 1 heure 30 à température ambiante, filtre, lave avec 20 $cm^3$ d'acétate d'éthyle puis 3 $cm^3$ d'éthanol. On obtient 1,87 g de produit recherché.
F = 225°C.

| Analyse pour $C_{19}H_{22}N_2,C_2H_2O_4$ | | | | | | |
|---|---|---|---|---|---|---|
| calculés | C% | 68,46 | H% | 6,56 | N% | 7,6 |
| trouvés | | 68,8 | | 6,7 | | 7,6 |

RMN $CDCl_3$ 300 MHz
Mélange delta E/delta Z 35/65
1,79 ppm } $CH_3$-CH=
1,88 ppm }
4,43 ppm (J = 14Hz,d) } $N-CH_2-C=$ isomère majoritaire
4,97 ppm (J = 14Hz,d) }
4,44 ppm (système AB) $N-CH_2-C=$ isomère minoritaire
5,53 ppm (9 1) } $=CH-CH_3$
5,82 ppm (9 1) }
de 7,06 à 7,5 ppm (m) 4H : aromatiques.

**EXEMPLE 11 : [16alpha,(±)]-14,15-dihydro-15-méthylène-20,21-dinoréburnaménine et son maléate acide.**

On opère comme à l'exemple 10 à partir de 2 g de (16alpha) 20,21-dinoréburnaménin-15(14H)-one (EP 0013315) en utilisant 5,36 g de bromure de méthyl triphényl phosphonium. On obtient 2 g de produit recherché sous forme de base.
Salification : On dissout les 2 g de produit obtenu ci-dessus dans 100 $cm^3$ d'acétate d'éthyle et on ajoute 872 mg d'acide maléïque en solution dans 50 $cm^3$ d'acétate d'éthyle bouillant. On agite 2 heures à température ambiante, essore, lave à l'acétate d'éthyle. On obtient 2,32 g du produit recherché.

F = 208°C.

| Analyse pour $C_{22}H_{24}N_2O_4$ | | | | | | |
|---|---|---|---|---|---|---|
| calculés | C% | 69,46 | H% | 6,36 | N% | 7,36 |
| trouvés | | 69,4 | | 6,2 | | 7,3 |

Spectre I.R. ($CHCl_3$)

Aromatique { 1623 $cm^{-1}$, 1481 $cm^{-1}$

=$CH_2$ { =CH 3080 $cm^{-1}$, C=C 1653 $cm^{-1}$, def 909 $cm^{-1}$

Bandes de Bohlmann.

**EXEMPLE 12 : [16alpha,(±)]-20,21-dinoréburnaménine-15-acétate d'éthyle et son maléate.**

On opère comme à l'exemple 10, à partir de 2,5 g de (16alpha) 20,21-dinoréburnaménin-15(14H)-one (EP 0013315) en utilisant 0,9 g d'hydrure de sodium à 50 % dans l'huile et 3,72 $cm^3$ de diéthyle phosphonate d'acétate d'éthyle.

On obtient 1,1 g de produit recherché sous forme de base.

Salification : A une solution de 1,1 g de la base obtenue ci-dessus dans 60 $cm^3$ d'acétate d'éthyle chaud, on ajoute 0,379 g d'acide maléïque en solution dans 10 $cm^3$ d'acétate d'éthyle chaud. On agite pendant 2 heures filtre et lave avec de l'acétate d'éthyle. On obtient 1 g de produit recherché. F = 180°C.

| Analyse pour $C_{21}H_{24}N_2O_2,C_4H_4O_4$ | | | | | | |
|---|---|---|---|---|---|---|
| calculés | C% | 66,36 | H% | 6,24 | N% | 6,19 |
| trouvés | | 66,4 | | 6,1 | | 6,2 |

Spectre I.R. ($CHCl_3$)

1728 $cm^{-1}$ >= O système non conjugué
1653 $cm^{-1}$ (F) C=C

**EXEMPLE 13 : [16alpha,(±)]-20,21-dinoréburnaménin-15-méthanol et son maléate acide**

STADE A : [16alpha,(±)] spiro[20,21-dinoréburnaménin-15(14H)-2'oxirane (isomère A) et son maléate acide.

A un mélange de 19 g d'iodure de triméthyl oxosulfonium et 9,6 g de terbutylate de potassium parfaitement anhydre, on ajoute 100 $cm^3$ de tétrahydrofuranne et agite pendant 2 heures 30 à 55°C. On ajoute ensuite en 20 minutes 15 g de (16alpha) 20,21-dinoréburnaménin-15(14H)-one (EP 0013315) en solution dans 100 $cm^3$ de tétrahydrofuranne et agite

1 heure à température ambiante. On filtre l'insoluble et évapore le filtrat à sec ; le résidu est lavé à l'eau puis chromatographié sur silice (éluant acétate d'éthyle), on obtient 10,54 g d'isomère A (F = 182°C) et 2 g d'isomère B (F = 167°C).

Salification : A une solution de 1,39 g de la base obtenue ci-dessus (isomère A) dans 100 $cm^3$ d'acétate d'éthyle, on ajoute une solution de 575 mg d'acide maléïque dans 50 $cm^3$ d'acétate d'éthyle. On agite pendant 4 heures, essore et lave avec 100 $cm^3$ d'acétate d'éthyle. On obtient 1,66 g de produit recherché. F = 200°C.

STADE B : [16alpha,(±)]-20,21-dinoréburnaménin-15-méthanol et son maléate acide

a) Préparation de la lithium diisopropylamide.

A une solution de 6 $cm^3$ de diisopropylamine dans 100 $cm^3$ de tétrahydrofuranne, agitée à -70°C, on ajoute goutte à goutte 28,3 $cm^3$ de butyllithium. On agite vingt minutes à 0°C/+10°C.

b) On refroidit la solution ci-dessus à -70°C et ajoute en maintenant cette température, une solution de 9,9 g de l'isomère A du (16alpha) 20,21-dinoréburnaménin-15(14H)-2'oxiranne dans 150 $cm^3$ de tétrahydrofuranne, agite pendant trente minutes à -70°C puis 1 heure à température ambiante. On ajoute 200 $cm^3$ d'eau glacée et ajuste à pH 7 par addition d'acide chlorhydrique N. On extrait avec du chlorure de méthylène, lave à l'eau, sèche et évapore à sec sous pression réduite. On reprend le résidu avec un mélange chlorure de méthylène-méthanol (2-1), filtre la solution sur florisil, évapore le filtrat à sec et obtient 9,38 g de produit attendu sous forme de base.

F = 188°C.

Salification : A une solution de 1,38 g de la base obtenue ci-desus dans 200 $cm^3$ d'un mélange acétate d'éthyle-éthanol (1-1) on ajoute une solution de 571 mg d'acide maléïque dans 50 $cm^3$ d'éthanol bouillant. On agite 3 heures à température ambiante. On essore, lave à l'éthanol, sèche et obtient 1,55 g du produit recherché. F = 220°C.

| Analyse pour $C_{22}H_{24}N_2O_5$ | | | | | | |
|---|---|---|---|---|---|---|
| calculés | C% | 66,65 | H% | 6,10 | N% | 7,07 |
| trouvés | | 66,5 | | 6,2 | | 6,9 |

Spectre I.R. ($CHCl_3$)

1608 $cm^{-1}$ (F) -OH

1653 $cm^{-1}$ (F) -C=C- + C=N

1627 $cm^{-1}$ (F) + aromatique

Bandes de Bohlmann.

**EXEMPLE 14 : [16alpha,(±)]-N,N-diméthyl-20,21-dinoréburnaménin-15-méthanamine et son maléate.**

STADE A : [16alpha,(±)]-14,15-dihydro N,N-diméthyl-15-méthanamine 20,21-dinoréburnaménin-15-ol.

Dans une solution de 4 g du produit obtenu au stade A de l'exemple 13 dans 80 $cm^3$ de tétrahydrofuranne, on fait barboter à 0/-5°C de la diméthylamine gaz jusqu'à saturation, on chauffe 24 heures au reflux sous pression, amène à sec, empâte à l'eau, essore et sèche sous pression réduite à 70°C. On obtient 4,37 g du produit recherché. F = 200°C, utilisé tel quel pour le stade suivant.

STADE B : [16alpha,(±)]-15-chloro-14,15-dihydro N,N-diméthyl-20,21-dinoréburnaménin-15-méthanamine.

A une solution de 2,75 g du produit obtenu au stade A dans 50 $cm^3$ de tétrahydrofuranne, on ajoute à -20°C, lentement, 6,3 $cm^3$ d'une solution de butyllithium à 1,5 M, et agite à 0°C pendant 45 minutes. On ajoute une solution de 0,74 $cm^3$ de chlorure de méthane sulfonyle dans 10 $cm^2$ de tétrahydrofuranne, agite 15 heures à température ambiante, filtre, lave avec du tétrahydrofuranne et concentre à sec le filtrat. On chromatographie le résidu sur silice (éluant : chlorure de méthylène-méthanol (95-5) et obtient 1,84 g du produit attendu. F = 140°C.

STADE C : [16alpha,(±)]-N,N-diméthyl-20,21-dinoréburnaménin15-méthanamine et son maléate.

A une solution de 1,84 g du produit obtenu au stade B dans 100 $cm^3$ de toluène, on ajoute 4 $cm^3$ de diazabicyclo-undécène (D.B.U.) et agite 24 heures au reflux. On refroidit, lave à l'eau, sèche et évapore à sec sous pression réduite. On chromatographie le résidu sur silice, (éluant acétate d'éthyle à 3 % de triéthylamine), on obtient 1,07 g de produit recherché sous forme de base. F = 111°C.
Salification : A une solution de 447 mg du produit obtenu ci-dessus dans 430 $cm^3$ d'acétate d'éthyle, on ajoute 337 mg d'acide maléïque en solution dans 10 $cm^3$ d'éthanol bouillant, on agite pendant 3 heures à température ambiante, puis à 0°C pendant 10 minutes. On essore, lave avec 2 x 10 $cm^3$ d'éthanol. Après séchage, sous pression réduite à 60°C, on obtient 570 mg de produit recherché. F = 176°C.

Spectre IR :

C = C 1653 $cm^{-1}$

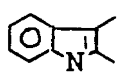 1628 $cm^{-1}$

Bandes de Bohlmann.

| Microanalyse pour $C_{28}H_{33}N_3O_8$ | | | | | | |
|---|---|---|---|---|---|---|
| calculés | C% | 62,33 | H% | 6,16 | N% | 7,79 |
| trouvés | | 62,0 | | 6,3 | | 7,8 |

**EXEMPLE 15 : [16alpha,(±)]-N-méthyl 20,21-dinoréburnaménin-15-méthanamine et son maléate.**

En opérant comme à l'exemple 14 à partir de 4 g du produit obtenu au stade A de l'exemple 13 et en utilisant de la monoéthylamine, on a obtenu le produit attendu.

**EXEMPLE 16 : [16alpha,(±)] 20,21-dinoréburnaménin-15-acétonitrile et son maléate acide.**

On ajoute goutte à goutte sous atmosphère inerte 1,35 $cm^3$ de phosphonate de diéthylcyanométhyle dans une suspension de 0,396 g d'hydrure de sodium dans 25 $cm^3$ de tétrahydrofuranne. On maintient 30 minutes sous agitation, ajoute 1 g de (16alpha) 20,21-dinoréburnaménine 15 (14H)-one (EP. 0 013 315) en solution dans 20 $cm^3$ de tétrahydrofuranne et agite 3 heures à température ambiante. On verse le milieu réactionnel sur de l'eau glacée, extrait à l'acétate d'éthyle, lave à l'eau, sèche, chromatographie sur silice (éluant : chlorure de méthylène-acétonitrile 8-2) et obtient 740 mg de produit attendu sous forme de base. On dissout à chaud 200 mg de base dans 30 $cm^3$ d'acétate d'éthyle et 5 $cm^3$ d'éthanol, ajoute 80 mg d'acide maléique, agite 3 heures à température ambiante, sèche à 70°C sous pression réduite et recueille 220 mg de produit attendu. F = 250°C.
Spectre IR ($CHCl_3$) :

C = C aromatique : 1656, 1633 $cm^{-1}$
C ≡ N : 2250 $cm^{-1}$

Bandes de Bohlmann.

| Microanalyse pour $C_{19}H_{19}N_3$, $C_4H_4O_4$ = 405,456 | | | | | | |
|---|---|---|---|---|---|---|
| calculés | C% | 68,13 | H% | 5,72 | N% | 10,36 |
| trouvés | | 68,3 | | 5,7 | | 10,4 |

**EXEMPLE 17 : [16alpha,(±)] (20,21-dinoréburnaménin-15-yl) propynoate d'éthyle.**

STADE A : (16alpha) (±) 14,15-dihydro 15-propiolate d'éthyle 20,21-dinoréburnaménine 15-ol.

On dissout 6,4 $cm^3$ de diisopropylamine dans 15 $cm^3$ de tétrahydrofuranne, ajoute à -10°/-20°C sous atmosphère inerte, 30 $cm^3$ d'une solution 1,6M de butyllithium dans l'hexane. On agite la solution 2 heures à -20°C puis 15 minutes à 0°C. On refroidit à -50°C, ajoute lentement 4,8 $cm^3$ de propiolate d'éthyle, agite 30 minutes à -50°C, ajoute 4 g de (16alpha) (±) 20,21-dinoréburnaménine 15 (14H)-one (EP. 0 013 315) en solution dans 60 $cm^3$ de tétrahydrofuranne et maintient sous agitation à cette température pendant 1 heure. On verse le milieu réactionnel dans l'eau, extrait à l'acétate d'éthyle, lave la phase organique à l'eau, la sèche et élimine les solvants sous pression réduite. On obtient 6,1 g de produit que l'on purifie par chromatographie sur silice (éluant : chlorure de méthylène-méthano1 95-5). On recueille 3,7 g de produit attendu. F = 228°C.
Spectre IR ($CHCl_3$) :

OH + associé : 3595 $cm^{-1}$
C ≡ C : 2444 $cm^{-1}$
>C = O : 1710 $cm^{-1}$ -> ester conjugué
C = C aromatique : 1630, 1604, 1512 $cm^{-1}$

STADE B : [16alpha,(±)] (20,21-dinoréburnaménin-15-yl) propynoate d'éthyle.

On dissout 3,7 g de produit obtenu au stade A dans 30 $cm^3$ de tétrahydrofuranne, ajoute sous atmosphère inerte 2,7 $cm^3$ de triéthylamine puis 0,97 $cm^3$ de chlorure de mésyle goutte à goutte. On agite 2 heures à température ambiante, filtre la suspension, concentre le filtrat et recueille 5,3 g de mésylate. On dissout le produit dans 130 $cm^3$ de toluène, ajoute 1,49 $cm^3$ de diazabicyclo-undecène, agite pendant 4 heures à température ambiante et concentre le milieu réactionnel sous pression réduite. On obtient 6,5 g de produit que l'on purifie par chromatographie sur silice (éluant : hexane-acétate d'éthyle 8-2 puis chlorure de méthylène-méthanol 95-5). On obtient le produit attendu. F = 100°C.

| Microanalyse pour $C_{22}H_{22}N_2O_2$ | | | | | | |
|---|---|---|---|---|---|---|
| calculés | C% | 76,27 | H% | 6,4 | N% | 8,08 |
| trouvés | | 76 | | 6,4 | | 7,8 |

**EXEMPLE 18 : (16alpha) (±) 20,21-dinoréburnaménin 15-acétamide et son maléate acide.**

On ajoute 10 $cm^3$ de soude 5N puis 1 $cm^3$ d'eau oxygénée dans 350 mg de (16alpha) (±) 20,21-dinoréburnaménin 15-acétonitrile préparé comme à l'exemple 16 en solution dans 10 $cm^3$ de méthanol. On agite 3 heures à température ambiante, ajoute 50 $cm^3$ d'eau à la suspension obtenue, essore le précipité, le lave à l'eau, le sèche à 70°C sous pression réduite et obtient 190 mg de produit attendu sous forme de base. F = 160°C. On dissout 250 mg de base obtenue comme ci-dessus dans un mélange de 10 $cm^3$ d'acétate d'éthyle et 10 $cm^3$ d'éthanol. On ajoute 87 mg d'acide d'acide fumarique solubilisé dans 10 $cm^3$ d'éthanol bouillant. On agite le milieu réactionnel pendant 2 heures à température ambiante et

récupère après séchage sous pression réduite et recristallisation dans l'éthanol, 140 mg de produit attendu. F = 230°C.

Spectre IR (nujol) :

C = O : 1672 $cm^{-1}$

système conjugué + aromatique $NH_2$ : 1648, 1618, 1600 $cm^{-1}$

| Microanalyse pour $C_{19}H_{21}N_30$, $C_4H_4O_4$ | | | | | | |
|---|---|---|---|---|---|---|
| calculés | C% | 65,24 | H% | 5,95 | N% | 9,92 |
| trouvés | | 65,3 | | 5,9 | | 9,9 |

**<u>EXEMPLE 19</u> : (16alpha) (±) (14,15-dihydro 20,21-dinoréburnaménin 15-ylidèn) acétonitrile et son maléate acide.**

On opère comme à l'exemple 16 en utilisant au départ 0,54 $cm^3$ de phosphonate de diéthylcyanométhyle, 0,16 g d'hydrure de sodium et 0,8 g de (16alpha) 20,21-dinoréburnaménin 15 (14H)-one (EP. 0 013 315). On obtient 560 mg de produit attendu sous forme de base puis 245 mg de maléate acide. F = 222°C.

Spectre IR ($CHCl_3$) :

C = C, C = N, aromatique : 1656, 1631 $cm^{-1}$

C ≡ N : 2224 $cm^{-1}$

Bandes de Bohlmann

| Microanalyse pour $C_{19}H_{19}N_3$, $C_4H_4O_4$ | | | | | | |
|---|---|---|---|---|---|---|
| calculés | C% | 68,13 | H% | 5,72 | N% | 10,36 |
| trouvés | | 68,2 | | 5,6 | | 10,4 |

**<u>EXEMPLE 20</u> : Acide (16alpha) (±) 20,21-dinoréburnaménin 15-acétique et son chlorhydrate.**

On chauffe 13 heures à 100°C 1,1g de /16alpha (±)/ 20,21-dinoréburnaménin 15-acétate d'éthyle préparé comme à l'exemple 12 en suspension dans 55 ml d'une solution d'hydroxyde de sodium 2N. On refroidit à 0°C, acidifie avec de l'acide chlorhydrique concentré, essore les cristaux, les lave à l'éthanol et les sèche à 50°C. On obtient 0,87 g de produit

brut que l'on recristallise dans l'eau. F > 260°C.

Spectre IR (nujol) :
=O : 1724 (max), 1700 $cm^{-1}$ (eq)
système conjugué
+ 1652, 1630, 1602, 1562 $cm^{-1}$
aromatique

| Microanalyse pour $C_{19}H_{20}N_2O_2$, HCl | | | | | | | | |
|---|---|---|---|---|---|---|---|---|
| calculés | C% | 66,18 | H% | 6,14 | N% | 8,12 | Cl% | 10,28 |
| trouvés | | 65,9 | | 6,0 | | 8,1 | | 10,1 |

**<u>EXEMPLE 21</u> : (16alpha) (±) 20,21-dinoréburnaménin 15-acétate d'hexyle et son oxalate.**

On agite pendant 10 minutes 0,8 g de produit préparé à l'exemple 20 en suspension dans 75 $cm^3$ de chlorure de méthylène, ajoute 0,66 g de chlorhydrate de 1-éthyl 3-(3-diméthyl-aminopropyl) carbodiimide. On agite 10 minutes, ajoute 0,28 g d'hexanol, maintient 16 heures sous agitation à température ambiante. On verse la suspension dans l'eau, extrait au chlorure de méthylène, lave la phase organique avec une solution aqueuse de bicarbonate de sodium puis à l'eau, sèche et élimine les solvants sous pression réduite. Après chromatographie sur silice (éluant : chlorure de méthylène-acétonitrile 9-1), on obtient 0,3 g de produit attendu sous forme de base. On dissout à chaud 0,3 g de la base dans 10 $cm^3$ d'acétate d'éthyle et ajoute 70 mg d'acide oxalique dissous à chaud dans 5 $cm^3$ d'acétate d'éthyle. On agite 1 heure la suspension obtenue, essore les cristaux, les lave à l'acétate d'éthyle et les sèche. On récupère 0,240 g de sel attendu. F = 190°C.

| Microanalyse pour $C_{25}H_{32}N_2O_2$, $C_2H_2O_4$ | | | | | | |
|---|---|---|---|---|---|---|
| calculés | C% | 67,20 | H% | 7,10 | N% | 5,8 |
| trouvés | | 67,4 | | 7,1 | | 5,5 |

**<u>EXEMPLE 22</u> : (16alpha) (±) 1-(20,21-dinoréburnaménin-15-yl) 2-propanone.**

En opérant comme à l'exemple 12 à partir du (16alpha) 20,21-dinoréburnaménin 15 (14H)-one (EP. 0 013 315) et du phosphonate approprié, on a obtenu le produit attendu.

En opérant comme à l'exemple 5 à partir du dérivé de 20,21-dinoréburnaménine 15 (14H)-one et du bromure d'alkyl magnésium appropriés, on a obtenu les produits suivants :

**EXEMPLE 23 : (16alpha) (±) 9-méthoxy 15-méthyl 20,21-dinoréburnaménine.**

**EXEMPLE 24 : (16alpha) (±) 15-(trifluorométhyl) 20,21-dinoréburnaménine.**

Les produits nommés ci-après peuvent également être obtenus selon un procédé de l'invention défini plus haut.

**EXEMPLE 25 : (16alpha) (±) 15-(fluorométhyl) 20,21-dinoréburnaménine.**

**EXEMPLE 26 : (16alpha) (±) 20,21-dinoréburnaménin 15-propanoate d'éthyle.**

**EXEMPLE 27 : (16alpha) (±) 20,21-dinoréburnaménin 15-carboxylate d'éthyle.**

**EXEMPLE 28 : (3alpha) 20,21-dinoréburnaménin 15-acétate d'éthyle.**

**EXEMPLE 29 : (16alpha) 20,21-dinoréburnaménin 15-acétate d'éthyle.**

**EXEMPLE 30 : (16alpha) (±) 20,21-dinoréburnaménin 15-acétate de diméthyléthyle.**

**EXEMPLE 31 : (16alpha) (±) alpha-méthyl 20,21-dinoréburnaménin 15-acétate d'éthyle.**

**EXEMPLE 32 : composition pharmaceutique.**

On a préparé des comprimés répondant à la formule suivante :

| | |
|---|---|
| - Produit de l'exemple 1 | 25 mg |
| - Excipient pour un comprimé terminé à | 300 mg |
| (détail de l'excipient : lactose, talc, amidon, stéarate de magnésium) | |

**EXEMPLE 33 : composition pharmaceutique.**

On a préparé des comprimés répondant à la formule suivante :

| | |
|---|---|
| - Produit de l'exemple 12 | 25 mg |
| - Excipient pour un comprimé terminé à | 300 mg |
| (détail de l'excipient : lactose, talc, amidon, stéarate de magnésium) | |

ETUDE PHARMACOLOGIQUE :

1) Affinité pour les récenteurs alpha2 adrénergiques :

On homogénéise dans 90 ml de sucrose 0,32 M, 10 cortex prélevés sur des cerveaux de rats mâles pesant 150 g en moyenne. Après centrifugation à 1000g du mélange homogénéisé pendant 10 minutes à 0° + 4°C. Le culot est mis en suspension dans 240 ml de tampon TrisHCl 50 mM pH 7,7 et centrifugé à 30 000 g pendant 15 minutes à 0° + 4°C. Le nouveau culot obtenu est mis en suspension dans 480 ml de tampon $NaKPO_4$ pH 7,4 50 mM.

On fait ensuite incuber pendant 45 minutes à 25°C, 2 ml de suspension en présence de $^3H$ rauwolscine à la concentration 0,15 nM :

I) seule,
II) avec des concentrations croissantes du produit à tester ou,

III) pour déterminer la fixation non spécifique, avec de la phentolamine non radioactive à la concentration $10^{-5}$M.

Les suspensions incubées sont filtrées sur Whatman GF/C et les filtres sont lavés par trois fois 5 ml de tampon $NakPO_4$ pH 7,4 à 0°C. La radioactivité des filtres est mesurée par scintillation liquide.

L'affinité du produit testé pour les récepteurs alpha2 adrénergiques est donnée relativement à la phentolamine comme produit de référence.
CD = concentration de phentolamine inhibant 50 % de la fixation spécifique de la $^3$H rauwolscine,
CX = concentration du produit à tester inhibant 50 % de la fixation spécifique de la $^3$H rauwolscine.

L'affinité relative est donnée par la relation :

$$ARL = 100 \frac{CD}{CX}$$

| Résultats produits des exemples | ARL |
|---|---|
| 2 | 367 |
| 5 | 456 |
| 7 | 980 |
| 11 | 343 |
| 12 | 942 |
| 13 | 346 |

Ces produits montrent une très forte affinité pour les récepteurs alpha2 adrénergique.

2) Anoxie hypobare

Des souris mâles de type $CD_1$ CHARLES RIVER de 20 à 25 grammes, à jeun depuis 6 heures sont placées dans une enceinte de 2 litres dans laquelle est réalisée une dépression de 620 mmHg selon la cinétique suivante :

| Temps (s) | Dépression (mmHg) |
|---|---|
| 0 (TO) | 0 |
| 3,5 | 350 |
| 6 | 400 |
| 9 | 450 |
| 12 | 500 |
| 16 | 550 |
| 28 | 600 |
| 34 | 610 |
| 55 | 620 |

Le temps de survie est mesuré à partir du temps TO et sur une durée maximale de 3 mn ; il est de l'ordre de 70 s chez les animaux témoins.

La température rectale est mesurée immédiatement avant la détermination du temps de survie. Les produits sont administrés par voie i.p. à la dose de 10 mg/kg et sous un volume de 10 ml/kg, 60 mn avant l'épreuve. Les animaux témoins reçoivent le véhicule. Les résultats sont donnés en pourcentage d'augmentation du temps de survie par rapport

aux animaux témoins.

| Produit de l'exemple | % augmentation du temps de survie |
|---|---|
| 5 | 34 |
| 13 | 17 |
| 14 | 12 |

## Revendications

**Revendications pour les Etats contractants suivants : AT, BE, CH, DE, DK, FR, GB, IT, LI, LU, NL, SE**

**1.** Produits de formule (I) :

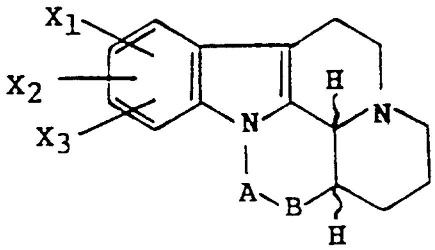

(I)

dans laquelle $X_1$, $X_2$ et $X_3$, identiques ou différents, représentent un atome d'hydrogène, un atome d'halogène, un radical alkyle, alkényle ou alkynyle, ces radicaux étant linéaires ou ramifiés renfermant au plus 18 atomes de carbone et éventuellement substitués, un radical alkyloxy linéaire ou ramifié renfermant au plus 7 atomes de carbone éventuellement substitué, un radical hydroxy, trifluorométhyle ou nitro, un radical amino, mono ou dialkylamino, ou un radical phényle éventuellement substitué,
et dans laquelle

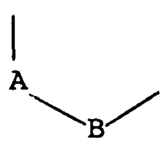

représente

A) soit le groupement

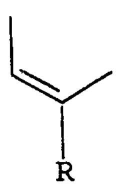

dans lequel R représente :

a) un radical alkyle, alkényle ou alkynyle linéaire ou ramifié renfermant au plus 7 atomes de carbone, ce radical étant éventuellement substitué,
b) un radical phényle éventuellement substitué,
c) un groupement carboxy libre, salifié ou estérifié,

B) soit le groupement

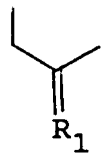

dans lequel $R_1$ représente l'un des deux groupements

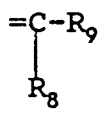

ou

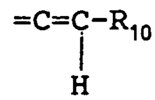

dans lesquels :
$R_8$ et $R_9$, identiques ou différents, sont tels que :

- l'un est choisi dans le groupe formé par :

  a) un atome d'hydrogène,
  b) un radical alkyle, alkényle ou alkynyle linéaire ou ramifié renfermant au plus 6 atomes de carbone, ce radical étant éventuellement substitué,
  c) un radical phényle éventuellement substitué,
  d) un radical carboxy estérifié,
  e) un radical cyano,
  f) un radical acyle ayant de 2 à 6 atomes de carbone,

- et l'autre est choisi dans le groupe formé par :

  a) un atome d'hydrogène,
  b) un radical alkyle, alkényle ou alkynyle linéaire ou ramifié renfermant au plus 6 atomes de carbone, ce radical étant éventuellement substitué,
  c) un radical phényle éventuellement substitué,

et $R_{10}$ représente :

a) un atome d'hydrogène,
b) un radical alkyle, alkényle ou alkynyle linéaire ou ramifié renfermant au plus 5 atomes de carbone, ce radical étant éventuellement substitué,
c) un radical phényle éventuellement substitué,

lesdits produits de formule (I) étant sous toutes les formes isomères possibles racémiques ou optiquement actives, ainsi que les sels d'addition avec les acides minéraux ou organiques ou avec les bases desdits produits de formule (I).

**2.** Produits de formule (I) tels que définis à la revendication 1, caractérisés en ce que $X_1$, $X_2$ et $X_3$ représentent un atome d'hydrogène, lesdits produits de formule (I) étant sous toutes les formes isomères possible racémiques ou optiquement actives, ainsi que les sels d'addition avec les acides minéraux ou organiques ou avec les bases desdits produits de formule (I).

**3.** Produits de formule (I) tels que définis à la revendication 1, caractérisés en ce que le ou les substituants que peuvent porter les radicaux alkyle, alkyloxy, alkényle ou alkynyle sont choisis dans le groupe formé par :

- le radical hydroxy,
- les atomes d'halogène,
- les radicaux alkyloxy ayant de 1 à 6 atomes de carbone,
- les radicaux formyle et acyle ayant de 2 à 6 atomes de carbone et le radical benzoyle,
- les radicaux carboxy libres et estérifiés par un radical alkyle linéaire ou ramifié renfermant au plus 5 atomes de carbone,
- le radical cyano,
- le radical carbamoyle éventuellement substitué,
- le radical phényl éventuellement substitué,
- le radical

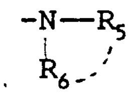

dans lequel :
**soit** $R_5$ et $R_6$, identiques ou différents, représentent :
- un atome d'hydrogène,
- un radical alkyle linéaire ou ramifié renfermant au plus 7 atomes de carbone éventuellement substitué par un ou plusieurs radicaux choisis dans le groupe formé par le radical hydroxy, les radicaux alkyloxy linéaires et ramifiés renfermant au plus 5 atomes de carbone et les radicaux carboxy libres ou estérifiés par un radical alkyle linéaire ou ramifié renfermant au plus 5 atomes de carbone,
- un radical aryle ou arylalkyle renfermant de 7 à 12 atomes de carbone, ces radicaux étant éventuellement substitués,

**soit** $R_5$ et $R_6$ forment ensemble avec l'atome d'azote auquel ils sont liés un hétérocycle de cinq à six chainons, renfermant éventuellement un second hétéroatome choisi parmi l'oxygène, le soufre ou l'azote, éventuellement substitué sur l'atome d'azote non lié à $R_5$ et $R_6$ par :
- un radical alkyle linéaire ou ramifié renfermant au plus 5 atomes de carbone éventuellement substitué par un radical hydroxy, un atome d'halogène ou un radical alkyloxy renfermant de 1 à 4 atomes de carbone,
- un radical aryle ou un radical arylalkyle renfermant de 7 à 12 atomes de carbone, ces radicaux étant éventuellement substitués, lesdits produits de formule (I) étant sous toutes les formes isomères possibles racémiques ou optiquement actives, ainsi que les sels d'addition avec les acides minéraux ou organiques desdits produits de formule (I).

**4.** Produits de formule (I) tels que définis à l'une des revendications 1 à 3, caractérisés en ce que le ou les substituants que peuvent porter les radicaux phényle, aryle et arylalkyle sont choisis dans le groupe formé par les atomes d'halogène, le radical hydroxy, les radicaux alkyle et alkyloxy linéaires et ramifiés renfermant au plus 5 atomes de carbone, les radicaux méthylthio, nitro, amino, monoalkyl- et dialkylamino, lesdits produits de formule (I) étant sous toutes les formes isomères possibles racémiques ou optiquement actives, ainsi que les sels d'addition avec les acides minéraux ou organiques desdits produits de formule (I).

**5.** L'oxalate de [16alpha,(±)]-15-(1-propynyl) -20,21-dinoréburnaménine,

- le [16alpha,(±)]-15-méthyl 20,21-dinoréburnaménine,
- le maléate acide de [16alpha,(±)]-14,15-dihydro 15-méthylène 20,21-dinoréburnaménine,
- le maléate de [16alpha,(±)]-20,21-dinoréburnaménine-15-acétate d'éthyle,
- le maléate acide de [16alpha,(±)] 20,21-dinoréburnaménine, 15-méthanol,
- le maléate acide de [16alpha,(±)]-N,N-diméthyl 20,21-dinoréburnaménine, 15-méthanamine.

**6.** Procédé de préparation des produits de formule (I) telle que définie à la revendication 1, caractérisé en ce que l'on fait réagir un produit de formule (II) :

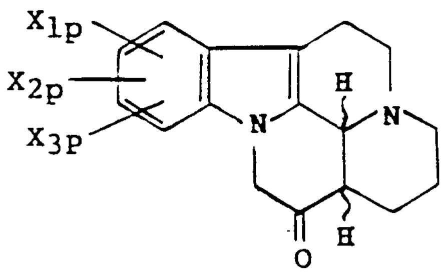

(II)

dans laquelle $X_{1p}$, $X_{2p}$ et $X_{3p}$ représentent $X_1$, $X_2$ et $X_3$ qui ont les significations indiquées à la revendication 1, dans lesquelles les fonctions réactives sont éventuellement protégées,

A) soit avec un halogénure de formule (III) :

R'-V-Hal (III)

dans laquelle V représente un atome de magnésium ou de zinc et Hal représente un atome d'halogène, ou un organométallique de formule (IV) :

R'-W (IV)

dans laquelle W représente un atome de lithium, sodium ou potassium, formules (III) et (IV) dans lesquelles R' représente ou bien les valeurs indiquées à la revendication 1 pour R, sauf si la valeur carboxy libre, salifié ou estérifié, ou bien lesdites valeurs de R dans lesquelles les fonctions réactives sont protégées, pour obtenir un composé de formule (V) :

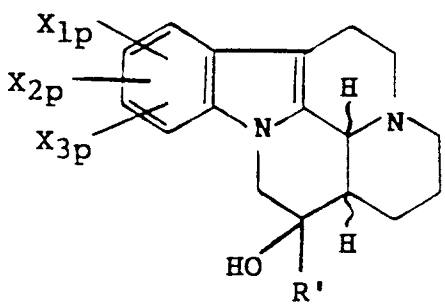

(V)

dans laquelle R' a la signification indiquée ci-dessus, que l'on soumet à une réaction de déshydratation et, si nécessaire, à une réaction d'élimination des groupements protecteurs des fonctions réactives que peuvent porter $X_{1p}$, $X_{2p}$, $X_{3p}$ et R', pour obtenir un composé de formule ($Ia_1$) :

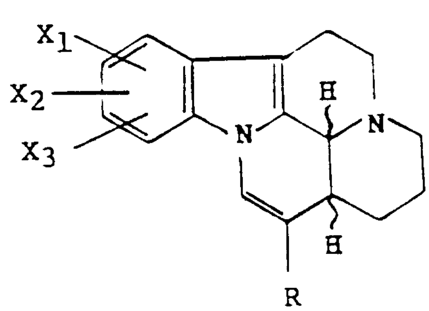

($Ia_1$)

dans laquelle $X_1$, $X_2$, $X_3$ et R ont les significations indiquées ci-dessus, produit de formule ($Ia_1$) que l'on soumet

le cas échéant lorsque R représente un radical $-CH_2OH$, à l'action d'un agent d'oxydation pour obtenir un produit de formule ($Ia_2$) :

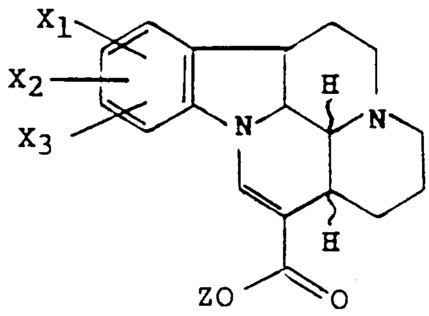

($Ia_2$)

dans laquelle $X_1$, $X_2$, $X_3$ ont les significations indiquées ci-dessus et Z représente un atome d'hydrogène ou le reste d'un groupement ester que l'on soumet si nécessaire ou si désiré à l'une ou aux deux réactions suivantes :

- hydrolyse du groupement ester,
- estérification ou salification par une base de la fonction carboxylique.

B) <u>soit</u> avec un dérivé de triphénylphosphorane de formule (VI) :

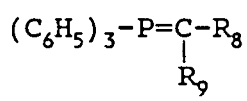

(VI)

ou un phosphonate de formule (VI') :

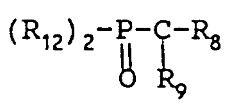

(VI')

dans laquelle $R_{12}$ représente un radical alkyloxy, formules (VI) et (VI') dans lesquelles $R_8$ et $R_9$ ont les significations indiquées à la revendication 1, pour donner selon les conditions opératoires utilisées et éventuellement aprés élimination des groupements protecteurs des fonctions réactives que peuvent porter $X_{1p}$, $X_{2p}$ et $X_{3p}$, un composé de formule ($Ib_1$) :

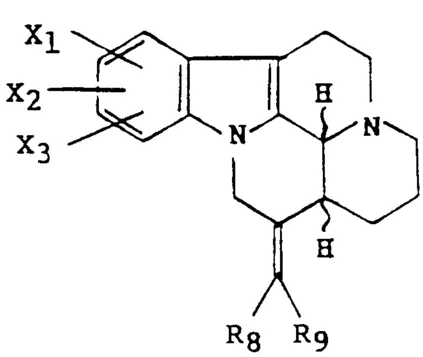

($Ib_1$)

ou un composé de formule ($Ia_3$) :

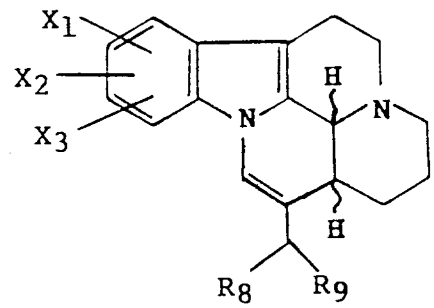

($Ia_3$)

dans lesquelles $X_1$, $X_2$, $X_3$, $R_8$ et $R_9$ ont les significations indiquées à la revendication 1,
C) soit avec un dérivé activé du produit de formule (VII) :

$$R_{10}\text{-C}\equiv\text{CH} \quad \text{(VII)}$$

dans laquelle $R_{10}$ a la signification donnée à la revendication 1, pour obtenir un composé de formule (VIII) :

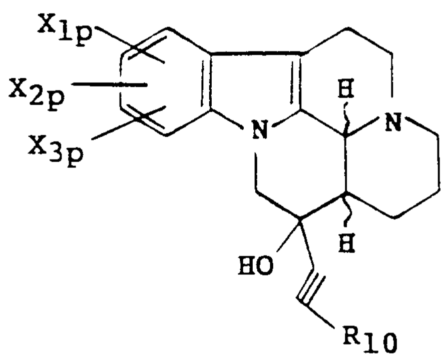

(VIII)

dans laquelle $X_{1p}$, $X_{2p}$, $X_{3p}$ et $R_{10}$ ont les significations indiquées ci-dessus, que l'on soumet, aprés activation du radical hydroxyle et éventuellement protection des fonctions réactives que peut comporter $R_{10}$, à une réaction de réduction, éventuellement d'une réaction d'élimination des groupements protecteurs des fonctions réactives protégées, pour obtenir un composé de formule ($Ib_2$) :

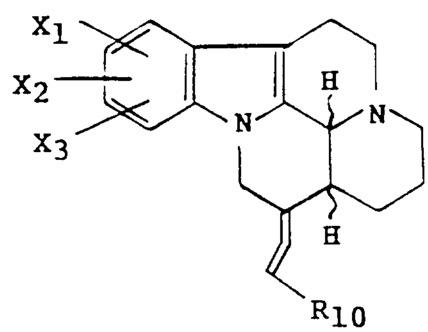

($Ib_2$)

dans laquelle $X_1$, $X_2$, $X_3$ et $R_{10}$ ont les significations indiquées ci-dessus, et traite, si désiré les produits de formules ($Ia_1$), ($Ia_3$), ($Ib_1$) et ($Ib_2$) par un acide minéral ou organique pour obtenir le sel correspondant, lesdits produits de formule (I) étant sous toutes les formes isoméres possibles racémiques ou énantiomères.

**7.** Procédé de préparation des produits de formule (I) telle que définie à la revendication 1 et dans laquelle :

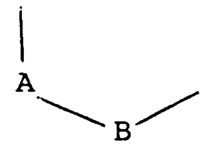

représente le groupement

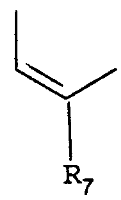

dans lequel $R_7$ représente un radical méthyle éventuellement substitué par un radical hydroxy par un atome d'halogène ou par un radical

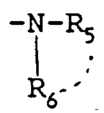

dans lequel $R_5$ et $R_6$ ont la signification indiquée à la revendication 3 ou $R_7$ représente un radical carboxy libre, salifié ou estérifié, caractérisé en ce que l'on fait réagir un produit de formule (II) :

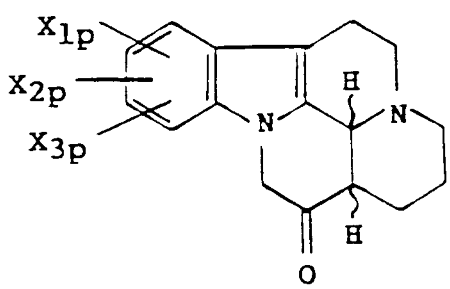

(II)

dans laquelle $X_{1p}$, $X_{2p}$ et $X_{3p}$ ont la signification indiquée à la revendication 6, avec un dérivé halogéné de méthyloxosulfonium de formule (IX) :

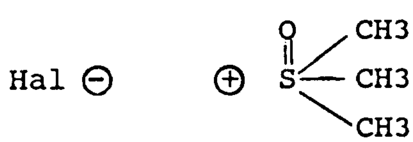

(IX)

en milieu basique, pour obtenir l'époxyde réactionnel correspondant de formule (X) :

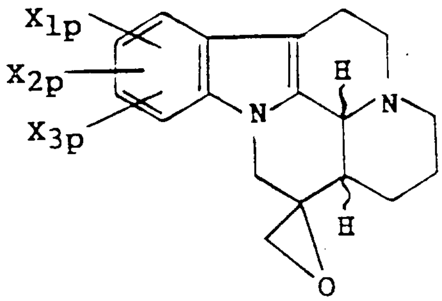

(X)

dans laquelle $X_{1p}$, $X_{2p}$ et $X_{3p}$ ont les significations indiquées ci-dessus, que l'on traite :
**ou bien** par une base pour donner l'alcool correspondant de formule (X') :

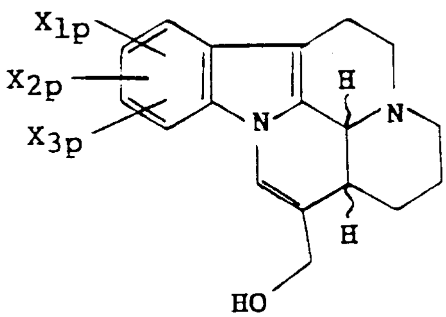

(X')

dans laquelle $X_{1p}$, $X_{2p}$ et $X_{3p}$ ont les significations indiquées ci-dessus, et, si nécessaire, que l'on soumet à une réaction d'élimination des groupements protecteurs des fonctions réactives que peuvent porter $X_{1p}$, $X_{2p}$ et $X_{3p}$, pour donner un produit de formule ($Ia_4$) :

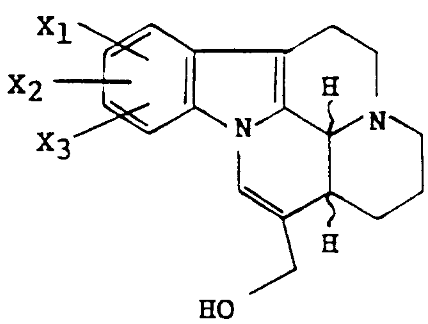

($Ia_4$)

dans laquelle $X_1$, $X_2$, $X_3$ ont les significations indiquées à la revendication 1, puis si nécessaire et si désiré, traite

le produit ($Ia_4$) obtenu à l'aide d'un agent d'oxydation pour obtenir un produit de formule ($Ia_2$) :

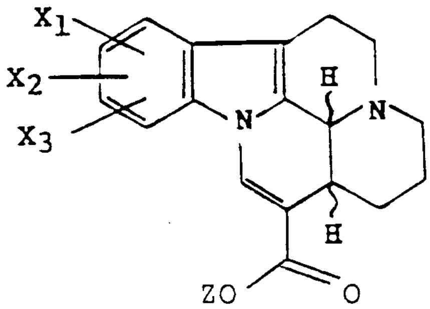

($Ia_2$)

dans laquelle $X_1$, $X_2$, $X_3$ et Z ont les significations indiquées ci-dessus, que l'on soumet si nécessaire et si désiré à l'une ou aux deux réactions suivantes :

- hydrolyse du groupement ester,
- estérification ou salification par une base de la fonction carboxylique,

**<u>ou bien</u>** par une amine de formule (XI) :

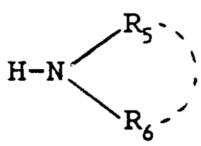

(XI)

dans laquelle $R_5$ et $R_6$ ont la signification indiquée à la revendication 3, pour obtenir le composé de formule (XII) :

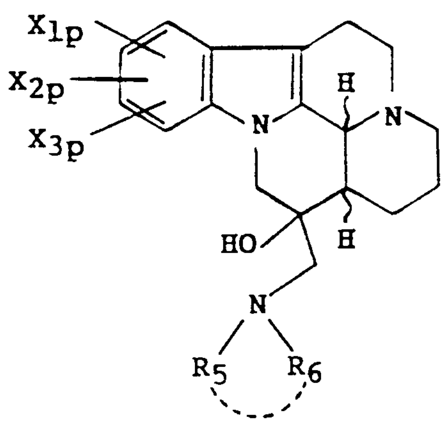

(XII)

dans laquelle $X_{1p}$, $X_{2p}$ et $X_{3p}$ ont les significations indiquées ci-dessus, lesdits composés de formules (X') et (XII) étant soumis aprés activation du radical hydroxyle :

- soit, si désiré dans le cas du composé (X'), à une réaction avec l'amine de formule (XI) telle que définie ci-dessus,
- soit, dans le cas du composé de formule (XII), à une réaction de déshydratation, pour obtenir, dans ces deux cas et aprés élimination, si nécessaire, des groupements protecteurs des fonctions réactives que peuvent porter

$X_{1p}$, $X_{2p}$ et $X_{3p}$, un composé de formule ($Ia_5$) :

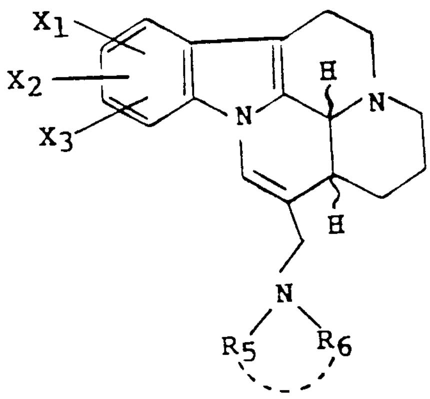

($Ia_5$)

dans laquelle $X_1$, $X_2$, $X_3$, $R_5$ et $R_6$ ont les significations indiquées à la revendication 3,

- ou bien par un halogénure de tétrabutylammonium pour obtenir un produit de formule (XIII) :

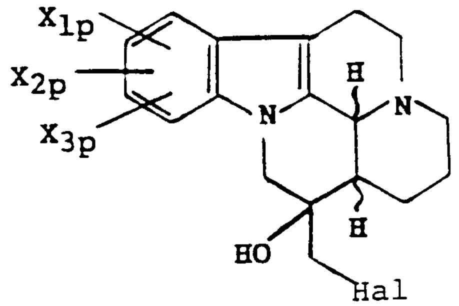

(XIII)

dans laquelle Hal représente un atome d'halogène, puis soumet à une réaction de déshydratation pour obtenir après élimination, si nécessaire, des groupements protecteurs des fonctions réactives que peuvent porter $X_{1p}$, $X_{2p}$ et $X_{3p}$ un composé de formule ($Ia_6$) :

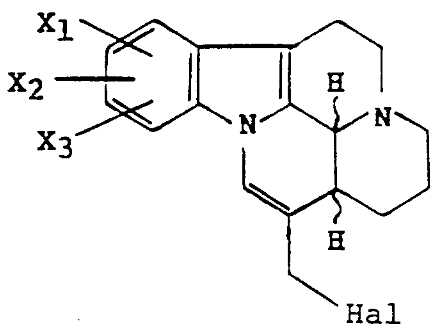

($Ia_6$)

dans lequel $X_1$, $X_2$, $X_3$ et Hal ont la signification indiquée ci-dessus et traite, si désiré le produit de formule ($Ia_3$), ($Ia_5$) et ($Ia_6$) par un acide minéral ou organique, pour obtenir le sel correspondant, lesdits produits de formule (I) étant sous toutes les formes isoméres possibles racémiques ou énantiomères.

**8.** Procédé de préparation des produits de formule (I) telle que définie à la revendication 1 et dans laquelle :

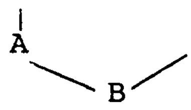

représente le groupement

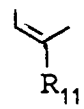

dans lequel $R_{11}$ représente le radical acétylénique de formule :

$$R_{10}\text{-C}\equiv\text{C-}$$

dans laquelle $R_{10}$ a la signification indiquée à la revendication 1, caractérisé en ce que l'on soumet un produit de formule (VIII) :

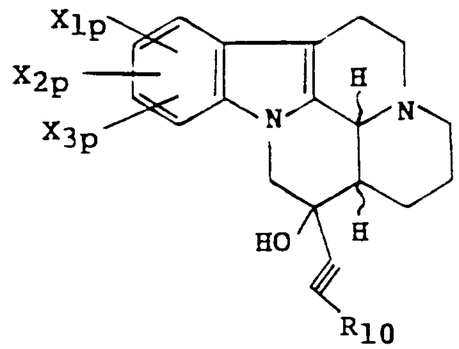

(VIII)

telle que définie à la revendication 6, dans laquelle $X_{1p}$, $X_{2p}$, $X_{3p}$ et $R_{10}$ ont la signification indiquée à la revendication 6, après activation du radical hydroxyle, à une réaction de déshydratation pour obtenir, aprés élimination, si nécessaire et si désiré, des groupements protecteurs des fonctions réactives que peuvent porter $X_{1p}$, $X_{2p}$ et $X_{3p}$, un composé de type ényne de formule
($Ia_7$) :

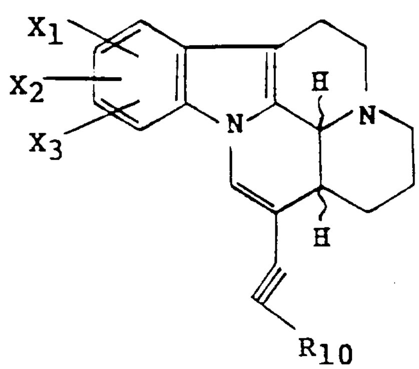

($Ia_7$)

dans laquelle $X_1$, $X_2$, $X_3$ et $R_{10}$ ont les significations indiquées à la revendication 1, et traite, si désiré le produit de formule ($Ia_7$) par un acide minéral ou organique, lesdits produits de formule (I) étant sous toutes les formes isoméres possibles racémiques ou énantiomères.

**9.** A titre de médicaments, les produits tels que définis par la formule (I) de la revendication 1, lesdits produits de formule (I) étant sous toutes les formes isoméres possibles racémiques ou énantiomères, ainsi que les sels d'addition avec les acides minéraux ou organiques ou avec les bases pharmaceutiquement acceptables.

**10.** A titre de médicaments, les produits de formule (I) tels que définis à l'une des revendications 2, 3, 4 ou 5, ainsi que leurs sels pharmaceutiquement acceptables.

**11.** Les compositions pharmaceutiques contenant à titre de principe actif, l'un au moins des médicaments tels que définis à l'une quelconque des revendications 9 et 10.

**12.** A titre de produits industriels nouveaux, les composés de formules (II") :

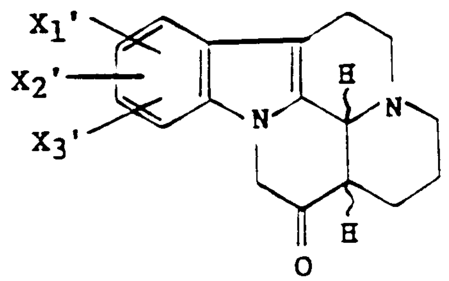

(II'')

dans laquelle $X_1$", $X_2$" et $X_3$" identiques ou différents représentent $X_1$, $X_2$ et $X_3$, à l'exception des produits dans lesquels l'un des substituants $X_1$", $X_2$" et $X_3$"représente un atome d'hydrogène et les deux autres radicaux, identiques ou différents, sont choisis parmi les atomes d'hydrogène ou d'halogène, les radicaux alkyle ou alkyloxy renfermant de 1 à 5 atomes de carbone, un radical hydroxy trifluorométhyle ou nitro.

**13.** A titre de produits industriels nouveaux, les composés de formules (V), (VIII), (X), (X'), (XII) et (XIII) telles que définies aux revendications 6 et 7.

**Revendications pour l'Etat contractant suivant : ES**

**1.** Procédé pour préparer des produits de formule (I) :

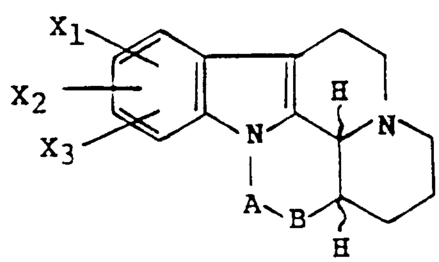

(I)

dans laquelle $X_1$, $X_2$ et $X_3$, identiques ou différents, représentent un atome d'hydrogène, un atome d'halogène, un radical alkyle, alkényle ou alkynyle, ces radicaux étant linéaires ou ramifiés renfermant au plus 18 atomes de carbone et éventuellement substitués, un radical alkyloxy linéaire ou ramifié renfermant au plus 7 atomes de carbone éventuellement substitué, un radical hydroxy, trifluorométhyle ou nitro, un radical amino, mono ou dialkylamino, ou un radical phényle éventuellement substitué,

et dans laquelle

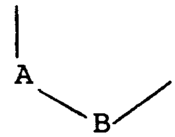

représente

A) soit le groupement

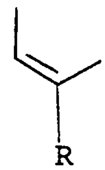

dans lequel R représente :

a) un radical alkyle, alkényle ou alkynyle linéaire ou ramifié renfermant au plus 7 atomes de carbone, ce radical étant éventuellement substitué,
b) un radical phényle éventuellement substitué,
c) un groupement carboxy libre, salifié ou estérifié,

B) soit le groupement

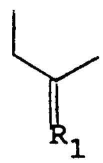

dans lequel $R_1$ représente l'un des deux groupements

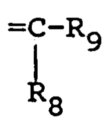 ou 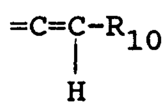

dans lesquels :
$R_8$ et $R_9$, identiques ou différents, sont tels que :

- l'un est choisi dans le groupe formé par :

  a) un atome d'hydrogène,
  b) un radical alkyle, alkényle ou alkynyle linéaire ou ramifié renfermant au plus 6 atomes de carbone, ce radical étant éventuellement substitué,
  c) un radical phényle éventuellement substitué,
  d) un radical carboxy estérifié,
  e) un radical cyano,
  f) un radical acyle ayant de 2 à 6 atomes de carbone,

- et l'autre est choisi dans le groupe formé par :

a) un atome d'hydrogène,
b) un radical alkyle, alkényle ou alkynyle linéaire ou ramifié renfermant au plus 6 atomes de carbone, ce radical étant éventuellement substitué,
c) un radical phényle éventuellement substitué,

et $R_{10}$ représente :

a) un atome d'hydrogène,
b) un radical alkyle, alkényle ou alkynyle linéaire ou ramifié renfermant au plus 5 atomes de carbone, ce radical étant éventuellement substitué,
c) un radical phényle éventuellement substitué,

lesdits produits de formule (I) étant sous toutes les formes isomères possibles racémiques ou optiquement actives, ainsi que les sels d'addition avec les acides minéraux ou organiques ou avec les bases desdits produits de formule (I), caractérisé en ce que l'on fait réagir un produit de formule (II) :

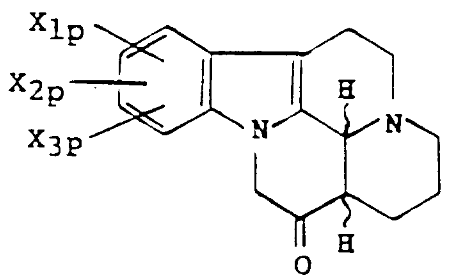

(II)

dans laquelle $X_{1p}$, $X_{2p}$ et $X_{3p}$ représentent $X_1$, $X_2$ et $X_3$ qui ont les significations indiquées à la revendication 1, dans lesquelles les fonctions réactives sont éventuellement protégées,

A) soit avec un halogénure de formule (III) :

R'-V-Hal (III)

dans laquelle V représente un atome de magnésium ou de zinc et Hal représente un atome d'halogène, ou un organométallique de formule (IV) :

R'-W (IV)

dans laquelle W représente un atome de lithium, sodium ou potassium, formules (III) et (IV) dans lesquelles R' représente ou bien les valeurs indiquées à la revendication 1 pour R, sauf si la valeur carboxy libre, salifié ou estérifié, ou bien lesdites valeurs de R dans lesquelles les fonctions réactives sont protégées, pour obtenir un composé de formule (V) :

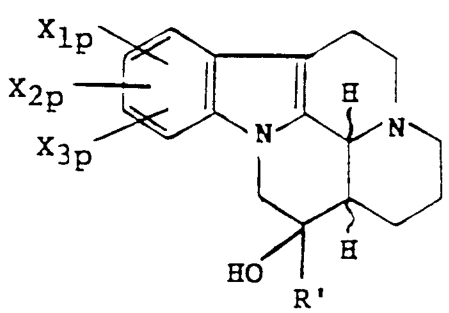

(V)

dans laquelle R' a la signification indiquée ci-dessus, que l'on soumet à une réaction de déshydratation et, si nécessaire, à une réaction d'élimination des groupements protecteurs des fonctions réactives que peuvent

porter $X_{1p}$, $X_{2p}$, $X_{3p}$ et R', pour obtenir un composé de formule ($Ia_1$) :

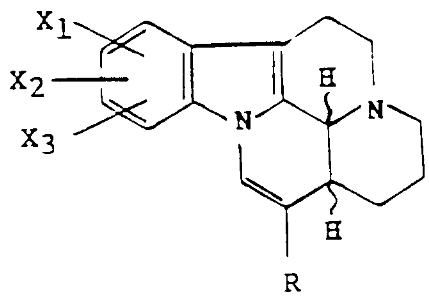

($Ia_1$)

dans laquelle $X_1$, $X_2$, $X_3$ et R ont les significations indiquées ci-dessus, produit de formule ($Ia_1$) que l'on soumet le cas échéant lorsque R représente un radical $-CH_2OH$, à l'action d'un agent d'oxydation pour obtenir un produit de formule ($Ia_2$) :

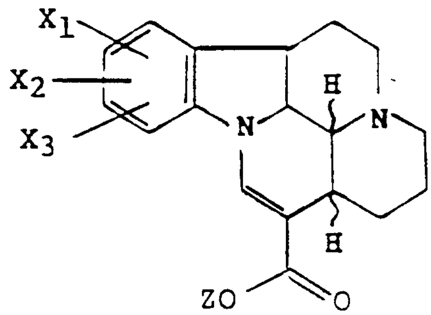

($Ia_2$)

dans laquelle $X_1$, $X_2$, $X_3$ ont les significations indiquées ci-dessus et Z représente un atome d'hydrogène ou le reste d'un groupement ester que l'on soumet si nécessaire ou si désiré à l'une ou aux deux réactions suivantes :

- hydrolyse du groupement ester,
- estérification ou salification par une base de la fonction carboxylique.

B) <u>soit</u> avec un dérivé de triphénylphosphorane de formule (VI) :

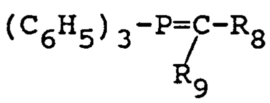

(VI)

ou un phosphonate de formule (VI') :

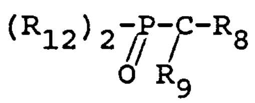

(VI')

dans laquelle $R_{12}$ représente un radical alkyloxy, formules (VI) et (VI') dans lesquelles $R_8$ et $R_9$ ont les significations indiquées ci-dessus, pour donner selon les conditions opératoires utilisées et éventuellement aprés élimination des groupements protecteurs des fonctions réactives que peuvent porter $X_{1p}$, $X_{2p}$ et $X_{3p}$, un com-

posé de formule ($Ib_1$) :

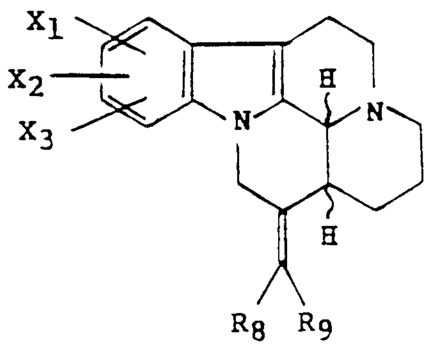

($Ib_1$)

ou un composé de formule ($Ia_3$) :

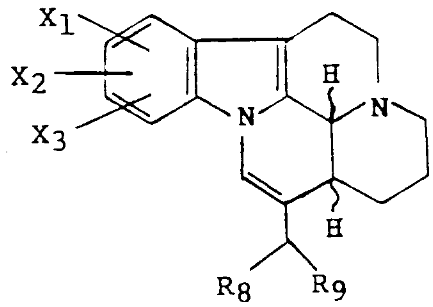

($Ia_3$)

dans lesquelles $X_1$, $X_2$, $X_3$, $R_8$ et $R_9$ ont les significations indiquées à la revendication 1,
C) <u>soit</u> avec un dérivé activé du produit de formule (VII) :

$$R_{10}\text{-}C{\equiv}CH \qquad \text{(VII)}$$

dans laquelle $R_{10}$ a la signification donnée à la revendication 1, pour obtenir un composé de formule (VIII) :

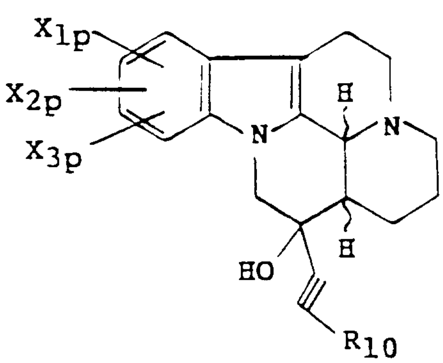

(VIII)

dans laquelle $X_{1p}$, $X_{2p}$, $X_{3p}$ et $R_{10}$ ont les significations indiquées ci-dessus, que l'on soumet, aprés activation du radical radical hydroxyle et éventuellement protection des fonctions réactives que peut comporter $R_{10}$, à une réaction de réduction, éventuellement d'une réaction d'élimination des groupements protecteurs des fonctions

réactives protégées, pour obtenir un composé de formule ($Ib_2$) :

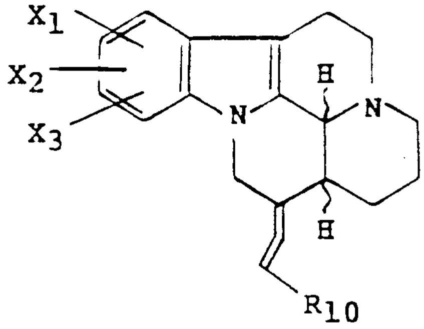

($Ib_2$)

dans laquelle $X_1$, $X_2$, $X_3$ et $R_{10}$ ont les significations indiquées ci-dessus, et traite, si désiré les produits de formules ($Ia_1$), ($Ia_3$), ($Ib_1$) et ($Ib_2$) par un acide minéral ou organique pour obtenir le sel correspondant, lesdits produits de formule (I) étant sous toutes les formes isoméres possibles racémiques ou énantiomères.

**2.** Procédé pour préparer des produits de formule (I) telle que définie à la revendication 1 et dans laquelle :

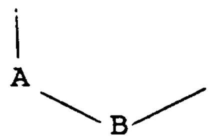

représente le groupement

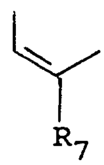

dans lequel $R_7$ représente un radical méthyle éventuellement substitué par un radical hydroxy par un atome d'halogène ou par un radical

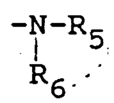

dans lequel $R_5$ et $R_6$, identiques ou différents, représentent :

- un atome d'hydrogène,
- un radical alkyle linéaire ou ramifié renfermant au plus 7 atomes de carbone éventuellement substitué par un ou plusieurs radicaux choisis dans le groupe formé par le radical hydroxy, les radicaux alkyloxy linéaires et ramifiés renfermant au plus 5 atomes de carbone et les radicaux carboxy libres ou estérifiés par un radical alkyle linéaire ou ramifié renfermant au plus 5 atomes de carbone,
- un radical aryle ou arylalkyle renfermant de 7 à 12 atomes de carbone, ces radicaux étant éventuellement substitués,

  **soit** $R_5$ et $R_6$ forment ensemble avec l'atome d'azote auquel ils sont liés un hétérocycle de cinq à six chainons, renfermant éventuellement un second hétéroatome choisi parmi l'oxygène, le soufre ou l'azote, éventuellement substitué sur l'atome d'azote non lié à $R_5$ et $R_6$ par :
- un radical alkyle linéaire ou ramifié renfermant au plus 5 atomes de carbone éventuellement substitué par un radical hydroxy, un atome d'halogène ou un radical alkyloxy renfermant de 1 à 4 atomes de carbone,
- un radical aryle ou un radical arylalkyle renfermant de 7 à 12 atomes de carbone, ces radicaux étant éventuellement substitués, ou $R_7$ représente un radical carboxy libre, salifié ou estérifié, caractérisé en ce que l'on fait

réagir un produit de formule (II) :

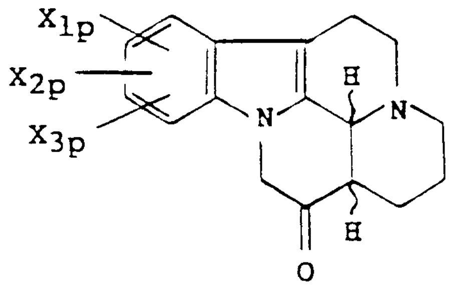

(II)

dans laquelle $X_{1p}$, $X_{2p}$ et $X_{3p}$ ont la signification indiquée à la revendication 1, avec un dérivé halogéné de méthyloxosulfonium de formule (IX) :

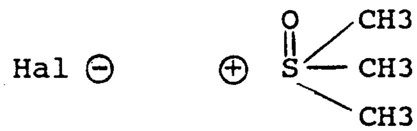

(IX)

en milieu basique, pour obtenir l'époxyde réactionnel correspondant de formule (X) :

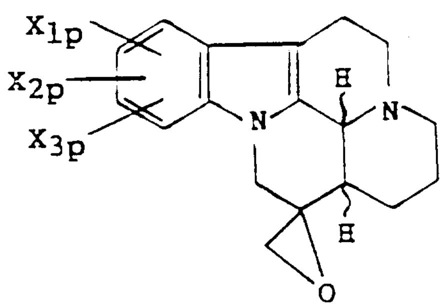

(X)

dans laquelle $X_{1p}$, $X_{2p}$ et $X_{3p}$ ont les significations indiquées ci-dessus, que l'on traite :

**<u>ou bien</u>** par une base pour donner l'alcool correspondant de formule (X') :

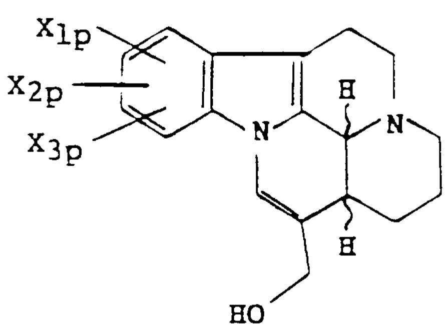

(X')

dans laquelle $X_{1p}$, $X_{2p}$ et $X_{3p}$ ont les significations indiquées ci-dessus, et, si nécessaire, que l'on soumet à une réaction d'élimination des groupements protecteurs des fonctions réactives que peuvent porter $X_{1p}$, $X_{2p}$ et $X_{3p}$, pour

donner un produit de formule ($Ia_4$) :

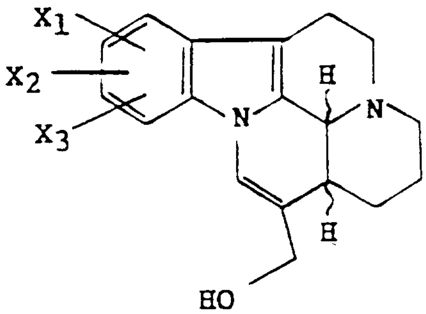

($Ia_4$)

dans laquelle $X_1$, $X_2$, $X_3$ ont les significations indiquées à la revendication 1, puis si nécessaire et si désiré, traite le produit ($Ia_4$) obtenu à l'aide d'un agent d'oxydation pour obtenir un produit de formule ($Ia_2$) :

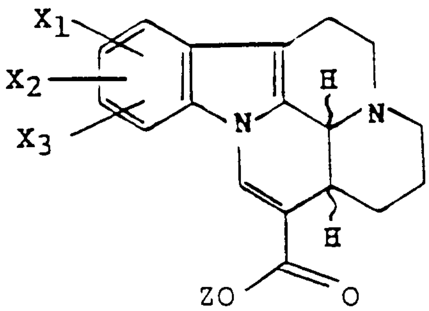

($Ia_2$)

dans laquelle $X_1$, $X_2$, $X_3$ et Z ont les significations indiquées ci-dessus, que l'on soumet si nécessaire et si désiré à l'une ou aux deux réactions suivantes :

- hydrolyse du groupement ester,
- estérification ou salification par une base de la fonction carboxylique,

**ou bien** par une amine de formule (XI) :

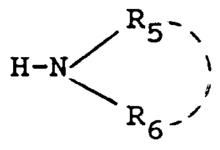

(XI)

dans laquelle $R_5$ et $R_6$ ont la signification indiquée ci-dessus pour obtenir le composé de formule (XII) :

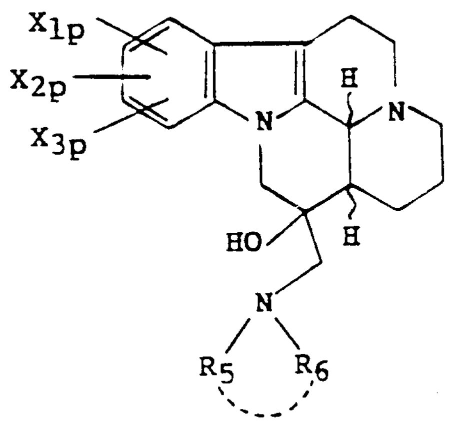

(XII)

dans laquelle $X_{1p}$, $X_{2p}$ et $X_{3p}$ ont les significations indiquées ci-dessus, lesdits composés de formules (X') et (XII) étant soumis aprés activation du radical hydroxyle :

- soit, si désiré dans le cas du composé (X'), à une réaction avec l'amine de formule (XI) telle que définie ci-dessus,
- soit, dans le cas du composé de formule (XII), à une réaction de déshydratation, pour obtenir, dans ces deux cas et aprés élimination, si nécessaire, des groupements protecteurs des fonctions réactives que peuvent porter $X_{1p}$, $X_{2p}$ et $X_{3p}$, un composé de formule ($Ia_5$) :

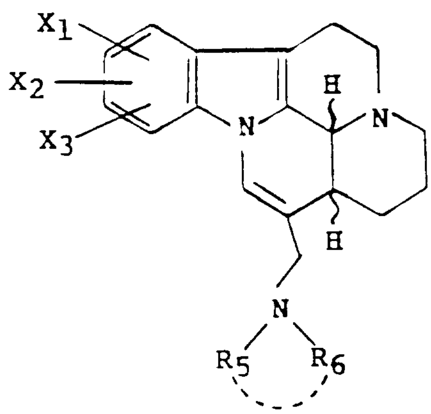

($Ia_5$)

dans laquelle $X_1$, $X_2$, $X_3$, $R_5$ et $R_6$ ont les significations indiquées ci-dessus,

- ou bien par un halogénure de tétrabutylammonium pour obtenir un produit de formule (XIII) :

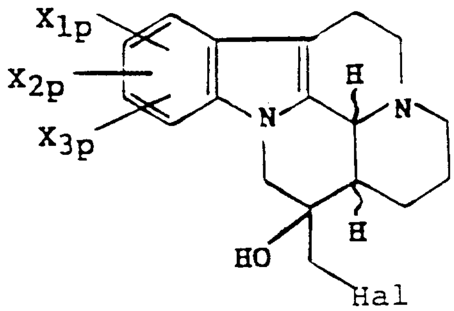

(XIII)

dans laquelle Hal représente un atome d'halogène, puis soumet à une réaction de déshydratation pour obtenir après élimination, si nécessaire, des groupements protecteurs des fonctions réactives que peuvent porter $X_{1p}$, $X_{2p}$ et $X_{3p}$ un composé de formule ($Ia_6$) :

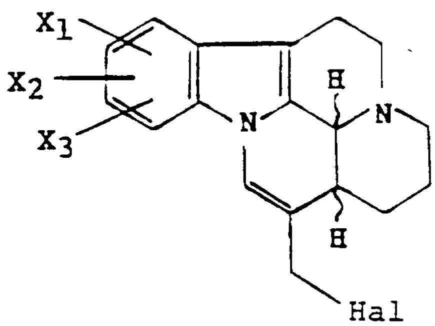

($Ia_6$)

dans lequel $X_1$, $X_2$, $X_3$ et Hal ont la signification indiquée ci-dessus et traite, si désiré le produit de formule ($Ia_3$), ($Ia_5$) et ($Ia_6$) par un acide minéral ou organique, pour obtenir le sel correspondant, lesdits produits de formule (I) étant sous toutes les formes isoméres possibles racémiques ou énantiomères.

**3.** Procédé pour préparer des produits de formule (I) telle que définie à la revendication 1 et dans laquelle :

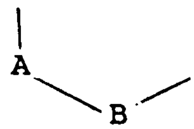

représente le groupement

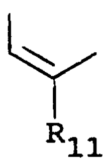

dans lequel $R_{11}$ représente le radical acétylénique de formule :

$R_{10}$-C≡C-

dans laquelle $R_{10}$ a la signification indiquée à la revendication 1, caractérisé en ce que l'on soumet un produit de formule (VIII) :

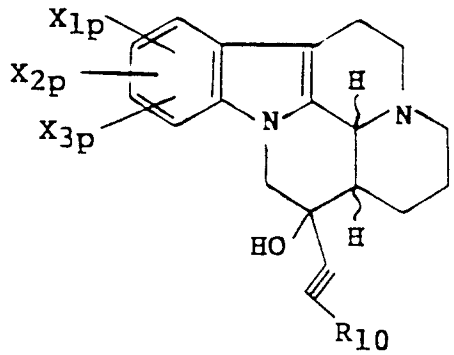

(VIII)

telle que définie à la revendication 1, dans laquelle $X_{1p}$, $X_{2p}$, $X_{3p}$ et $R_{10}$ ont la signification indiquée à la revendication 1, après activation du radical hydroxyle, à une réaction de déshydratation pour obtenir, aprés élimination, si nécessaire et si désiré, des groupements protecteurs des fonctions réactives que peuvent porter $X_{1p}$, $X_{2p}$ et $X_{3p}$, un composé de type ényne de formule ($Ia_7$) :

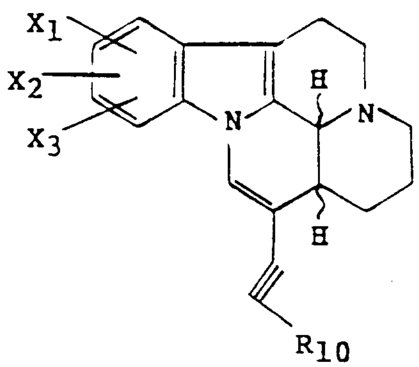

($Ia_7$)

dans laquelle $X_1$, $X_2$, $X_3$ et $R_{10}$ ont les significations indiquées à la revendication 1, et traite, si désiré le produit de formule ($Ia_7$) par un acide minéral ou organique, lesdits produits de formule (I) étant sous toutes les formes isoméres possibles racémiques ou énantiomères.

**4.** Procédé selon la revendication 1 pour préparer des produits de formule (I) telle que définie à la revendication 1 répondant à la formule ($I_A$) :

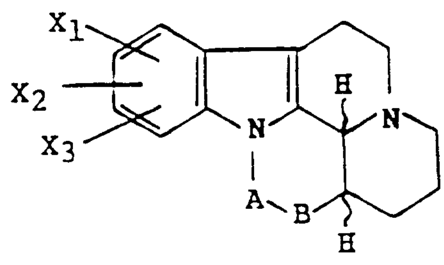

($I_A$)

dans laquelle $X_1$, $X_2$ et $X_3$ ont la signification indiquée à la revendication 1 et

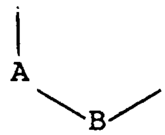

représente soit le groupement

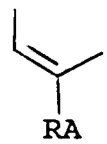

dans lequel RA a la signification indiquée à la revendication 1 pour R à l'exception de la valeur carboxy libre, salifié ou estérifié, soit le groupement

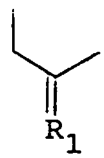

dans lequel $R_1$ a la signification indiquée à la revendication 1, caractérisé en ce que l'on prépare des produits de formule ($Ia_1$), ($Ib_1$), ($Ib_2$), ($Ia_3$) selon le procédé décrit à la revendication 1.

5. Procédé selon la revendication 2 pour préparer des produits de formule ($I_A$) telle que définie à la revendication 4 et dans laquelle RA représente un radical méthyle éventuellement substitué par un radical hydroxy ou par un radical

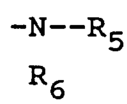

dans lequel $R_5$ et $R_6$ ont la signification indiquée à la revendication 2, caractérisé en ce que l'on prépare des produits de formule ($Ia_4$) ou ($Ia_5$) selon le procédé décrit à la revendication 2.

6. Procédé selon l'une quelconque des revendications 1 à 5, caractérisé en ce que l'on utilise au départ un produit de formule (II) dans laquelle $X_1$ , $X_2$ et $X_3$ représentent un atome d'hydrogène.

7. Procédé selon l'une quelconque des revendications 1 à 5, caractérisé en ce que l'on utilise au départ un produit de formule (II), (III), (IV), (VI), (VI') ou (VII) dans laquelle le ou les substituants que peuvent porter les radicaux alkyle, alkyloxy, alkényle ou alkynyle sont choisis dans le groupe formé par :

- le radical hydroxy,
- les atomes d'halogène,
- les radicaux alkyloxy ayant de 1 à 6 atomes de carbone,
- les radicaux formyle et acyle ayant de 2 à 6 atomes de carbone et le radical benzoyle,
- les radicaux carboxy libres et estérifiés par un radical alkyle linéaire ou ramifié renfermant au plus 5 atomes de carbone,
- le radical cyano,
- le radical carbamoyle éventuellement substitué,
- le radical phényl éventuellement substitué,

- le radical

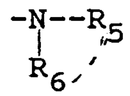

dans lequel :
**soit** $R_5$ et $R_6$, identiques ou différents, représentent :

- un atome d'hydrogène,
- un radical alkyle linéaire ou ramifié renfermant au plus 7 atomes de carbone éventuellement substitué par un ou plusieurs radicaux choisis dans le groupe formé par le radical hydroxy, les radicaux alkyloxy linéaires et ramifiés renfermant au plus 5 atomes de carbone et les radicaux carboxy libres ou estérifiés par un radical alkyle linéaire ou ramifié renfermant au plus 5 atomes de carbone,
- un radical aryle ou arylalkyle renfermant de 7 à 12 atomes de carbone, ces radicaux étant éventuellement substitués,

**soit** $R_5$ et $R_6$ forment ensemble avec l'atome d'azote auquel ils sont liés un hétérocycle de cinq à six chainons, renfermant éventuellement un second hétéroatome choisi parmi l'oxygène, le soufre ou l'azote, éventuellement substitué sur l'atome d'azote non lié à $R_5$ et $R_6$ par :

- un radical alkyle linéaire ou ramifié renfermant au plus 5 atomes de carbone éventuellement substitué par un radical hydroxy, un atome d'halogène ou un radical alkyloxy renfermant de 1 à 4 atomes de carbone,
- un radical aryle ou un radical arylalkyle renfermant de 7 à 12 atomes de carbone, ces radicaux étant éventuellement substitués.

**8.** Procédé selon l'une quelconque des revendications 1 à 5, caractérisé en ce que l'on utilise au départ un produit de formule (II), (III), (IV), (VI), (VI'), (VII) ou (XI) dans laquelle le ou les substituants que peuvent porter les radicaux phényle, aryle et arylalkyle sont choisis dans le groupe formé par les atomes d'halogène, le radical hydroxy, les radicaux alkyle et alkyloxy linéaires et ramifiés renfermant au plus 5 atomes de carbone, les radicaux méthylthio, nitro, amino, monoalkyl- et dialkylamino.

**Revendications pour l'Etat contractant suivant : GR**

**1.** Procédé pour préparer des produits de formule (I) :

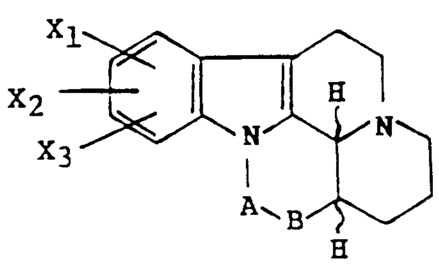

(I)

dans laquelle $X_1$, $X_2$ et $X_3$, identiques ou différents, représentent un atome d'hydrogène, un atome d'halogène, un radical alkyle, alkényle ou alkynyle, ces radicaux étant linéaires ou ramifiés renfermant au plus 18 atomes de carbone et éventuellement substitués, un radical alkyloxy linéaire ou ramifié renfermant au plus 7 atomes de carbone éventuellement substitué, un radical hydroxy, trifluorométhyle ou nitro, un radical amino, mono ou dialkylamino, ou un radical phényle éventuellement substitué,

et dans laquelle

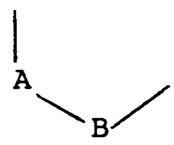

représente

A) soit le groupement

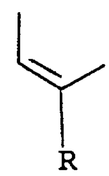

dans lequel R représente :

a) un radical alkyle, alkényle ou alkynyle linéaire ou ramifié renfermant au plus 7 atomes de carbone, ce radical étant éventuellement substitué,
b) un radical phényle éventuellement substitué,
c) un groupement carboxy libre, salifié ou estérifié,

B) soit le groupement

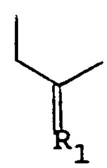

dans lequel $R_1$ représente l'un des deux groupements

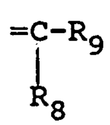

ou

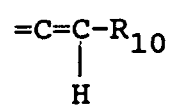

dans lesquels :
$R_8$ et $R_9$, identiques ou différents, sont tels que :

- l'un est choisi dans le groupe formé par :

  a) un atome d'hydrogène,
  b) un radical alkyle, alkényle ou alkynyle linéaire ou ramifié renfermant au plus 6 atomes de carbone, ce radical étant éventuellement substitué,
  c) un radical phényle éventuellement substitué,
  d) un radical carboxy estérifié,
  e) un radical cyano,
  f) un radical acyle ayant de 2 à 6 atomes de carbone,

- et l'autre est choisi dans le groupe formé par :

a) un atome d'hydrogène,
b) un radical alkyle, alkényle ou alkynyle linéaire ou ramifié renfermant au plus 6 atomes de carbone, ce radical étant éventuellement substitué,
c) un radical phényle éventuellement substitué,

et $R_{10}$ représente :

a) un atome d'hydrogène,
b) un radical alkyle, alkényle ou alkynyle linéaire ou ramifié renfermant au plus 5 atomes de carbone, ce radical étant éventuellement substitué,
c) un radical phényle éventuellement substitué,

lesdits produits de formule (I) étant sous toutes les formes isomères possibles racémiques ou optiquement actives, ainsi que les sels d'addition avec les acides minéraux ou organiques ou avec les bases desdits produits de formule (I), caractérisé en ce que l'on fait réagir un produit de formule (II) :

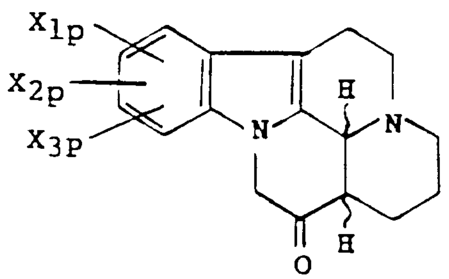

(II)

dans laquelle $X_{1p}$, $X_{2p}$ et $X_{3p}$ représentent $X_1$, $X_2$ et $X_3$ qui ont les significations indiquées à la revendication 1, dans lesquelles les fonctions réactives sont éventuellement protégées,

A) soit avec un halogénure de formule (III) :

R'-V-Hal (III)

dans laquelle V représente un atome de magnésium ou de zinc et Hal représente un atome d'halogène, ou un organométallique de formule (IV) :

R'-W (IV)

dans laquelle W représente un atome de lithium, sodium ou potassium, formules (III) et (IV) dans lesquelles R' représente ou bien les valeurs indiquées à la revendication 1 pour R, sauf si la valeur carboxy libre, salifié ou estérifié, ou bien lesdites valeurs de R dans lesquelles les fonctions réactives sont protégées, pour obtenir un composé de formule (V) :

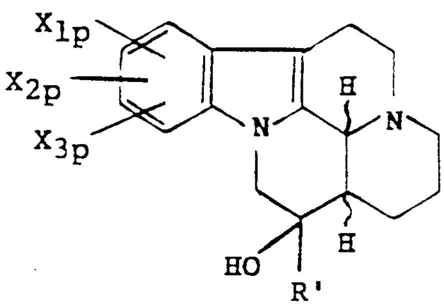

(V)

dans laquelle R' a la signification indiquée ci-dessus, que l'on soumet à une réaction de déshydratation et, si nécessaire, à une réaction d'élimination des groupements protecteurs des fonctions réactives que peuvent

porter $X_{1p}$, $X_{2p}$, $X_{3p}$ et R', pour obtenir un composé de formule ($Ia_1$) :

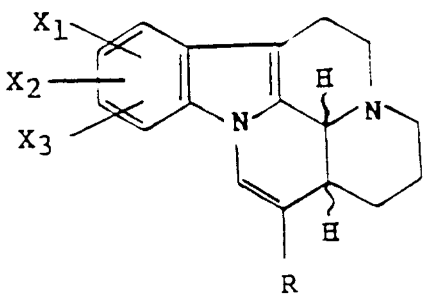

($Ia_1$)

dans laquelle $X_1$, $X_2$, $X_3$ et R ont les significations indiquées ci-dessus, produit de formule ($Ia_1$) que l'on soumet le cas échéant lorsque R représente un radical -$CH_2OH$, à l'action d'un agent d'oxydation pour obtenir un produit de formule ($Ia_2$) :

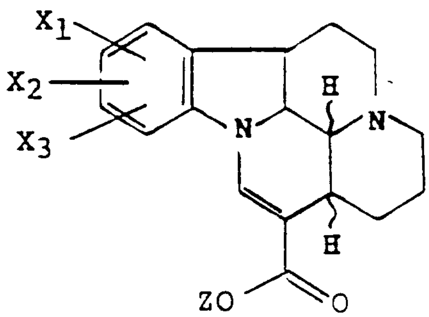

($Ia_2$)

dans laquelle $X_1$, $X_2$, $X_3$ ont les significations indiquées ci-dessus et Z représente un atome d'hydrogène ou le reste d'un groupement ester que l'on soumet si nécessaire ou si désiré à l'une ou aux deux réactions suivantes :

- hydrolyse du groupement ester,
- estérification ou salification par une base de la fonction carboxylique.

B) <u>soit</u> avec un dérivé de triphénylphosphorane de formule (VI) :

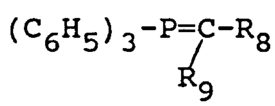

(VI)

ou un phosphonate de formule (VI') :

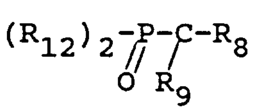

(VI')

dans laquelle $R_{12}$ représente un radical alkyloxy, formules (VI) et (VI') dans lesquelles $R_8$ et $R_9$ ont les significations indiquées ci-dessus, pour donner selon les conditions opératoires utilisées et éventuellement aprés élimination des groupements protecteurs des fonctions réactives que peuvent porter $X_{1p}$, $X_{2p}$ et $X_{3p}$, un com-

posé de formule ($Ib_1$) :

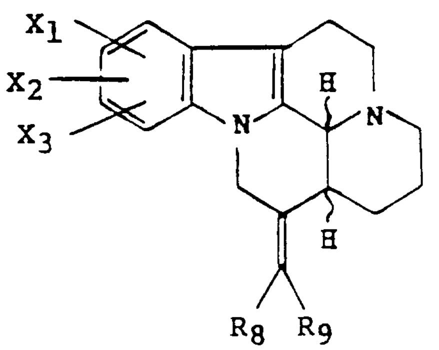

($Ib_1$)

ou un composé de formule ($Ia_3$) :

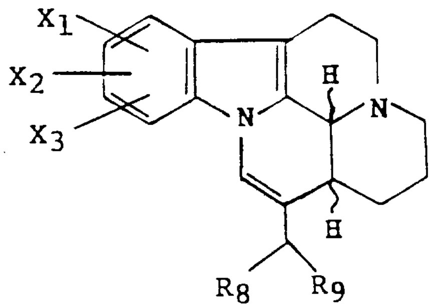

($Ia_3$)

dans lesquelles $X_1$, $X_2$, $X_3$, $R_8$ et $R_9$ ont les significations indiquées à la revendication 1,

C) soit avec un dérivé activé du produit de formule (VII) :

$$R_{10}\text{-}C{\equiv}CH \qquad \text{(VII)}$$

dans laquelle $R_{10}$ a la signification donnée à la revendication 1, pour obtenir un composé de formule (VIII) :

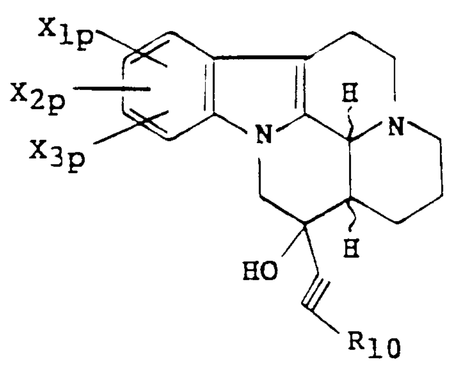

(VIII)

dans laquelle $X_{1p}$, $X_{2p}$, $X_{3p}$ et $R_{10}$ ont les significations indiquées ci-dessus, que l'on soumet, aprés activation du radical radical hydroxyle et éventuellement protection des fonctions réactives que peut comporter $R_{10}$, à une réaction de réduction, éventuellement d'une réaction d'élimination des groupements protecteurs des fonctions

réactives protégées, pour obtenir un composé de formule ($Ib_2$) :

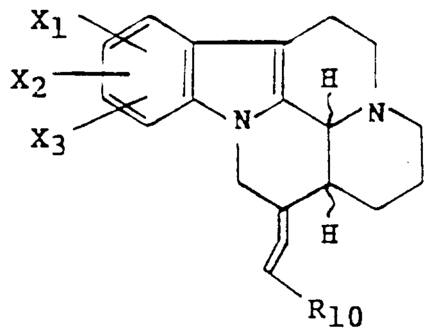

($Ib_2$)

dans laquelle $X_1$, $X_2$, $X_3$ et $R_{10}$ ont les significations indiquées ci-dessus, et traite, si désiré les produits de formules ($Ia_1$), ($Ia_3$), ($Ib_1$) et ($Ib_2$) par un acide minéral ou organique pour obtenir le sel correspondant, lesdits produits de formule (I) étant sous toutes les formes isoméres possibles racémiques ou énantiomères.

**2.** Procédé pour préparer des produits de formule (I) telle que définie à la revendication 1 et dans laquelle :

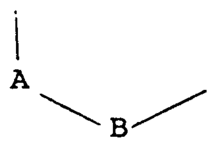

représente le groupement

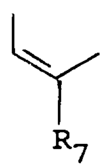

dans lequel $R_7$ représente un radical méthyle éventuellement substitué par un radical hydroxy par un atome d'halogène ou par un radical

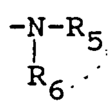

dans lequel $R_5$ et $R_6$, identiques ou différents, représentent :

- un atome d'hydrogène,
- un radical alkyle linéaire ou ramifié renfermant au plus 7 atomes de carbone éventuellement substitué par un ou plusieurs radicaux choisis dans le groupe formé par le radical hydroxy, les radicaux alkyloxy linéaires et ramifiés renfermant au plus 5 atomes de carbone et les radicaux carboxy libres ou estérifiés par un radical alkyle linéaire ou ramifié renfermant au plus 5 atomes de carbone,
- un radical aryle ou arylalkyle renfermant de 7 à 12 atomes de carbone, ces radicaux étant éventuellement substitués,

  **soit** $R_5$ et $R_6$ forment ensemble avec l'atome d'azote auquel ils sont liés un hétérocycle de cinq à six chainons, renfermant éventuellement un second hétéroatome choisi parmi l'oxygène, le soufre ou l'azote, éventuellement substitué sur l'atome d'azote non lié à $R_5$ et $R_6$ par :
- un radical alkyle linéaire ou ramifié renfermant au plus 5 atomes de carbone éventuellement substitué par un radical hydroxy, un atome d'halogène ou un radical alkyloxy renfermant de 1 à 4 atomes de carbone,
- un radical aryle ou un radical arylalkyle renfermant de 7 à 12 atomes de carbone, ces radicaux étant éventuellement substitués, ou $R_7$ représente un radical carboxy libre, salifié ou estérifié, caractérisé en ce que l'on fait

réagir un produit de formule (II) :

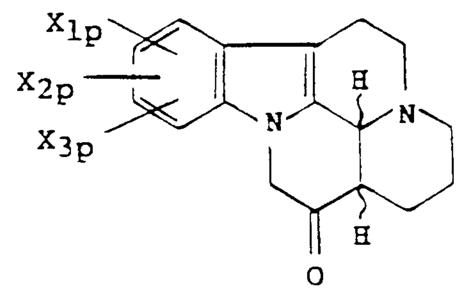

(II)

dans laquelle $X_{1p}$, $X_{2p}$ et $X_{3p}$ ont la signification indiquée à la revendication 1, avec un dérivé halogéné de méthyloxosulfonium de formule (IX) :

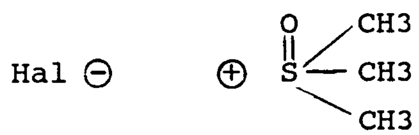

(IX)

en milieu basique, pour obtenir l'époxyde réactionnel correspondant de formule (X) :

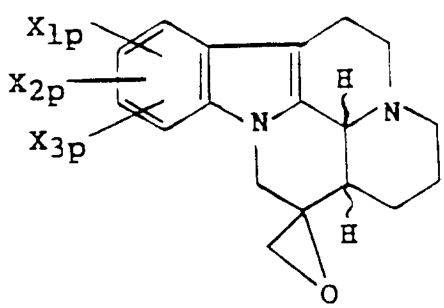

(X)

dans laquelle $X_{1p}$, $X_{2p}$ et $X_{3p}$ ont les significations indiquées ci-dessus, que l'on traite :

**<u>ou bien</u>** par une base pour donner l'alcool correspondant de formule (X') :

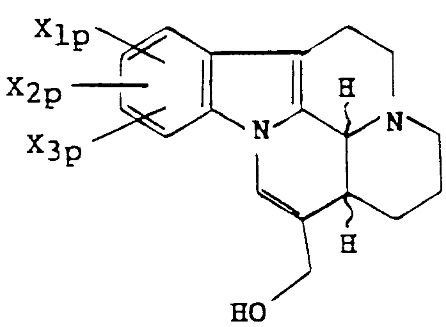

(X')

dans laquelle $X_{1p}$, $X_{2p}$ et $X_{3p}$ ont les significations indiquées ci-dessus, et, si nécessaire, que l'on soumet à une réaction d'élimination des groupements protecteurs des fonctions réactives que peuvent porter $X_{1p}$, $X_{2p}$ et $X_{3p}$, pour

donner un produit de formule ($Ia_4$) :

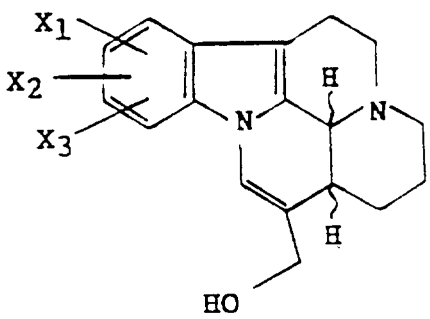

($Ia_4$)

dans laquelle $X_1$, $X_2$, $X_3$ ont les significations indiquées à la revendication 1, puis si nécessaire et si désiré, traite le produit ($Ia_4$) obtenu à l'aide d'un agent d'oxydation pour obtenir un produit de formule ($Ia_2$) :

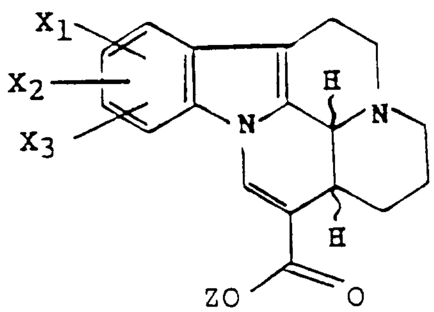

($Ia_2$)

dans laquelle $X_1$, $X_2$, $X_3$ et Z ont les significations indiquées ci-dessus, que l'on soumet si nécessaire et si désiré à l'une ou aux deux réactions suivantes :

- hydrolyse du groupement ester,
- estérification ou salification par une base de la fonction carboxylique,

**ou bien** par une amine de formule (XI) :

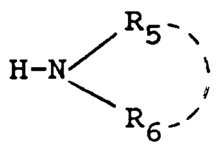

(XI)

dans laquelle $R_5$ et $R_6$ ont la signification indiquée ci-dessus pour obtenir le composé de formule (XII) :

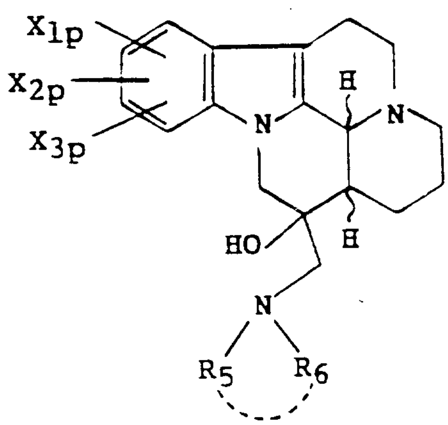

(XII)

dans laquelle $X_{1p}$, $X_{2p}$ et $X_{3p}$ ont les significations indiquées ci-dessus, lesdits composés de formules (X') et (XII) étant soumis aprés activation du radical hydroxyle :

- soit, si désiré dans le cas du composé (X'), à une réaction avec l'amine de formule (XI) telle que définie ci-dessus,
- soit, dans le cas du composé de formule (XII), à une réaction de déshydratation, pour obtenir, dans ces deux cas et aprés élimination, si nécessaire, des groupements protecteurs des fonctions réactives que peuvent porter $X_{1p}$, $X_{2p}$ et $X_{3p}$, un composé de formule ($Ia_5$) :

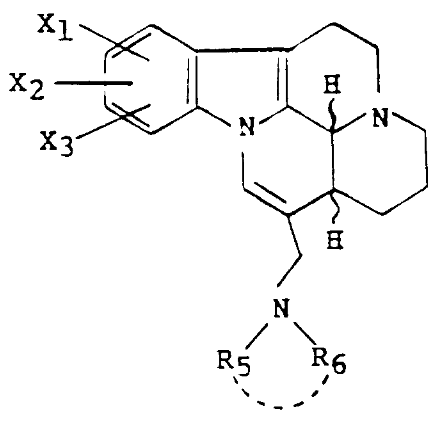

($Ia_5$)

dans laquelle $X_1$, $X_2$, $X_3$, $R_5$ et $R_6$ ont les significations indiquées ci-dessus,

- ou bien par un halogénure de tétrabutylammonium pour obtenir un produit de formule (XIII) :

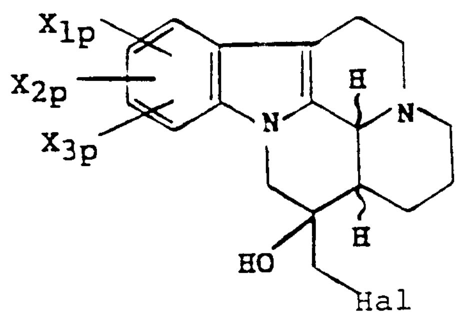

(XIII)

dans laquelle Hal représente un atome d'halogène, puis soumet à une réaction de déshydratation pour obtenir après élimination, si nécessaire, des groupements protecteurs des fonctions réactives que peuvent porter $X_{1p}$, $X_{2p}$ et $X_{3p}$ un composé de formule ($Ia_6$) :

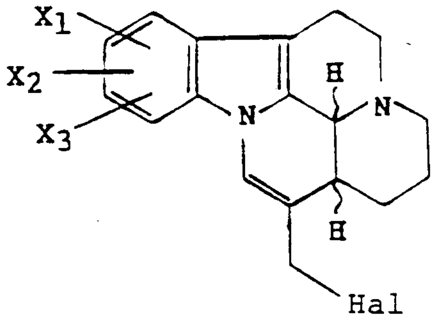

($Ia_6$)

dans lequel $X_1$, $X_2$, $X_3$ et Hal ont la signification indiquée ci-dessus et traite, si désiré le produit de formule ($Ia_3$), ($Ia_5$) et ($Ia_6$) par un acide minéral ou organique, pour obtenir le sel correspondant, lesdits produits de formule (I) étant sous toutes les formes isoméres possibles racémiques ou énantiomères.

**3.** Procédé pour préparer des produits de formule (I) telle que définie à la revendication 1 et dans laquelle :

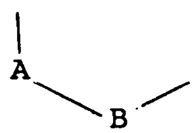

représente le groupement

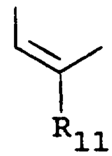

dans lequel $R_{11}$ représente le radical acétylénique de formule :

$$R_{10}\text{-}C\equiv C\text{-}$$

dans laquelle $R_{10}$ a la signification indiquée à la revendication 1, caractérisé en ce que l'on soumet un produit de formule (VIII) :

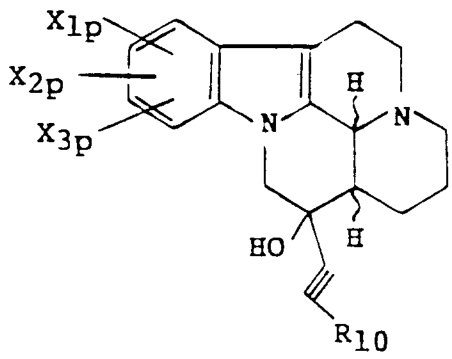

(VIII)

telle que définie à la revendication 1, dans laquelle $X_{1p}$, $X_{2p}$, $X_{3p}$ et $R_{10}$ ont la signification indiquée à la revendication 1, après activation du radical hydroxyle, à une réaction de déshydratation pour obtenir, aprés élimination, si nécessaire et si désiré, des groupements protecteurs des fonctions réactives que peuvent porter $X_{1p}$, $X_{2p}$ et $X_{3p}$, un composé de type ényne de formule ($Ia_7$) :

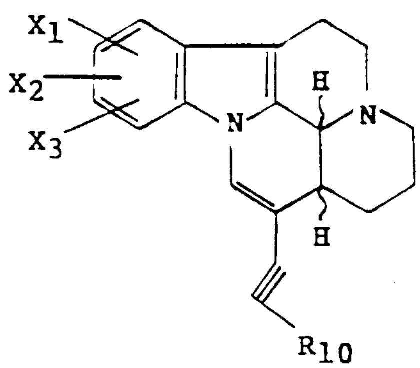

($Ia_7$)

dans laquelle $X_1$, $X_2$, $X_3$ et $R_{10}$ ont les significations indiquées à la revendication 1, et traite, si désiré le produit de formule ($Ia_7$) par un acide minéral ou organique, lesdits produits de formule (I) étant sous toutes les formes isoméres possibles racémiques ou énantiomères.

4. Procédé selon la revendication 1 pour préparer des produits de formule (I) telle que définie à la revendication 1 répondant à la formule ($I_A$) :

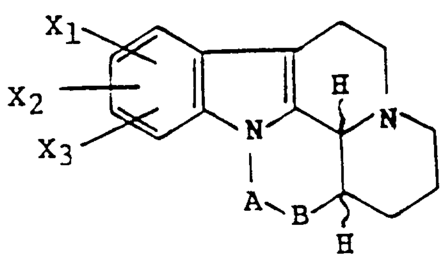

($I_A$)

dans laquelle $X_1$, $X_2$ et $X_3$ ont la signification indiquée à la revendication 1 et

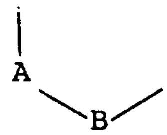

représente soit le groupement

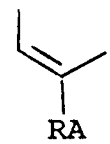

dans lequel RA a la signification indiquée à la revendication 1 pour R à l'exception de la valeur carboxy libre, salifié ou estérifié, soit le groupement

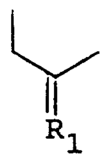

dans lequel $R_1$ a la signification indiquée à la revendication 1, caractérisé en ce que l'on prépare des produits de formule ($Ia_1$), ($Ib_1$), ($Ib_2$), ($Ia_3$) selon le procédé décrit à la revendication 1.

5. Procédé selon la revendication 2 pour préparer des produits de formule ($I_A$) telle que définie à la revendication 4 et dans laquelle RA représente un radical méthyle éventuellement substitué par un radical hydroxy ou par un radical

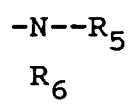

dans lequel $R_5$ et $R_6$ ont la signification indiquée à la revendication 2, caractérisé en ce que l'on prépare des produits de formule ($Ia_4$) ou ($Ia_5$) selon le procédé décrit à la revendication 2.

6. Procédé selon l'une quelconque des revendications 1 à 5, caractérisé en ce que l'on utilise au départ un produit de formule (II) dans laquelle $X_1$ , $X_2$ et $X_3$ représentent un atome d'hydrogène.

7. Procédé selon l'une quelconque des revendications 1 à 5, caractérisé en ce que l'on utilise au départ un produit de formule (II), (III), (IV), (VI), (VI') ou (VII) dans laquelle le ou les substituants que peuvent porter les radicaux alkyle, alkyloxy, alkényle ou alkynyle sont choisis dans le groupe formé par :

- le radical hydroxy,
- les atomes d'halogène,
- les radicaux alkyloxy ayant de 1 à 6 atomes de carbone,
- les radicaux formyle et acyle ayant de 2 à 6 atomes de carbone et le radical benzoyle,
- les radicaux carboxy libres et estérifiés par un radical alkyle linéaire ou ramifié renfermant au plus 5 atomes de carbone,
- le radical cyano,
- le radical carbamoyle éventuellement substitué,
- le radical phényl éventuellement substitué,

- le radical

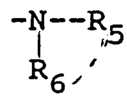

dans lequel :

**soit** $R_5$ et $R_6$, identiques ou différents, représentent :

- un atome d'hydrogène,
- un radical alkyle linéaire ou ramifié renfermant au plus 7 atomes de carbone éventuellement substitué par un ou plusieurs radicaux choisis dans le groupe formé par le radical hydroxy, les radicaux alkyloxy linéaires et ramifiés renfermant au plus 5 atomes de carbone et les radicaux carboxy libres ou estérifiés par un radical alkyle linéaire ou ramifié renfermant au plus 5 atomes de carbone,
- un radical aryle ou arylalkyle renfermant de 7 à 12 atomes de carbone, ces radicaux étant éventuellement substitués,

**soit** $R_5$ et $R_6$ forment ensemble avec l'atome d'azote auquel ils sont liés un hétérocycle de cinq à six chainons, renfermant éventuellement un second hétéroatome choisi parmi l'oxygène, le soufre ou l'azote, éventuellement substitué sur l'atome d'azote non lié à $R_5$ et $R_6$ par :

- un radical alkyle linéaire ou ramifié renfermant au plus 5 atomes de carbone éventuellement substitué par un radical hydroxy, un atome d'halogène ou un radical alkyloxy renfermant de 1 à 4 atomes de carbone,
- un radical aryle ou un radical arylalkyle renfermant de 7 à 12 atomes de carbone, ces radicaux étant éventuellement substitués.

**8.** Procédé selon l'une quelconque des revendications 1 à 5, caractérisé en ce que l'on utilise au départ un produit de formule (II), (III), (IV), (VI), (VI'), (VII) ou (XI) dans laquelle le ou les substituants que peuvent porter les radicaux phényle, aryle et arylalkyle sont choisis dans le groupe formé par les atomes d'halogène, le radical hydroxy, les radicaux alkyle et alkyloxy linéaires et ramifiés renfermant au plus 5 atomes de carbone, les radicaux méthylthio, nitro, amino, monoalkyl- et dialkylamino.

**9.** Procédé selon la revendication 4, caractérisé en ce que l'on prépare l'un quelconque des produits de formule (I) dont les noms suivent :

- l'oxalate de [16alpha,(±)]-15-(1-propynyl) -20,21-dinoréburnaménine,
- le [16alpha,(±)]-15-méthyl 20,21-dinoréburnaménine,
- le maléate acide de [16alpha,(±)]-14,15-dihydro 15-méthylène 20,21-dinoréburnaménine,
- le maléate de [16alpha,(±)]-20,21-dinoréburnaménine-15-acétate d'éthyle,
- le maléate acide de [16alpha,(±)] 20,21-dinoréburnaménine, 15-méthanol,
- le maléate acide de [16alpha,(±)]-N,N-diméthyl 20,21-dinoréburnaménine, 15-méthanamine.

**10.** Procédé de préparation de compositions pharmaceutiques caractérisé en ce que l'on met à titre de principe actif l'un au moins des produits de formule (I), telle que définie à la revendication 1 ou l'un au moins de ses sels avec les acides ou les bases pharmaceutiquement acceptables, sous une forme destinée à cet usage.

**11.** Procédé de préparation de compositions pharmaceutiques caractérisé en ce que l'on met à titre de principe actif l'un au moins des produits de formule ($I_A$), telle que définie à la revendication 4 ou l'un au moins de ses sels avec les acides ou les bases pharmaceutiquement acceptables, sous une forme destinée à cet usage.

**12.** Procédé de préparation de compositions pharmaceutiques caractérisé en ce que l'on met à titre de principe actif l'un au moins des produits de formule (I), cités à la revendication 9 ou le cas échéant l'un au moins de ses sels avec les acides ou les bases pharmaceutiquement acceptables, sous une forme destinée à cet usage.

**13.** A titre de produits industriels nouveaux, les composés de formules (V), (VIII), (X), (X'), (XII) et (XIII) telles que définies aux revendications 1 et 2.

## Claims

**Claims for the following Contracting States : AT, BE, CH, DE, DK, FR, GB, IT, LI, LU, NL, SE**

**1.** Products of formula (I):

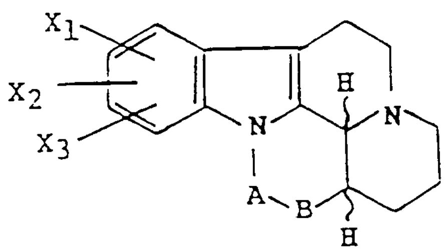

(I)

in which $X_1$, $X_2$ and $X_3$, identical or different, represent a hydrogen atom, a halogen atom, an alkyl, alkenyl or alkynyl radical, these radicals being linear or branched, containing at most 18 carbon atoms and optionally substituted, an optionally substituted linear or branched alkyloxy radical containing at most 7 carbon atoms, a hydroxy, trifluoromethyl or nitro radical, an amino, mono or dialkylamino radical, or an optionally substituted phenyl radical, and in which

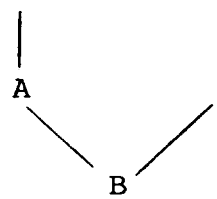

represents

A) either the group

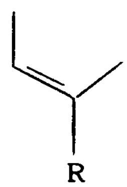

in which R represents:

a) a linear or branched alkyl, alkenyl or alkynyl radical containing at most 7 carbon atoms, this radical being optionally substituted,
b) an optionally substituted phenyl radical,
c) a free, salified or esterified carboxy group,

B) or the group

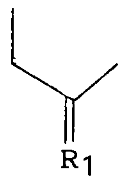

in which $R_1$ represents one of the two groups

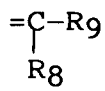

or

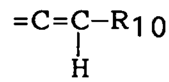

in which:
$R_8$ and $R_9$, identical or different, are such that:

- one is chosen from the group formed by:

  a) a hydrogen atom,
  b) a linear or branched alkyl, alkenyl or alkynyl radical containing at most 6 carbon atoms, this radical being optionally substituted,
  c) an optionally substituted phenyl radical,
  d) an esterified carboxy radical,
  e) a cyano radical,
  f) an acyl radical having 2 to 6 carbon atoms,

- and the other is chosen from the group formed by:

  a) a hydrogen atom,
  b) a linear or branched alkyl, alkenyl or alkynyl radical containing at most 6 carbon atoms, this radical being optionally substituted,
  c) an optionally substituted phenyl radical,

and $R_{10}$ represents:

a) a hydrogen atom,
b) a linear or branched alkyl, alkenyl or alkynyl radical containing at most 5 carbon atoms, this radical being optionally substituted,
c) an optionally substituted phenyl radical,

the said products of formula (I) being in all the possible racemic or optically active isomer forms, as well as the addition salts with mineral or organic acids or with the bases of the said products of formula (I).

**2.** Products of formula (I) as defined in claim 1, characterized in that $X_1$, $X_2$ and $X_3$ represent a hydrogen atom, the said products of formula (I) being in all the possible racemic or optically active isomer forms, as well as the addition salts with mineral or organic acids or with bases of the said products of formula (I).

**3.** Products of formula (I) as defined in claim 1, characterized in that the substituent or substituents that can be carried by the alkyl, alkyloxy, alkenyl or alkynyl radicals are chosen from the group formed by:

- the hydroxy radical,
- halogen atoms,
- alkyloxy radicals having 1 to 6 carbon atoms,
- formyl and acyl radicals having 2 to 6 carbon atoms and the benzoyl radical,
- free carboxy radicals and carboxy radicals esterified by a linear or branched alkyl radical containing at most 5 carbon atoms,

- the cyano radical,
- the optionally substituted carbamoyl radical,
- the optionally substituted phenyl radical,
- the radical

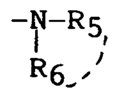

in which:
either $R_5$ and $R_6$, identical or different, represent:
- a hydrogen atom,
- a linear or branched alkyl radical containing at most 7 carbon atoms optionally substituted by one or more radicals chosen from the group formed by the hydroxy radical, linear and branched alkyloxy radicals containing at most 5 carbon atoms and carboxy radicals, free, or esterified by a linear or branched alkyl radical containing at most 5 carbon atoms,
- an aryl or arylalkyl radical containing 7 to 12 carbon atoms, these radicals being optionally substituted,

or $R_5$ and $R_6$ form, together with the nitrogen atom to which they are linked, a heterocycle with 5 or 6 links, optionally containing a second heteroatom chosen from oxygen, sulphur or nitrogen, optionally substituted on the nitrogen atom which is not linked to $R_5$ and $R_6$ by:
- a linear or branched alkyl radical containing at most 5 carbon atoms optionally substituted by a hydroxy radical, a halogen atom or an alkyloxy radical containing 1 to 4 carbon atoms,
- an aryl or arylalkyl radical containing 7 to 12 carbon atoms, these radicals being optionally substituted, the said products of formula (I) being in all the possible racemic or optically active isomer forms, as well as the addition salts with mineral or organic acids of the said products of formula (I).

4. Products of formula (I) as defined in one of claims 1 to 3, characterized in that the substituent or substituents which can be carried by the phenyl, aryl and arylalkyl radicals are chosen from the group formed by halogen atoms, the hydroxy radical, linear and branched alkyl and alkyloxy radicals containing at the most 5 carbon atoms, methylthio, nitro, amino, monoalkyl- and dialkylamino radicals, the said products of formula (I) being in all the possible racemic or optically active isomer forms, as well as the addition salts with mineral or organic acids of the said products of formula (I).

5. [16alpha,(±)]-15-(1-propynyl)-20,21-dinoreburnamenine oxalate,

- [16alpha,(±)]-15-methyl-20,21-dinoreburnamenine,
- [16alpha,(±)]-14,15-dihydro-15-methylene-20,21-dinoreburnamenine acid maleate,
- Ethyl [16alpha,(±)]-20,21-dinoreburnamenine-15-acetate maleate,
- [16alpha,(±)]-20,21-dinoreburnamenine-15-methanol acid maleate,
- [16alpha,(±)]-N,N-dimethyl-20,21-dinoreburnamenine-15-methanamine acid maleate.

6. Preparation process for products of formula (I) as defined in claim 1, characterized in that a product of formula (II):

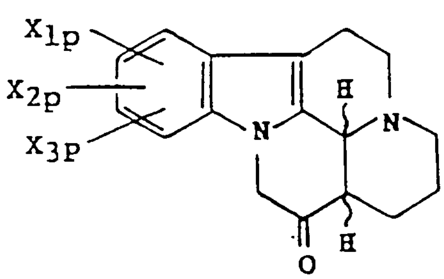

(II)

in which $X_{1p}$, $X_{2p}$ and $X_{3p}$ represent $X_1$, $X_2$ and $X_3$ which have the meanings indicated in claim 1, in which the reactive functions are optionally protected, is reacted

A) either with a halide of formula (III):

R'-V-Hal (III)

in which V represents a magnesium or zinc atom and Hal represents a halogen atom, or an organometallic compound of formula (IV):

R'-W (IV)

in which W represents a lithium, sodium or potassium atom, in which formulae (III) and (IV) R' represents either the values indicated in claim 1 for R, except for the free, salified or esterified carboxy value, or the said values of R in which the reactive functions are protected, in order to obtain a compound of formula (V):

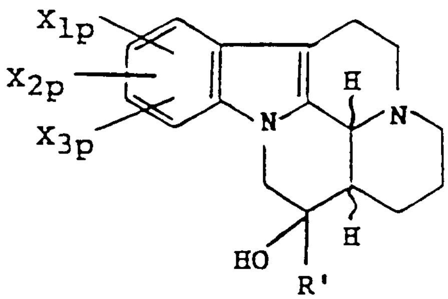

(V)

in which R' has the meaning indicated above, which is subjected to a dehydration reaction and, if necessary, to an elimination reaction of the protector groups of the reactive functions which can be carried by $X_{1p}$, $X_{2p}$, $X_{3p}$ and R', in order to obtain a compound of formula ($Ia_1$):

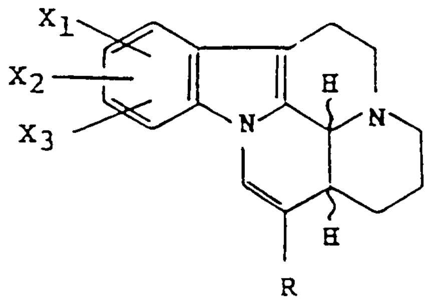

($Ia_1$)

in which $X_1$, $X_2$, $X_3$ and R have the meanings indicated above, which product of formula ($Ia_1$) is subjected, if appropriate, when R represents a $-CH_2OH$ radical, to the action of an oxidizing agent in order to obtain a product of formula ($Ia_2$):

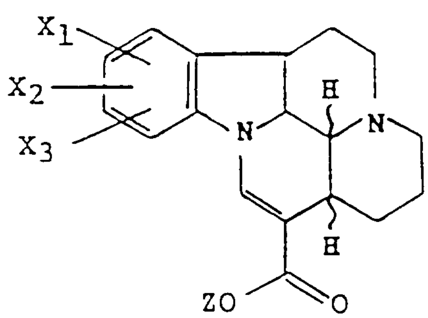

($Ia_2$)

in which $X_1$, $X_2$, $X_3$ have the meanings indicated above and Z represents a hydrogen atom or the remainder of an ester group, which is subjected, if necessary or if desired, to one or both of the following reactions:

- hydrolysis of the ester group,

- esterification or salification by a base of the carboxyl function.

B) <u>or</u> with a derivative of triphenylphosphorane of formula (VI):

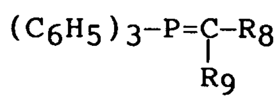

(VI)

or a phosphonate of formula (VI'):

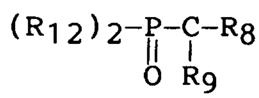

(VI')

in which $R_{12}$ represents an alkyloxy radical, in which formulae (VI) and (VI') $R_8$ and $R_9$ have the meanings indicated in claim 1, in order to give, according to the operating conditions used and optionally after elimination of the protector groups of the reactive functions which can be carried by $X_{1p}$, $X_{2p}$ and $X_{3p}$, a compound of formula ($Ib_1$):

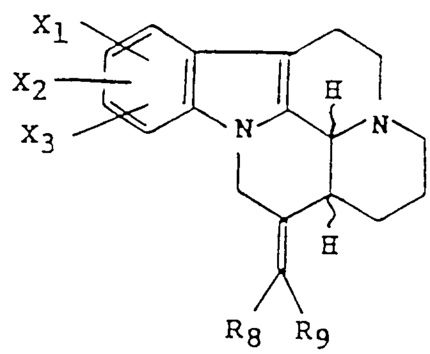

($Ib_1$)

or a compound of formula ($Ia_3$):

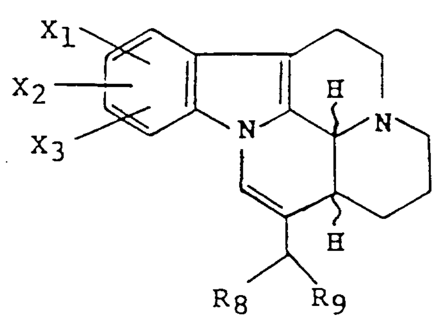

($Ia_3$)

in which $X_1$, $X_2$, $X_3$, $R_8$ and $R_9$ have the meanings indicated in claim 1,
C) <u>or</u> with an activated derivative of the product of formula (VII):

$$R_{10}\text{-C}\equiv\text{CH} \qquad \text{(VII)}$$

in which $R_{10}$ has the meaning given in claim 1, in order to obtain a compound of formula (VIII):

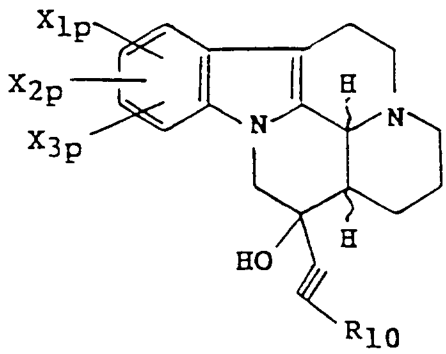

(VIII)

in which $X_{1p}$, $X_{2p}$, $X_{3p}$ and $R_{10}$ have the meanings indicated above, which is subjected, after activation of the hydroxyl radical and optional protection of the reactive functions which $R_{10}$ can contain, to a reduction reaction, and optionally an elimination reaction of the protector groups of the protected reactive functions, in order to obtain a compound of formula ($Ib_2$):

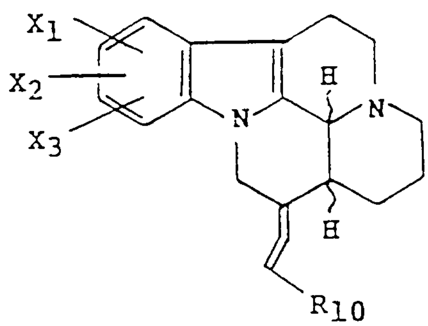

($Ib_2$)

in which $X_1$, $X_2$, $X_3$ and $R_{10}$ have the meanings indicated above, and if desired the products of formulae ($Ia_1$), ($Ia_3$), ($Ib_1$) and ($Ib_2$) are treated with a mineral or organic acid in order to obtain the corresponding salt, the said products of formula (I) being in all the possible racemic or enantiomeric isomer forms.

**7.** Preparation process for products of formula (I) as defined in claim 1 and in which:

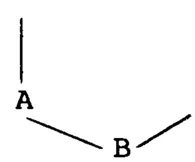

represents the group

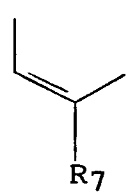

in which $R_7$ represents a methyl radical optionally substituted by a hydroxy radical, by a halogen atom, or by an

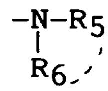

radical in which $R_5$ and $R_6$ have the meaning indicated in claim 3 or $R_7$ represents a free, salified or esterified carboxy radical, characterized in that a product of formula (II):

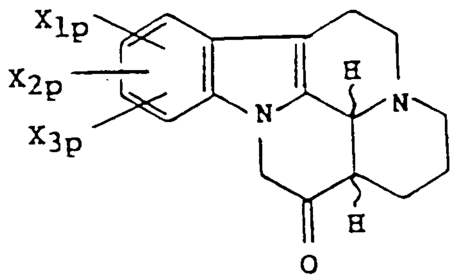

(II)

in which $X_{1p}$, $X_{2p}$ and $X_{3p}$ have the meaning indicated in claim 6, is reacted with a halogenated derivative of methyloxosulphonium of formula (IX):

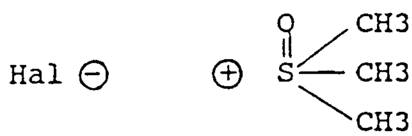

(IX)

in a basic medium, in order to obtain the corresponding reactional epoxide of formula (X):

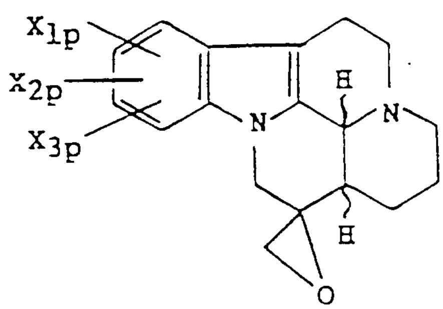

(X)

in which $X_{1p}$, $X_{2p}$ and $X_{3p}$ have the meanings indicated above, which is treated:

**either** with a base in order to produce the corresponding alcohol of formula (X'):

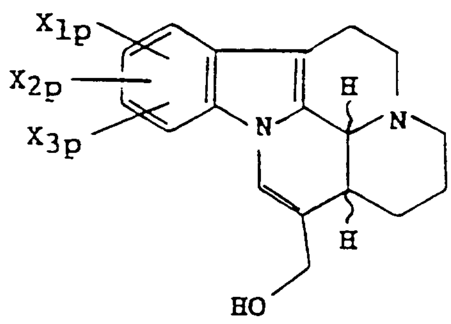

(X')

in which $X_{1p}$, $X_{2p}$ and $X_{3p}$ have the meanings indicated above, and which, if necessary, is subjected to an elimination reaction of the protector groups of the reactive functions that can be carried by $X_{1p}$, $X_{2p}$ and $X_{3p}$, in order to produce a product of formula ($Ia_4$):

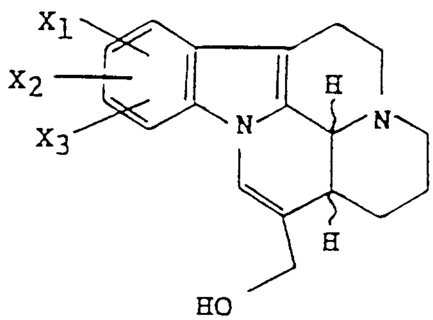

($Ia_4$)

in which $X_1$, $X_2$ and $X_3$ have the meanings indicated in claim 1, then if necessary and if desired, the product ($Ia_4$) obtained is treated with an oxidizing agent to obtain a product of formula ($Ia_2$):

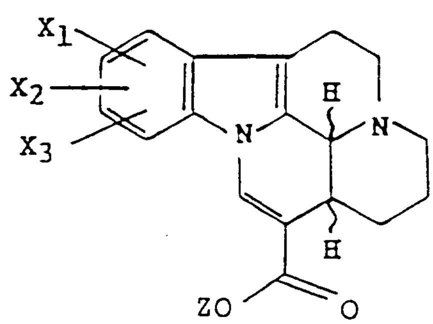

($Ia_2$)

in which $X_1$, $X_2$, $X_3$ and Z have the meanings indicated above, which is subjected, if necessary and if desired, to one or both of the following reactions:

- hydrolysis of the ester group,
- esterification or salification by a base of the carboxyl function,

**or** with an amine of formula (XI):

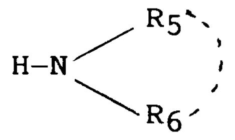

(XI)

in which $R_5$ and $R_6$ have the meaning indicated in claim 3, in order to obtain the compound of formula (XII):

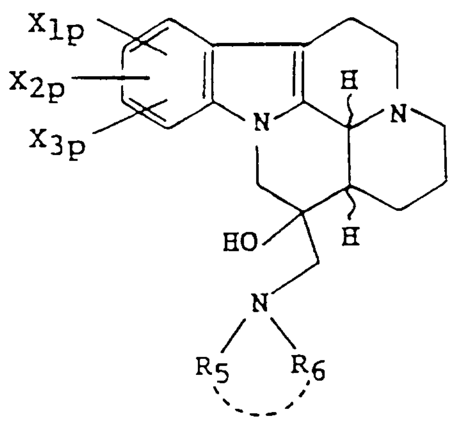

(XII)

in which $X_{1p}$, $X_{2p}$ and $X_{3p}$ have the meanings indicated above, the said compounds of formulae (X') and (XII) being subjected, after activation of the hydroxyl radical:

- either, if desired in the case of the compound (X'), to a reaction with the amine of formula (XI) as defined above,
- or, in the case of the compound (XII), to a dehydration reaction, to obtain, in these two cases and after elimination, if necessary, of the protector groups of the reactive functions which can be carried by $X_{1p}$, $X_{2p}$ and $X_{3p}$, a compound of formula ($Ia_5$):

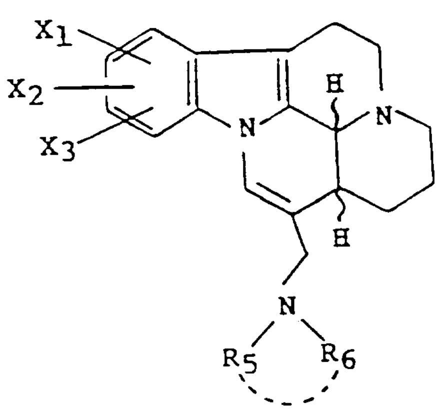

($Ia_5$)

in which $X_1$, $X_2$, $X_3$, $R_5$ and $R_6$ have the meanings indicated in claim 3,

- or with a tetrabutylammonium halide in order to obtain a product of formula (XIII):

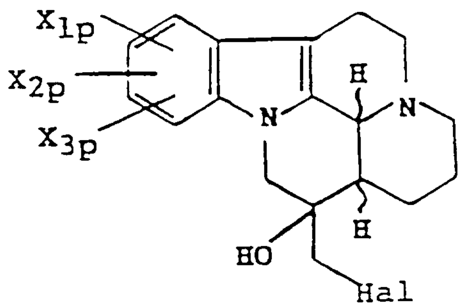

(XIII)

in which Hal represents a halogen atom, then subjected to a dehydration reaction to obtain, after elimination, if necessary, of the protector groups of the reactive functions which can be carried by $X_{1p}$, $X_{2p}$ and $X_{3p}$, a compound of formula ($Ia_6$):

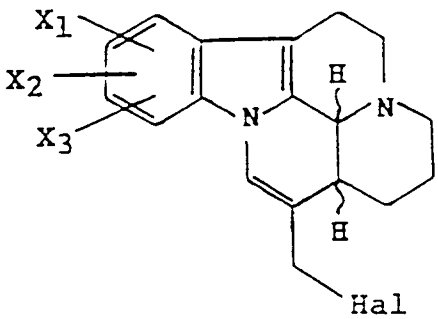

($Ia_6$)

in which $X_1$, $X_2$, $X_3$ and Hal have the meaning indicated above, and if desired the products of formulae ($Ia_3$), ($Ia_5$) and ($Ia_6$) are treated with a mineral or organic acid in order to obtain the corresponding salt, the said products of formula (I) being in all the possible racemic and enantiomeric isomer forms.

**8.** Preparation process for products of formula (I) as defined in claim 1 and in which:

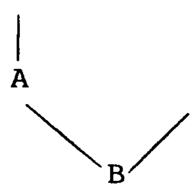

represents the group

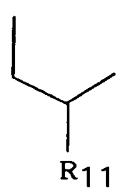

in which $R_{11}$ represents the acetylene radical of formula:

$$R_{10}\text{-}C\equiv C\text{-}$$

in which $R_{10}$ has the meaning indicated in claim 1, characterized in that a product of formula (VIII):

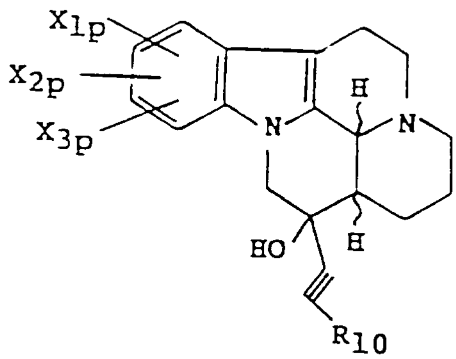

(VIII)

as defined in claim 6, in which $X_{1p}$, $X_{2p}$, $X_{3p}$ and $R_{10}$ have the meaning indicated in claim 6, after activation of the hydroxyl radical, is subjected to a dehydration reaction to obtain, if necessary and if desired after elimination of the protector groups of the reactive functions that can be carried by $X_{1p}$, $X_{2p}$ and $X_{3p}$, a compound of -enyne type of formula ($Ia_7$):

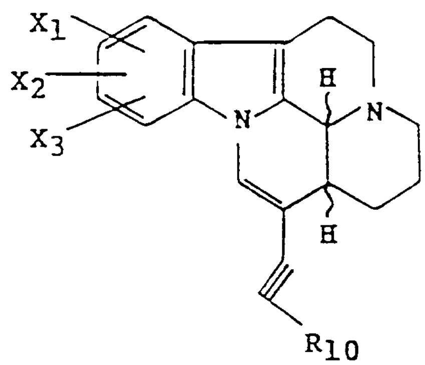

($Ia_7$)

in which $X_1$,$X_2$, $X_3$ and $R_{10}$ have the meanings indicated in claim 1, and if desired the product of formula ($Ia_7$) is treated with a mineral or organic acid, the said products of formula (I) being in all the possible racemic or enantiomeric isomer forms.

**9.** As medicaments, the products as defined by formula (I) of claim 1, the said products of formula (I) being in all the possible racemic or enantiomeric isomer forms, as well as the addition salts with the pharmaceutically acceptable mineral or organic acids or bases.

**10.** As medicaments, the products of formula (I) as defined in one of claims 2, 3, 4 or 5, as well as their pharmaceutically acceptable salts.

**11.** The pharmaceutical compositions containing as active ingredient, at least one of the medicaments as defined in one of claims 9 and 10.

**12.** As new industrial products, the compounds of formulae (II"):

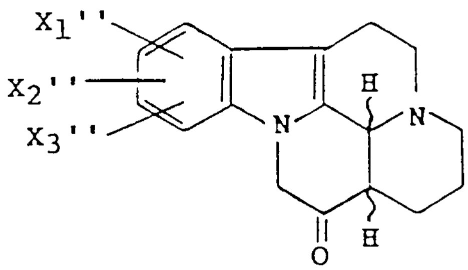

(II")

in which $X_1$", $X_2$" and $X_3$", identical or different, represent $X_1$, $X_2$ and $X_3$, except for the products in which one of the substituents $X_1$", $X_2$" and $X_3$" represents a hydrogen atom and the other two radicals, identical or different, are chosen from hydrogen or halogen atoms, alkyl or alkyloxy radicals containing 1 to 5 carbon atoms, a hydroxy, trifluoromethyl or nitro radical.

**13.** As new industrial products, the compounds of formulae (V), (VIII), (X), (X'), (XII) and (XIII) as defined in claims 6 and 7.

**Claims for the following Contracting State : ES**

**1.** Process for preparing products of formula (I):

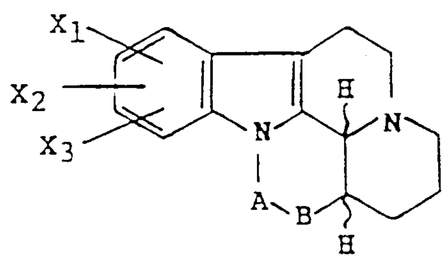

(I)

in which $X_1$, $X_2$ and $X_3$, identical or different, represent a hydrogen atom, a halogen atom, an alkyl, alkenyl or alkynyl radical, these radicals being linear or branched, containing at most 18 carbon atoms and optionally substituted, an optionally substituted linear or branched alkyloxy radical containing at most 7 carbon atoms, a hydroxy, trifluoromethyl or nitro radical, an amino, mono or dialkylamino radical, or an optionally substituted phenyl radical, and in which

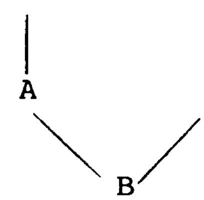

represents

A) either the group

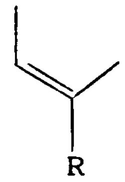

in which R represents:

a) a linear or branched alkyl, alkenyl or alkynyl radical containing at most 7 carbon atoms, this radical being optionally substituted,
b) an optionally substituted phenyl radical,
c) a free, salified or esterified carboxy group,

B) or the group

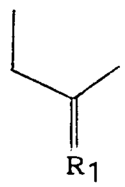

in which $R_1$ represents one of the two groups

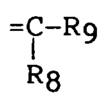

or

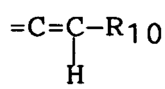

in which:
$R_8$ and $R_9$, identical or different, are such that:

- one is chosen from the group formed by:

  a) a hydrogen atom,
  b) a linear or branched alkyl, alkenyl or alkynyl radical containing at most 6 carbon atoms, this radical being optionally substituted,
  c) an optionally substituted phenyl radical,
  d) an esterified carboxy radical,
  e) a cyano radical,
  f) an acyl radical having 2 to 6 carbon atoms,

- and the other is chosen from the group formed by:

  a) a hydrogen atom,
  b) a linear or branched alkyl, alkenyl or alkynyl radical containing at most 6 carbon atoms, this radical being optionally substituted,
  c) an optionally substituted phenyl radical,

and $R_{10}$ represents:

a) a hydrogen atom,

b) a linear or branched alkyl, alkenyl or alkynyl radical containing at most 5 carbon atoms, this radical being optionally substituted,

c) an optionally substituted phenyl radical,

the said products of formula (I) being in all the possible racemic or optically active isomer forms, as well as the addition salts with mineral or organic acids or with the bases of the said products of formula (I), characterized in that a product of formula (II):

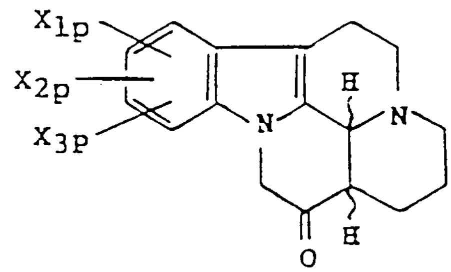

(II)

in which $X_{1p}$, $X_{2p}$ and $X_{3p}$ represent $X_1$, $X_2$ and $X_3$ which have the meanings indicated above, in which the reactive functions are optionally protected, is reacted

A) <u>either</u> with a halide of formula (III):

R'-V-Hal (III)

in which V represents a magnesium or zinc atom and Hal represents a halogen atom, or an organometallic compound of formula (IV):

R'-W (IV)

in which W represents a lithium, sodium or potassium atom, in which formulae (III) and (IV) R' represents either the values indicated above for R, except for the free, salified or esterified carboxy value, or the said values of R in which the reactive functions are protected, in order to obtain a compound of formula (V):

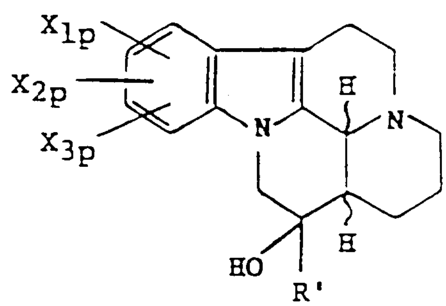

(V)

in which R' has the meaning indicated above, which is subjected to a dehydration reaction and, if necessary, to an elimination reaction of the protector groups of the reactive functions which can be carried by $X_{1p}$, $X_{2p}$, $X_{3p}$

and R', in order to obtain a compound of formula ($Ia_1$):

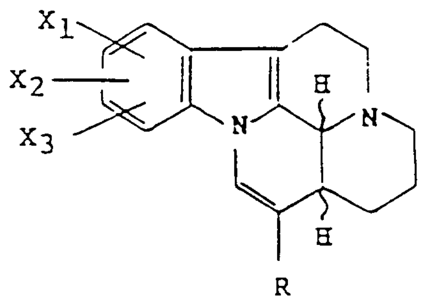

($Ia_1$)

in which $X_1$, $X_2$, $X_3$ and R have the meanings indicated above, which product of formula ($Ia_1$) is subjected, if appropriate, when R represents a $-CH_2OH$ radical, to the action of an oxidizing agent in order to obtain a product of formula ($Ia_2$):

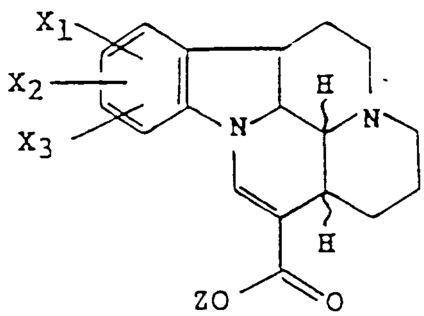

($Ia_2$)

in which $X_1$, $X_2$, $X_3$ have the meanings indicated above and Z represents a hydrogen atom or the remainder of an ester group, which is subjected, if necessary or if desired, to one or both of the following reactions:

- hydrolysis of the ester group,
- esterification or salification by a base of the carboxyl function.

B) <u>or</u> with a derivative of triphenylphosphorane of formula (VI):

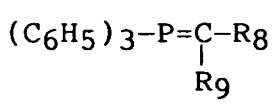

(VI)

or a phosphonate of formula (VI'):

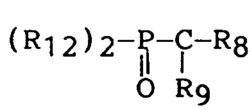

(VI')

in which $R_{12}$ represents an alkyloxy radical, in which formulae (VI) and (VI') $R_8$ and $R_9$ have the meanings indicated above, in order to give, according to the operating conditions used and optionally after elimination of the protector groups of the reactive functions which can be carried by $X_{1p}$, $X_{2p}$ and $X_{3p}$, a compound of formula

($Ib_1$):

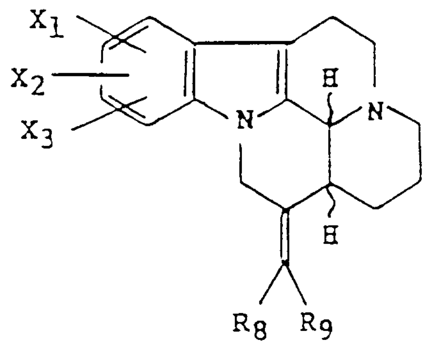

($Ib_1$)

or a compound of formula ($Ia_3$):

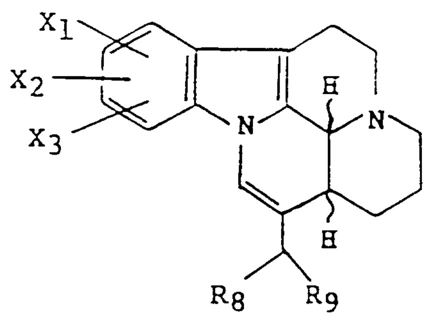

($Ia_3$)

in which $X_1$, $X_2$, $X_3$, $R_8$ and $R_9$ have the meanings indicated above,
C) <u>or</u> with an activated derivative of the product of formula (VII):

$$R_{10}\text{-}C{\equiv}CH \qquad (VII)$$

in which $R_{10}$ has the meaning given above, in order to obtain a compound of formula (VIII):

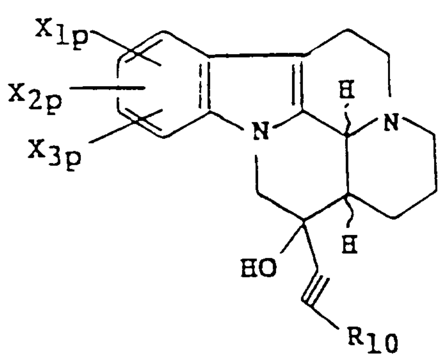

(VIII)

in which $X_{1p}$, $X_{2p}$, $X_{3p}$ and $R_{10}$ have the meanings indicated above, which is subjected, after activation of the hydroxyl radical and optional protection of the reactive functions which $R_{10}$ can contain, to a reduction reaction, and optionally an elimination reaction of the protector groups of the protected reactive functions, in order to

obtain a compound of formula ($Ib_2$):

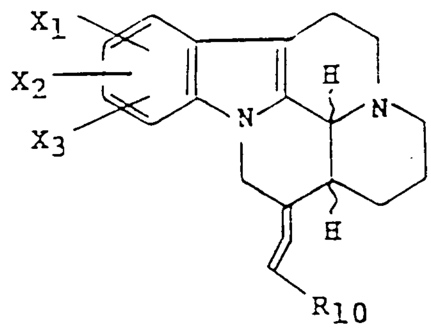

($Ib_2$)

in which $X_1$, $X_2$, $X_3$ and $R_{10}$ have the meanings indicated above, and if desired the products of formulae ($Ia_1$), ($Ia_3$), ($Ib_1$) and ($Ib_2$) are treated with a mineral or organic acid in order to obtain the corresponding salt, the said products of formula (I) being in all the possible racemic or enantiomeric isomer forms.

**2.** Process for preparing products of formula (I) as defined in claim 1 and in which:

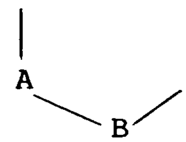

represents the group

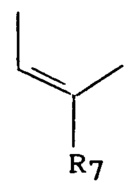

in which $R_7$ represents a methyl radical optionally substituted by a hydroxy radical, by a halogen atom, or by an

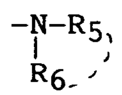

radical in which $R_5$ and $R_6$, identical or different, represent:

- a hydrogen atom,
- a linear or branched alkyl radical containing at most 7 carbon atoms optionally substituted by one or more radicals chosen from the group formed by the hydroxy radical, linear and branched alkyloxy radicals containing at most 5 carbon atoms and carboxy radicals, free, or esterified by a linear or branched alkyl radical containing at most 5 carbon atoms,
- an aryl or arylalkyl radical containing 7 to 12 carbon atoms, these radicals being optionally substituted,
  <u>or</u> $R_5$ and $R_6$ form, together with the nitrogen atom to which they are linked, a heterocycle with 5 or 6 links, optionally containing a second heteroatom chosen from oxygen, sulphur or nitrogen, optionally substituted on the nitrogen atom which is not linked to $R_5$ and $R_6$ by:
- a linear or branched alkyl radical containing at most 5 carbon atoms optionally substituted by a hydroxy radical, a halogen atom or an alkyloxy radical containing 1 to 4 carbon atoms,

- an aryl radical or arylalkyl radical containing 7 to 12 carbon atoms, these radicals being optionally substituted, or $R_7$ represents a free, salified or esterified carboxy radical, characterized in that a product of formula (II):

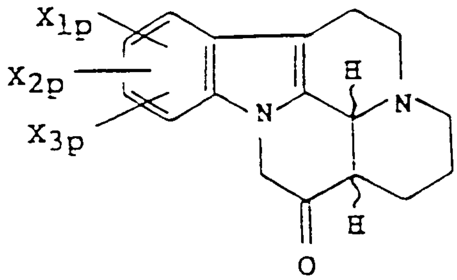

(II)

in which $X_{1p}$, $X_{2p}$ and $X_{3p}$ have the meaning indicated in claim 1, is reacted with a halogenated derivative of methyloxosulphonium of formula (IX):

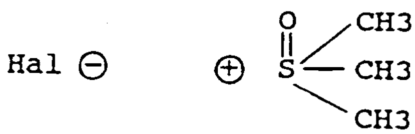

(IX)

in a basic medium, in order to obtain the corresponding reactional epoxide of formula (X):

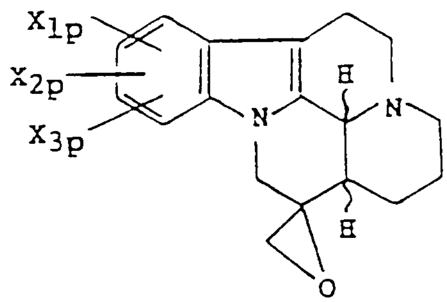

(X)

in which $X_{1p}$, $X_{2p}$ and $X_{3p}$ have the meanings indicated above, which is treated:

**either** with a base in order to produce the corresponding alcohol of formula (X'):

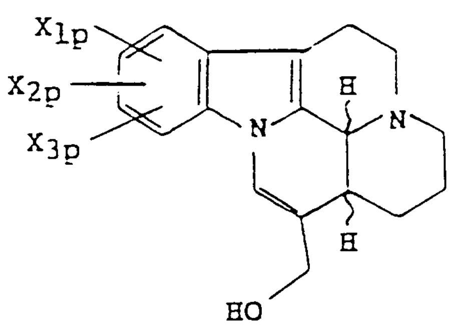

(X')

in which $X_{1p}$, $X_{2p}$ and $X_{3p}$ have the meanings indicated above, and which, if necessary, is subjected to an elimination reaction of the protector groups of the reactive functions that can be carried by $X_{1p}$, $X_{2p}$ and $X_{3p}$, in order to produce

a product of formula ($Ia_4$):

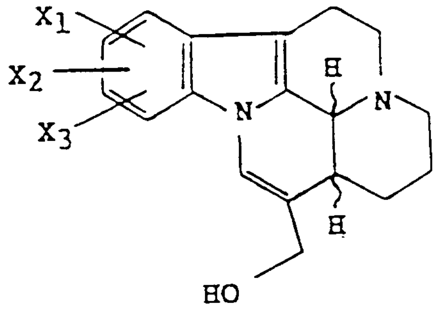

($Ia_4$)

in which $X_1$, $X_2$ and $X_3$ have the meanings indicated in claim 1, then if necessary and if desired, the product ($Ia_4$) obtained is treated with an oxidizing agent to obtain a product of formula ($Ia_2$):

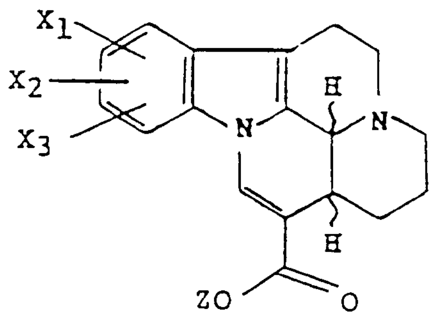

($Ia_2$)

in which $X_1$, $X_2$, $X_3$ and Z have the meanings indicated above, which is subjected, if necessary and if desired, to one or both of the following reactions:

- hydrolysis of the ester group,
- esterification or salification by a base of the carboxyl function,

<u>or</u> with an amine of formula (XI):

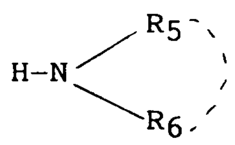

(XI)

in which $R_5$ and $R_6$ have the meaning indicated above, in order to obtain the compound of formula (XII):

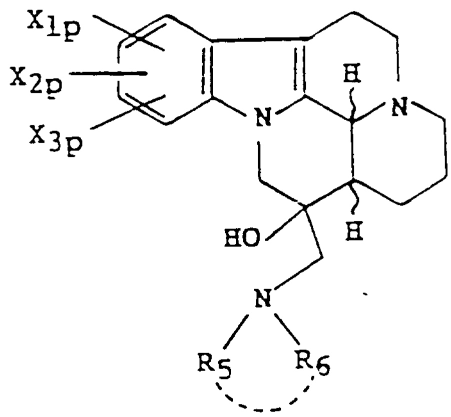

(XII)

in which $X_{1p}$, $X_{2p}$ and $X_{3p}$ have the meanings indicated above, the said compounds of formulae (X') and (XII) being subjected, after activation of the hydroxyl radical:

- either, if desired in the case of the compound (X'), to a reaction with the amine of formula (XI) as defined above,
- or, in the case of the compound (XII), to a dehydration reaction, to obtain, in these two cases and after elimination, if necessary, of the protector groups of the reactive functions which can be carried by $X_{1p}$, $X_{2p}$ and $X_{3p}$, a compound of formula ($Ia_5$):

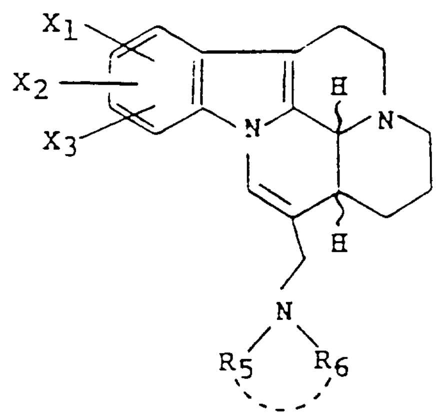

($Ia_5$)

in which $X_1$, $X_2$, $X_3$, $R_5$ and $R_6$ have the meanings indicated above,

- or with a tetrabutylammonium halide in order to obtain a product of formula (XIII):

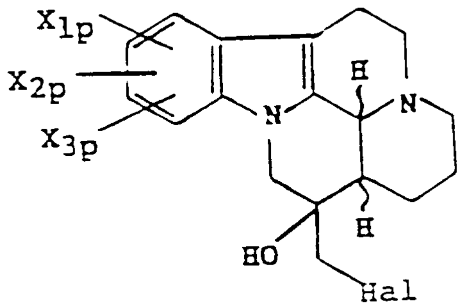

(XIII)

in which Hal represents a halogen atom, then subjected to a dehydration reaction to obtain, after elimination, if necessary, of the protector groups of the reactive functions which can be carried by $X_{1p}$, $X_{2p}$ and $X_{3p}$, a compound of formula ($Ia_6$):

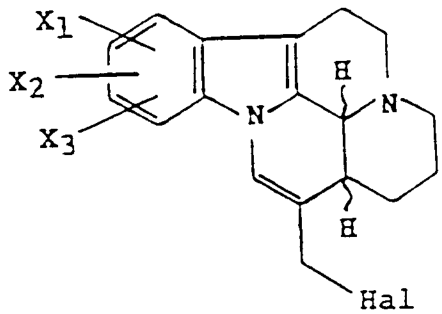

($Ia_6$)

in which $X_1$, $X_2$, $X_3$ and Hal have the meaning indicated above, and if desired the products of formulae ($Ia_3$), ($Ia_5$) and ($Ia_6$) are treated with a mineral or organic acid in order to obtain the corresponding salt, the said products of formula (I) being in all the possible racemic and enantiomeric isomer forms.

**3.** Process for preparing products of formula (I) as defined in claim 1 and in which:

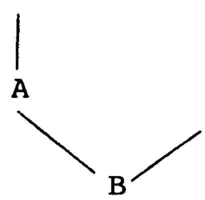

represents the group

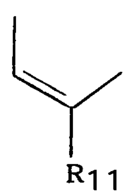

in which $R_{11}$ represents the acetylene radical of formula:

$R_{10}$-C≡C-

in which $R_{10}$ has the meaning indicated in claim 1, characterized in that a product of formula (VIII):

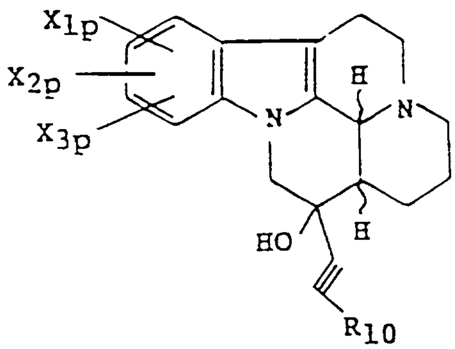

(VIII)

as defined in claim 1, in which $X_{1p}$, $X_{2p}$, $X_{3p}$ and $R_{10}$ have the meaning indicated in claim 1, after activation of the hydroxyl radical, is subjected to a dehydration reaction to obtain, if necessary and if desired after elimination of the protector groups of the reactive functions that can be carried by $X_{1p}$, $X_{2p}$ and $X_{3p}$, a compound of -enyne type of formula ($Ia_7$):

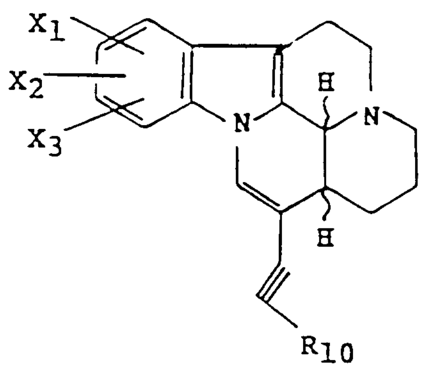

($Ia_7$)

in which $X_1$,$X_2$, $X_3$ and $R_{10}$ have the meanings indicated in claim 1, and if desired the product of formula ($Ia_7$) is treated with a mineral or organic acid, the said products of formula (I) being in all the possible racemic or enantiomeric isomer forms.

**4.** Process according to claim 1 for preparing products of formula (I) as defined in claim 1 corresponding to formula ($I_A$):

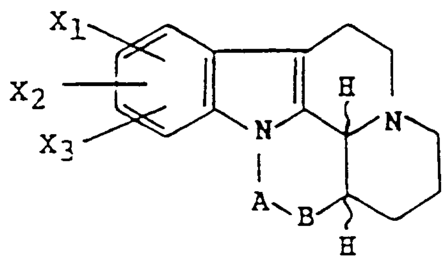

($I_A$)

in which $X_1$, $X_2$ and $X_3$ have the meaning indicated in claim 1 and

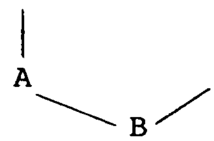

represents either the group

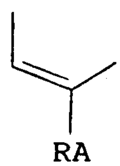

in which RA has the meaning indicated in claim 1 for R with the exception of the free, salified or esterified carboxy value, or the group

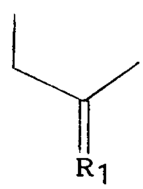

in which $R_1$ has the meaning indicated in claim 1, characterized in that products of formulae ($Ia_1$), ($Ib_1$), ($Ib_2$), ($Ia_3$) are prepared according to the process described in claim 1.

**5.** Process according to claim 2 for preparing products of formula ($I_A$) as defined in claim 4 and in which RA represents a methyl radical optionally substituted by a hydroxy radical or by an

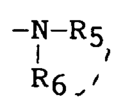

radical in which $R_5$ and $R_6$ have the meaning indicated in claim 2, characterized in that products of formula ($Ia_4$) or ($Ia_5$) are prepared according to the process described in claim 2.

**6.** Process according to any one of claims 1 to 5, characterized in that a product of formula (II) is used at the start in which $X_1$, $X_2$ and $X_3$ represent a hydrogen atom.

7. Process according to any one of claims 1 to 5, characterized in that a product of formula (II), (III), (IV), (VI), (VI') or (VII) is used at the start in which the substituent or substituents which can be carried by the alkyl, alkyloxy, alkenyl or alkynyl radicals are chosen from the group formed by:

- the hydroxy radical,
- halogen atoms,
- alkyloxy radicals having 1 to 6 carbon atoms,
- formyl and acyl radicals having 2 to 6 carbon atoms and the benzoyl radical,
- free carboxy radicals and carboxy radicals esterified by a linear or branched alkyl radical containing at most 5 carbon atoms,
- the cyano radical,
- the optionally substituted carbamoyl radical,
- the optionally substituted phenyl radical,
- the radical

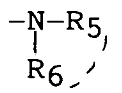

in which:
<u>either</u> $R_5$ and $R_6$, identical or different, represent:
- a hydrogen atom,
- a linear or branched alkyl radical containing at most 7 carbon atoms optionally substituted by one or more radicals chosen from the group formed by the hydroxy radical, linear and branched alkyloxy radicals containing at most 5 carbon atoms and carboxy radicals, free, or esterified by a linear or branched alkyl radical containing at most 5 carbon atoms,
- an aryl or arylalkyl radical containing 7 to 12 carbon atoms, these radicals being optionally substituted,

<u>or</u> $R_5$ and $R_6$ form, together with the nitrogen atom to which they are linked, a heterocycle with 5 or 6 links, optionally containing a second heteroatom chosen from oxygen, sulphur or nitrogen, optionally substituted on the nitrogen atom which is not linked to $R_5$ and $R_6$ by:
- a linear or branched alkyl radical containing at most 5 carbon atoms optionally substituted by a hydroxy radical, a halogen atom or an alkyloxy radical containing 1 to 4 carbon atoms,
- an aryl radical or arylalkyl radical containing 7 to 12 carbon atoms, these radicals being optionally substituted.

8. Process according to any one of claims 1 to 5, characterized in that a product of formula (II), (III), (IV), (VI), (VI'), (VII) or (XI) is used at the start in which the substituent or substituents which can be carried by the phenyl, aryl and arylalkyl radicals are chosen from the group formed by halogen atoms, the hydroxy radical, linear and branched alkyl and alkyloxy radicals containing at most 5 carbon atoms, methylthio, nitro, amino, monoalkyl-and dialkylamino radicals.

**Claims for the following Contracting State : GR**

1. Process for preparing products of formula (I):

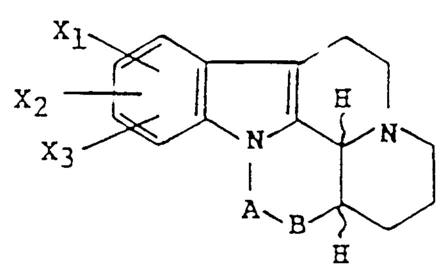

(I)

in which $X_1$, $X_2$ and $X_3$, identical or different, represent a hydrogen atom, a halogen atom, an alkyl, alkenyl or alkynyl radical, these radicals being linear or branched, containing at most 18 carbon atoms and optionally substituted, an optionally substituted linear or branched alkyloxy radical containing at most 7 carbon atoms, a hydroxy, trifluoromethyl or nitro radical, an amino, mono or dialkylamino radical, or an optionally substituted phenyl radical, and in which

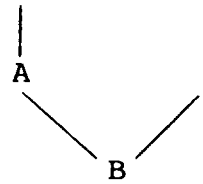

represents

A) either the group

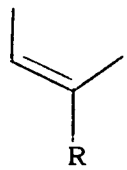

in which R represents:

a) a linear or branched alkyl, alkenyl or alkynyl radical containing at most 7 carbon atoms, this radical being optionally substituted,
b) an optionally substituted phenyl radical,
c) a free, salified or esterified carboxy group,

B) or the group

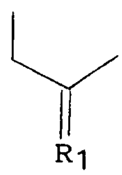

in which $R_1$ represents one of the two groups

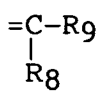 or 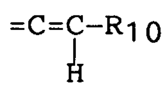

in which:
$R_8$ and $R_9$, identical or different, are such that:

- one is chosen from the group formed by:

a) a hydrogen atom,
b) a linear or branched alkyl, alkenyl or alkynyl radical containing at most 6 carbon atoms, this radical being optionally substituted,
c) an optionally substituted phenyl radical,
d) an esterified carboxy radical,
e) a cyano radical,
f) an acyl radical having 2 to 6 carbon atoms,

- and the other is chosen from the group formed by:

  a) a hydrogen atom,
  b) a linear or branched alkyl, alkenyl or alkynyl radical containing at most 6 carbon atoms, this radical being optionally substituted,
  c) an optionally substituted phenyl radical,

and $R_{10}$ represents:

a) a hydrogen atom,
b) a linear or branched alkyl, alkenyl or alkynyl radical containing at most 5 carbon atoms, this radical being optionally substituted,
c) an optionally substituted phenyl radical,

the said products of formula (I) being in all the possible racemic or optically active isomer forms, as well as the addition salts with mineral or organic acids or with the bases of the said products of formula (I), characterized in that a product of formula (II):

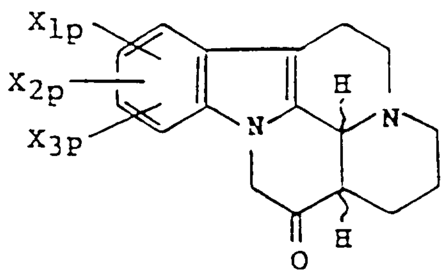

(II)

in which $X_{1p}$, $X_{2p}$ and $X_{3p}$ represent $X_1$, $X_2$ and $X_3$ which have the meanings indicated above, in which the reactive functions are optionally protected, is reacted

A) <u>either</u> with a halide of formula (III):

R'-V-Hal (III)

in which V represents a magnesium or zinc atom and Hal represents a halogen atom, or an organometallic compound of formula (IV):

R'-W (IV)

in which W represents a lithium, sodium or potassium atom, in which formulae (III) and (IV) R' represents either the values indicated above for R, except for the free, salified or esterified carboxy value, or the said values of R in which the reactive functions are protected, in order to obtain a compound of formula (V):

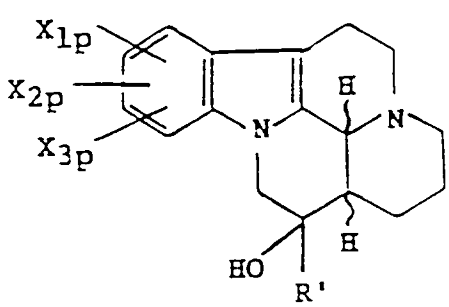

(V)

in which R' has the meaning indicated above, which is subjected to a dehydration reaction and, if necessary, to an elimination reaction of the protector groups of the reactive functions which can be carried by $X_{1p}$, $X_{2p}$, $X_{3p}$

and R', in order to obtain a compound of formula ($Ia_1$):

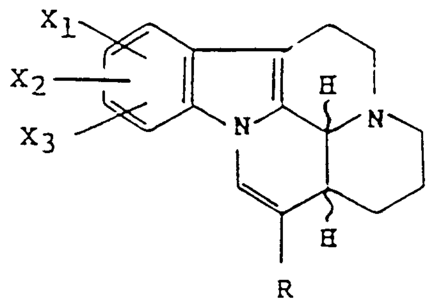

($Ia_1$)

in which $X_1$, $X_2$, $X_3$ and R have the meanings indicated above, which product of formula ($Ia_1$) is subjected, if appropriate, when R represents a $-CH_2OH$ radical, to the action of an oxidizing agent in order to obtain a product of formula ($Ia_2$):

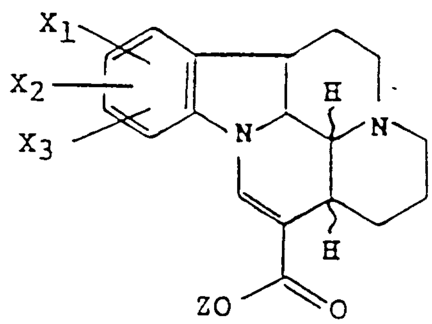

($Ia_2$)

in which $X_1$, $X_2$, $X_3$ have the meanings indicated above and Z represents a hydrogen atom or the remainder of an ester group, which is subjected, if necessary or if desired, to one or both of the following reactions:

- hydrolysis of the ester group,
- esterification or salification by a base of the carboxyl function.

B) <u>or</u> with a derivative of triphenylphosphorane of formula (VI):

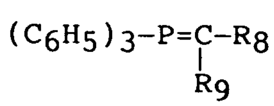

(VI)

or a phosphonate of formula (VI'):

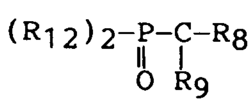

(VI')

in which $R_{12}$ represents an alkyloxy radical, in which formulae (VI) and (VI') $R_8$ and $R_9$ have the meanings indicated above, in order to give, according to the operating conditions used and optionally after elimination of the protector groups of the reactive functions which can be carried by $X_{1p}$, $X_{2p}$ and $X_{3p}$, a compound of formula

($Ib_1$):

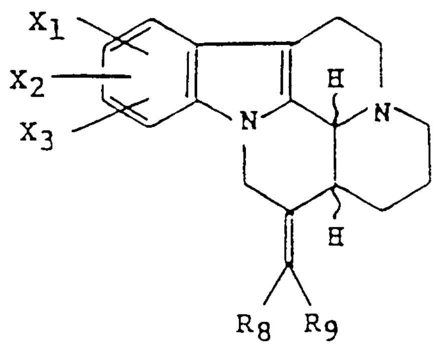

($Ib_1$)

or a compound of formula ($Ia_3$):

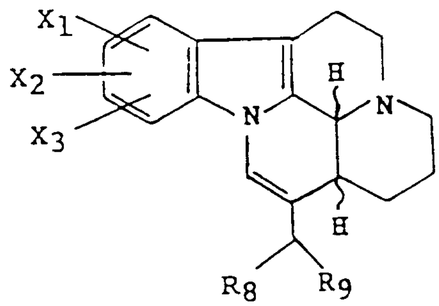

($Ia_3$)

in which $X_1$, $X_2$, $X_3$, $R_8$ and $R_9$ have the meanings indicated above,
C) or with an activated derivative of the product of formula (VII):

$$R_{10}\text{-}C{\equiv}CH \qquad \text{(VII)}$$

in which $R_{10}$ has the meaning given above, in order to obtain a compound of formula (VIII):

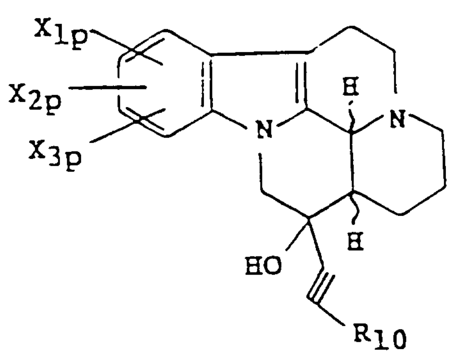

(VIII)

in which $X_{1p}$, $X_{2p}$, $X_{3p}$ and $R_{10}$ have the meanings indicated above, which is subjected, after activation of the hydroxyl radical and optional protection of the reactive functions which $R_{10}$ can contain, to a reduction reaction, and optionally an elimination reaction of the protector groups of the protected reactive functions, in order to

obtain a compound of formula ($Ib_2$):

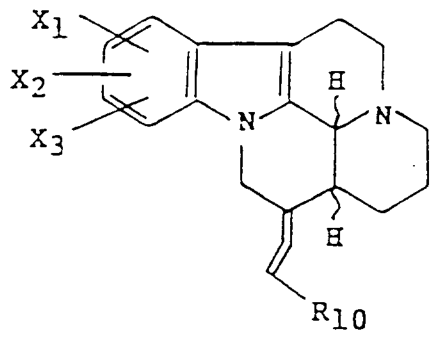

($Ib_2$)

in which $X_1$, $X_2$, $X_3$ and $R_{10}$ have the meanings indicated above, and if desired the products of formulae ($Ia_1$), ($Ia_3$), ($Ib_1$) and ($Ib_2$) are treated with a mineral or organic acid in order to obtain the corresponding salt, the said products of formula (I) being in all the possible racemic or enantiomeric isomer forms.

**2.** Process for preparing products of formula (I) as defined in claim 1 and in which:

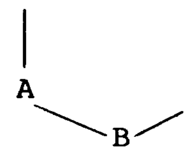

represents the group

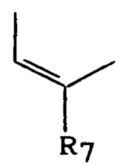

in which $R_7$ represents a methyl radical optionally substituted by a hydroxy radical, by a halogen atom, or by an

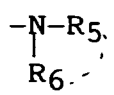

radical in which $R_5$ and $R_6$, identical or different, represent:

- a hydrogen atom,
- a linear or branched alkyl radical containing at most 7 carbon atoms optionally substituted by one or more radicals chosen from the group formed by the hydroxy radical, linear and branched alkyloxy radicals containing at most 5 carbon atoms and carboxy radicals, free, or esterified by a linear or branched alkyl radical containing at most 5 carbon atoms,
- an aryl or arylalkyl radical containing 7 to 12 carbon atoms, these radicals being optionally substituted, <u>or</u> $R_5$ and $R_6$ form, together with the nitrogen atom to which they are linked, a heterocycle with 5 or 6 links, optionally containing a second heteroatom chosen from oxygen, sulphur or nitrogen, optionally substituted on the nitrogen atom which is not linked to $R_5$ and $R_6$ by:
- a linear or branched alkyl radical containing at most 5 carbon atoms optionally substituted by a hydroxy radical, a halogen atom or an alkyloxy radical containing 1 to 4 carbon atoms,

- an aryl radical or arylalkyl radical containing 7 to 12 carbon atoms, these radicals being optionally substituted, or $R_7$ represents a free, salified or esterified carboxy radical, characterized in that a product of formula (II):

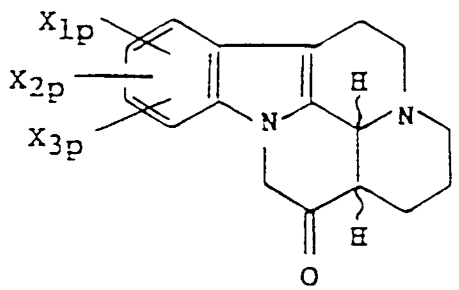

(II)

in which $X_{1p}$, $X_{2p}$ and $X_{3p}$ have the meaning indicated in claim 1, is reacted with a halogenated derivative of methyloxosulphonium of formula (IX):

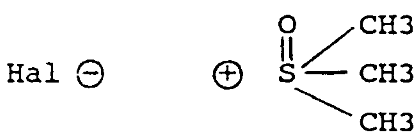

(IX)

in a basic medium, in order to obtain the corresponding reactional epoxide of formula (X):

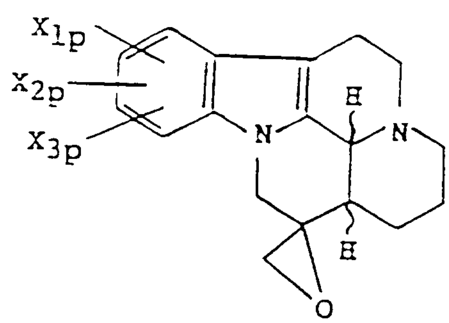

(X)

in which $X_{1p}$, $X_{2p}$ and $X_{3p}$ have the meanings indicated above, which is treated:

**either** with a base in order to produce the corresponding alcohol of formula (X'):

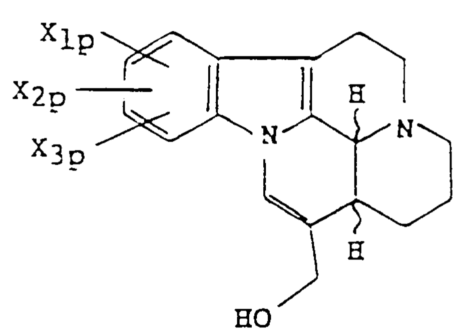

(X')

in which $X_{1p}$, $X_{2p}$ and $X_{3p}$ have the meanings indicated above, and which, if necessary, is subjected to an elimination

reaction of the protector groups of the reactive functions that can be carried by $X_{1p}$, $X_{2p}$ and $X_{3p}$, in order to produce a product of formula ($Ia_4$):

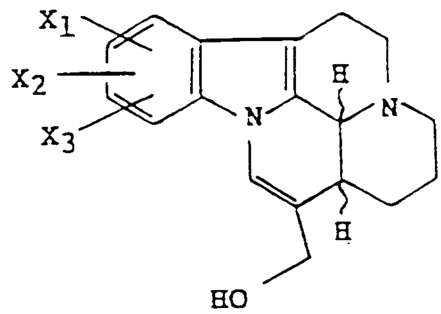

($Ia_4$)

in which $X_1$, $X_2$ and $X_3$ have the meanings indicated in claim 1, then if necessary and if desired, the product ($Ia_4$) obtained is treated with an oxidizing agent to obtain a product of formula ($Ia_2$):

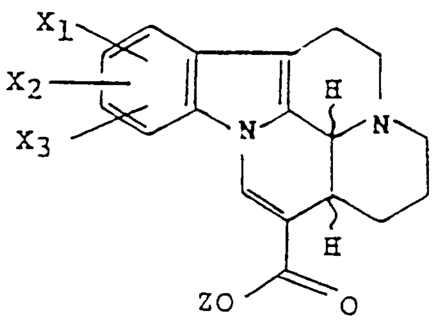

($Ia_2$)

in which $X_1$, $X_2$, $X_3$ and Z have the meanings indicated above, which is subjected, if necessary and if desired, to one or both of the following reactions:

- hydrolysis of the ester group,
- esterification or salification by a base of the carboxyl function,

**or** with an amine of formula (XI):

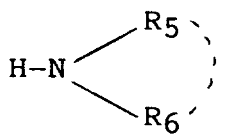

(XI)

in which $R_5$ and $R_6$ have the meaning indicated above, in order to obtain the compound of formula (XII):

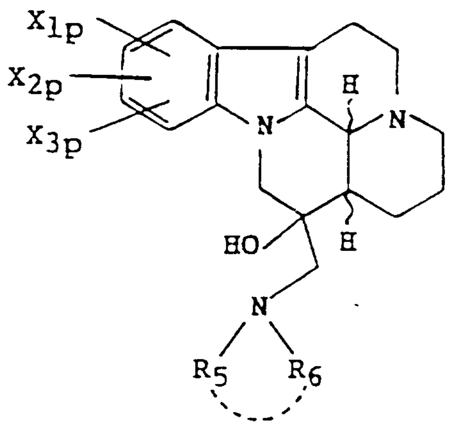

(XII)

in which $X_{1p}$, $X_{2p}$ and $X_{3p}$ have the meanings indicated above, the said compounds of formulae (X') and (XII) being subjected, after activation of the hydroxyl radical:

- either, if desired in the case of the compound (X'), to a reaction with the amine of formula (XI) as defined above,
- or, in the case of the compound (XII), to a dehydration reaction, to obtain, in these two cases and after elimination, if necessary, of the protector groups of the reactive functions which can be carried by $X_{1p}$, $X_{2p}$ and $X_{3p}$, a compound of formula ($Ia_5$):

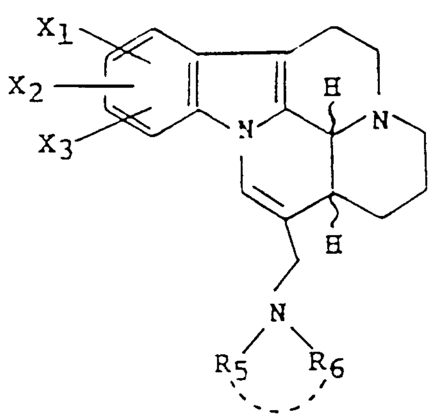

($Ia_5$)

in which $X_1$, $X_2$, $X_3$, $R_5$ and $R_6$ have the meanings indicated above,

- <u>or</u> with a tetrabutylammonium halide in order to obtain a product of formula (XIII):

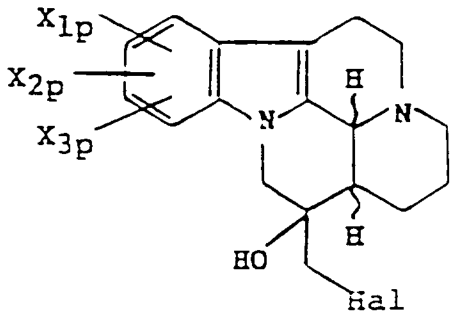

(XIII)

in which Hal represents a halogen atom, then subjected to a dehydration reaction to obtain, after elimination, if necessary, of the protector groups of the reactive functions which can be carried by $X_{1p}$, $X_{2p}$ and $X_{3p}$, a compound of formula ($Ia_6$):

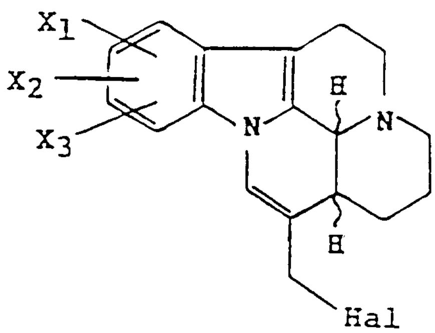

($Ia_6$)

in which $X_1$, $X_2$, $X_3$ and Hal have the meaning indicated above, and if desired the products of formulae ($Ia_3$), ($Ia_5$) and ($Ia_6$) are treated with a mineral or organic acid in order to obtain the corresponding salt, the said products of formula (I) being in all the possible racemic and enantiomeric isomer forms.

**3.** Process for preparing products of formula (I) as defined in claim 1 and in which:

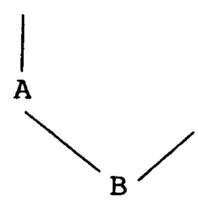

represents the group

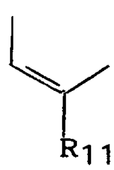

in which $R_{11}$ represents the acetylene radical of formula:

$R_{10}$-C≡C-

in which $R_{10}$ has the meaning indicated in claim 1, characterized in that a product of formula (VIII):

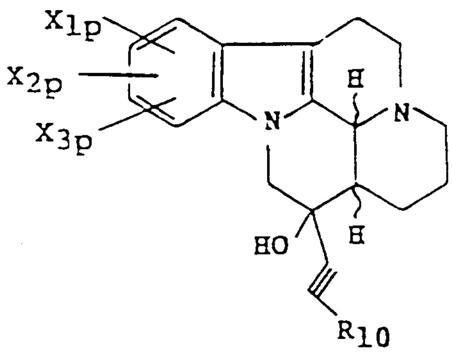

(VIII)

as defined in claim 1, in which $X_{1p}$, $X_{2p}$, $X_{3p}$ and $R_{10}$ have the meaning indicated in claim 1, after activation of the hydroxyl radical, is subjected to a dehydration reaction to obtain, if necessary and if desired after elimination of the protector groups of the reactive functions that can be carried by $X_{1p}$, $X_{2p}$ and $X_{3p}$, a compound of -enyne type of formula ($Ia_7$):

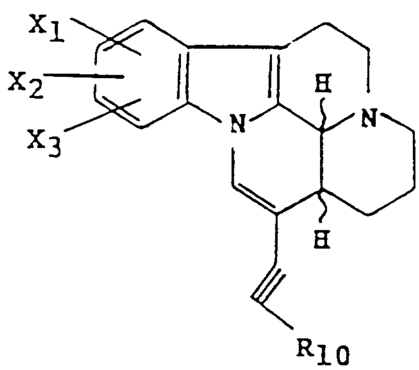

($Ia_7$)

in which $X_1$,$X_2$, $X_3$ and $R_{10}$ have the meanings indicated in claim 1, and if desired the product of formula ($Ia_7$) is treated with a mineral or organic acid, the said products of formula (I) being in all the possible racemic or enantiomeric isomer forms.

4. Process according to claim 1 for preparing products of formula (I) as defined in claim 1 corresponding to formula ($I_A$):

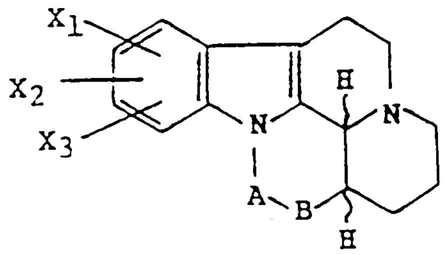

($I_A$)

in which $X_1$, $X_2$ and $X_3$ have the meaning indicated in claim 1 and

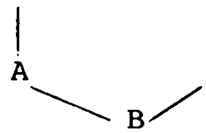

represents either the group

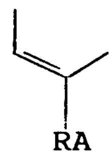

in which RA has the meaning indicated in claim 1 for R with the exception of the free, salified or esterified carboxy value, or the group

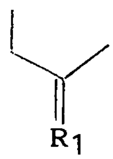

in which $R_1$ has the meaning indicated in claim 1, characterized in that products of formulae ($Ia_1$), ($Ib_1$), ($Ib_2$), ($Ia_3$) are prepared according to the process described in claim 1.

5. Process according to claim 2 for preparing products of formula ($I_A$) as defined in claim 4 and in which RA represents a methyl radical optionally substituted by a hydroxy radical or by an

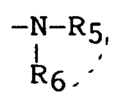

radical in which $R_5$ and $R_6$ have the meaning indicated in claim 2, characterized in that products of formula ($Ia_4$) or ($Ia_5$) are prepared according to the process described in claim 2.

6. Process according to any one of claims 1 to 5, characterized in that a product of formula (II) is used at the start in which $X_1$, $X_2$ and $X_3$ represent a hydrogen atom.

**7.** Process according to any one of claims 1 to 5, characterized in that a product of formula (II), (III), (IV), (VI), (VI') or (VII) is used at the start in which the substituent or substituents which can be carried by the alkyl, alkyloxy, alkenyl or alkynyl radicals are chosen from the group formed by:

- the hydroxy radical,
- halogen atoms,
- alkyloxy radicals having 1 to 6 carbon atoms,
- formyl and acyl radicals having 2 to 6 carbon atoms and the benzoyl radical,
- free carboxy radicals and carboxy radicals esterified by a linear or branched alkyl radical containing at most 5 carbon atoms,
- the cyano radical,
- the optionally substituted carbamoyl radical,
- the optionally substituted phenyl radical,
- the radical

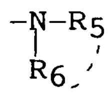

in which:
<u>either</u> $R_5$ and $R_6$, identical or different, represent:
- a hydrogen atom,
- a linear or branched alkyl radical containing at most 7 carbon atoms optionally substituted by one or more radicals chosen from the group formed by the hydroxy radical, linear and branched alkyloxy radicals containing at most 5 carbon atoms and carboxy radicals, free, or esterified by a linear or branched alkyl radical containing at most 5 carbon atoms,
- an aryl or arylalkyl radical containing 7 to 12 carbon atoms, these radicals being optionally substituted,
<u>or</u> $R_5$ and $R_6$ form, together with the nitrogen atom to which they are linked, a heterocycle with 5 or 6 links, optionally containing a second heteroatom chosen from oxygen, sulphur or nitrogen, optionally substituted on the nitrogen atom which is not linked to $R_5$ and $R_6$ by:
- a linear or branched alkyl radical containing at most 5 carbon atoms optionally substituted by a hydroxy radical, a halogen atom or an alkyloxy radical containing 1 to 4 carbon atoms,
- an aryl radical or arylalkyl radical containing 7 to 12 carbon atoms, these radicals being optionally substituted.

**8.** Process according to any one of claims 1 to 5, characterized in that a product of formula (II), (III), (IV), (VI), (VI'), (VII) or (XI) is used at the start in which the substituent or substituents which can be carried by the phenyl, aryl and arylalkyl radicals are chosen from the group formed by halogen atoms, the hydroxy radical, linear and branched alkyl and alkyloxy radicals containing at most 5 carbon atoms, methylthio, nitro, amino, monoalkyl-and dialkylamino radicals.

**9.** Process according to claim 4, characterized in that any one of the products of formula (I) of which the names follow is prepared:

- [16alpha,(±)]-15-(1-propynyl)-20,21-dinoreburnamenine oxalate,
- [16alpha,(±)]-15-methyl 20,21-dinoreburnamenine,
- [16alpha,(±)]-14,15-dihydro 15-methylene 20,21-dinoreburnamenine acid maleate,
- ethyl [16alpha,(±)]-20,21-dinoreburnamenine-15-acetate maleate,
- [16alpha,(±)]-20,21-dinoreburnamenine-15-methanol acid maleate,
- [16alpha,(±)]-N,N-dimethyl 20,21-dinoreburnamenine-15-methanamine acid maleate.

**10.** Preparation process for pharmaceutical compositions characterized in that at least one of the products of formula (I) as defined in claim 1 or at least one of its salts with pharmaceutically acceptable acids or bases is used as active ingredient in a form intended for this use.

**11.** Preparation process for pharmaceutical compositions characterized in that at least one of the products of formula ($I_A$) as defined in claim 4 or at least one of its salts with pharmaceutically acceptable acids or bases is used as active ingredient in a form intended for this use.

12. Preparation process for pharmaceutical compositions characterized in that at least one of the products of formula (I), mentioned in claim 9, or if appropriate at least one of its salts with pharmaceutically acceptable acids or bases is used as active ingredient in a form intended for this use.

13. As new industrial products, the compounds of formulae (V), (VIII), (X), (X'), (XII) and (XIII) as defined in claims 1 and 2.

**Patentansprüche**

**Patentansprüche für folgende Vertragsstaaten : AT, BE, CH, DE, DK, FR, GB, IT, LI, LU, NL, SE**

1. Produkte der Formel (I)

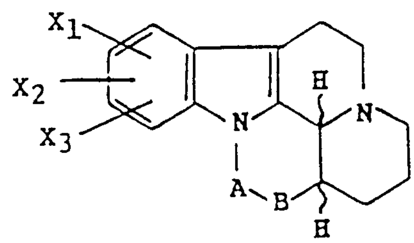

(I)

worin $X_1$, $X_2$ und $X_3$, die identisch oder verschieden sind, ein Wasserstoffatom, ein Halogenatom, einen Alkyl-, Alkenyl- oder Alkinylrest bedeuten, wobei diese Reste linear oder verzweigt sind und höchstens 18 Kohlenstoffatome enthalten und gegebenenfalls substituiert sind, einen linearen oder verzweigten Alkyloxyrest mit höchstens 7 Kohlenstoffatomen, gegebenenfalls substituiert, einen Hydroxy-, Trifluoromethyl- oder Nitrorest, einen Amino-, Mono- oder Dialkylaminorest, oder einen gegebenenfalls substituierten Phenylrest bedeuten, und worin

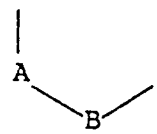

bedeutet

A) entweder die Gruppe

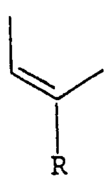

worin R bedeutet:

a) einen linearen oder verzweigten Alkyl-, Alkenyl- oder Alkinylrest mit höchstens 7 Kohlenstoffatomen, wobei dieser Rest gegebenenfalls substituiert ist,

b) einen gegebenenfalls substituierten Phenylrest,

c) eine freie, in das Salz überführte oder veresterte Carboxygruppe,

B) oder die Gruppe

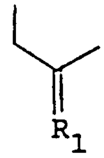

worin $R_1$ eine der beiden Gruppen

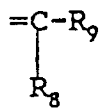

oder

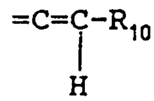

bedeutet, worin:
$R_8$ und $R_9$, die identisch oder verschieden sind, derart sind, daß:

- eines ausgewählt ist aus der Gruppe gebildet durch:

  a) ein Wasserstoffatom,

  b) einen linearen oder verzweigten Alkyl-, Alkenyl- oder Alkinylrest mit höchstens 6 Kohlenstoffatomen, wobei dieser Rest gegebenenfalls substituiert ist,

  c) einen gegebenenfalls substituierten Phenylrest,

  d) einen veresterten Carboxyrest,

  e) einen Cyanorest,

  f) einen Acylrest mit 2 bis 6 Kohlenstoffatomen,

- und der andere ist ausgewählt aus der Gruppe gebildet durch:

  a) ein Wasserstoffatom,

  b) einen linearen oder verzweigten Alkyl-, Alkenyl- oder Alkinylrest mit höchstens 6 Kohlenstoffatomen, wobei dieser Rest gegebenenfalls substituiert ist,

  c) einen gegebenenfalls substituierten Phenylrest,

und $R_{10}$ bedeutet:

a) ein Wasserstoffatom,

b) einen linearen oder verzweigten Alkyl-, Alkenyl- oder Alkinylrest mit höchstens 5 Kohlenstoffatomen, wobei dieser Rest gegebenenfalls substituiert ist,

c) einen gegebenenfalls substituierten Phenylrest,

wobei diese Produkte der Formel (I) unter allen möglichen racemischen oder optisch aktiven isomeren Formen vorliegen sowie die Additionssalze dieser Produkte der Formel (I) mit Mineral- oder organischen Säuren oder mit Basen.

2. Produkte der Formel (I), wie sie in Anspruch 1 definiert sind, dadurch gekennzeichnet, daß $X_1$, $X_2$ und $X_3$ ein Wasserstoffatom darstellen, wobei diese Produkte der Formel (I) in allen möglichen racemischen oder optisch aktiven isomeren Formen vorliegen, sowie die Additionssalze dieser Produkte der Formel (I) mit Mineral- oder organischen Säuren oder mit Basen.

3. Produkte der Formel (I), wie sie in Anspruch 1 definiert sind, dadurch gekennzeichnet, daß der oder die Substituent(en), welche die Alkyl-, Alkyloxy-, Alkenyl- oder Alkinylreste tragen können, ausgewählt sind aus der Gruppe gebildet durch:

- den Hydroxyrest,
- die Halogenatome,
- die Alkyloxyreste mit 1 bis 6 Kohlenstoffatomen,
- die Formyl- und Acylreste mit 2 bis 6 Kohlenstoffatomen und den Benzoylrest,
- die freien und durch einen linearen oder verzweigten Alkylrest mit höchstens 5 Kohlenstoffatomen veresterten Carboxyreste,
- den Cyanorest,
- den gegebenenfalls substituierten Carbamoylrest,
- den gegebenenfalls substituierten Phenylrest,
- den Rest

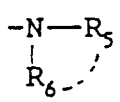

,

worin:
<u>entweder</u> $R_5$ und $R_6$, die identisch oder verschieden sind, bedeuten:

- ein Wasserstoffatom,
- einen linearen oder verzweigten Alkylrest mit höchstens 7 Kohlenstoffatomen, gegebenenfalls substituiert durch einen oder mehrere Rest(e), ausgewählt aus der Gruppe gebildet durch den Hydroxyrest, die linearen oder verzweigten Alkyloxyreste mit höchstens 5 Kohlenstoffatomen,und die freien oder durch einen linearen oder verzweigten Alkylrest mit höchstens 5 Kohlenstoffatomen veresterten Carboxyreste,
- einen Aryl- oder Arylalkylrest mit 7 bis 12 Kohlenstoffatomen, wobei diese gegebenenfalls substituiert sind, <u>oder</u> $R_5$ und $R_6$ bilden zusammen mit dem Stickstoffatom, an das sie gebunden sind, einen Heterozyklus mit 5 bis 6 Kettengliedern, enthaltend gegebenenfalls ein zweites Heteroatom, ausgewählt unter Sauerstoff, Schwefel oder Stickstoff, gegebenenfalls substituiert am nicht an $R_5$ und $R_6$ gebundenen Stickstoff durch:
- einen linearen oder verzweigten Alkylrest mit höchstens 5 Kohlenstoffatomen, gegebenenfalls substituiert durch einen Hydroxyrest, ein Halogenatom oder einen Alkyloxyrest mit 1 bis 4 Kohlenstoffatomen,
- einen Arylrest oder einen Arylalkylrest mit 7 bis 12 Kohlenstoffatomen, wobei diese Reste gegebenenfalls substituiert sind, wobei diese Produkte der Formel (I) unter allen möglichen isomeren racemischen oder optisch aktiven Formen vorliegen, sowie die Additionssalze dieser Produkte der Formel (I) mit Mineral- oder organischen Säuren.

4. Produkte der Formel (I), wie sie in einem der Ansprüche 1 bis 3 definiert sind, dadurch gekennzeichnet, daß der oder die Substituent(en), welche(n) die Phenyl-, Aryl- und Arylalkylreste tragen kann/können, ausgewählt sind aus der Gruppe gebildet durch die Halogenatome, den Hydroxyrest, die linearen oder verzweigten Alkyl- und Alkyoxyreste mit höchstens 5 Kohlenstoffatomen, die Methylthio-, Nitro-, Amino-, Monoalkyl- und Dialkylaminoreste, wobei diese Produkte der Formel (I) in allen möglichen isomeren, racemischen oder optisch aktiven Formen vorliegen, sowie die Additionssalze dieser Produkte der Formel (I) mit Mineral- oder organischen Säuren.

**5.** [16α,(±)]-15-(1-Propinyl)-20,21-Dinoreburnameninoxalat, [16α,(±]-15-Methyl-20,21-dinoreburnamenin,
das saure Maleat von [16α,(±)]-14,15-dihydro-15-methylen-20,21-dinoreburnamenin,
das Maleat von [16α,(±)]-20,21-dinoreburnamenin-15-ethylacetat,
das saure Maleat von [16α,(±)]-20,21-dinoreburnamenin-15-methanol,
das saure Maleat von [16α,(±)]-N,N-Dimethyl-20,21-dinoreburnamenin-15-methanamin.

**6.** Verfahren zur Herstellung der Produkte der Formel (I), wie in Anspruch 1 definiert, dadurch gekennzeichnet, daß man ein Produkt der Formel (II)

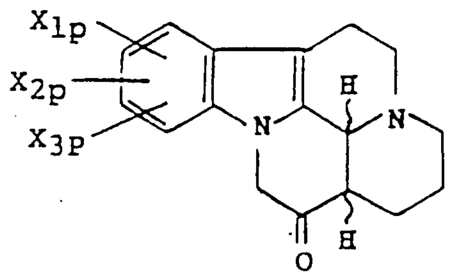

(II)

worin $X_{1p}$, $X_{2p}$ und $X_{3p}$ bedeuten $X_1$, $X_2$ und $X_3$, deren Bedeutungen in Anspruch 1 angegeben sind, worin die reaktionsfähigen Funktionen gegebenenfalls geschützt sind,

A) <u>entweder</u> mit einem Halogenid der Formel (III)

R'-V-Hal (III)

worin V ein Magnesium- oder Zinkatom darstellt und Hal ein Halogenatom bedeutet,
oder einer Organometallverbindung der Formel (IV)

R'-W (IV)

worin W ein Lithium-, Natrium- oder Kaliumatom darstellt, und in den Formeln (III) und (IV) R' entweder die in Anspruch 1 für R angegebenen Werte bedeutet, außer wenn der Wert Carboxy in freier, Salz- oder Esterform vorliegt, oder die genannten Werte von R, worin die reaktionsfähigen Funktionen geschützt sind, zur Reaktion bringt, um eine Verbindung der Formel (V)

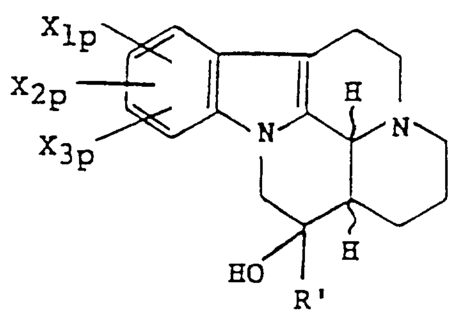

(V)

zu erhalten, worin R' die oben angegebene Bedeutung hat, die man einer Dehydratisierungsreaktion unterwirft, und falls notwendig einer Reaktion zur Entfernung der Schutzgruppen der reaktionsfähigen Funktionen, welche

$X_{1p}$, $X_{2p}$, $X_{3p}$ und R' tragen können, um eine Verbindung der Formel ($Ia_1$):

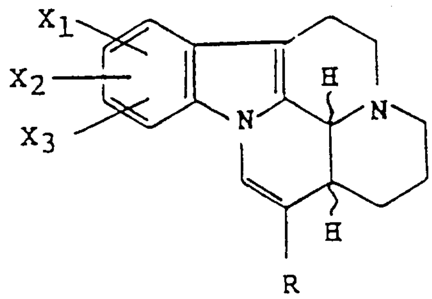

($Ia_1$)

zu erhalten, worin $X_1$, $X_2$, $X_3$ und R die oben angegebenen Bedeutungen haben, daß man das Produkt der Formel ($Ia_1$) gegebenenfalls, wenn R einen Rest $-CH_2OH$ darstellt, der Einwirkung eines Oxidationsmittels unterwirft, um ein Produkt der Formel ($Ia_2$)

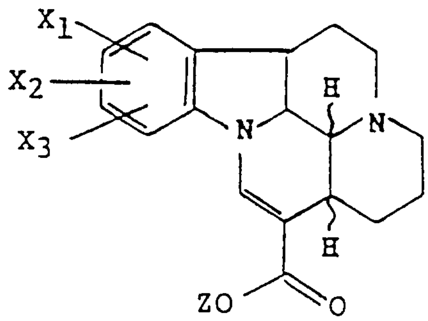

($Ia_2$)

zu erhalten, worin $X_1$, $X_2$ und $X_3$ die oben angegebenen Bedeutungen haben und Z ein Wasserstoffatom oder den Rest einer Estergruppe bedeutet das man falls notwendig oder gewünscht einer oder beiden der folgenden Reaktionen unterwirft:

- Hydrolyse der Estergruppe,
- Veresterung oder Salzbildung mit einer Base der Carboxylfunktion;

B) oder mit einem Derivat des Triphenylphosphorans der Formel (VI):

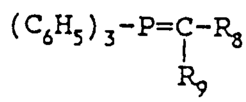

(VI)

oder einem Phosphonat der Formel (VI'):

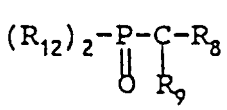

(VI')

umsetzt, worin $R_{12}$ einen Alkyloxyrest bedeutet, worin $R_8$ und $R_9$ in den Formeln (VI) und (VI') die in Anspruch 1 angegebenen Bedeutungen haben, um je nach den verwendeten Arbeitsbedingungen und gegebenenfalls nach Entfernung der Schutzgruppen der reaktionsfähigen Funktionen, welche $X_{1p}$, $X_{2p}$ und $X_{3p}$ tragen können,

eine Verbindung der Formel ($Ib_1$):

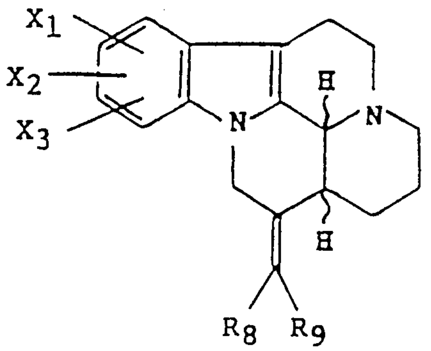

($Ib_1$)

oder eine Verbindung der Formel ($Ia_3$):

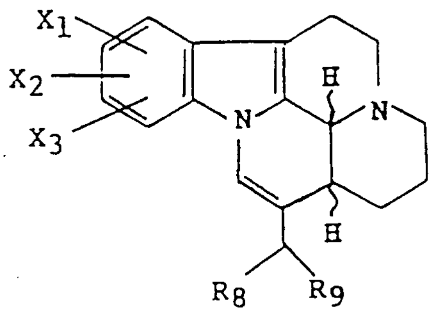

($Ia_3$)

zu ergeben, worin $X_1$, $X_2$, $X_3$, $R_8$ und $R_9$ die in Anspruch 1 angegebenen Bedeutungen haben,
C) <u>oder</u> mit einem aktivierten Derivat des Produkts der Formel (VII):

$$R_{10}\text{-C}\equiv\text{CH} \qquad \text{(VII)}$$

worin $R_{10}$ die in Anspruch 1 angegebene Bedeutung hat, um eine Verbindung der Formel (VIII):

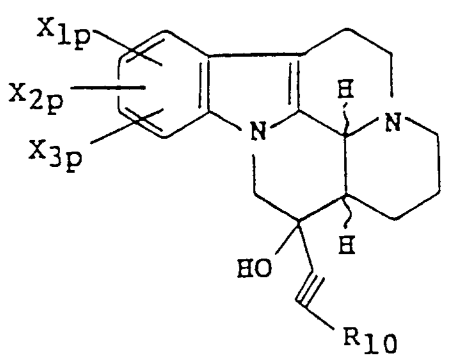

(VIII)

zu erhalten, worin $X_{1p}$, $X_{2p}$, $X_{3p}$ und $R_{10}$ die oben angegebenen Bedeutungen haben, welche man nach Aktivierung des Hydroxylrestes und gegebenenfalls Schutz der reaktiven Funktionen, welche $R_{10}$ tragen kann, einer Reduktionsreaktion, gegebenenfalls einer Entfernungsreaktion der Schutzgruppen der geschützten reaktiven Funktionen unterwirft, um eine Verbindung der Formel ($Ib_2$):

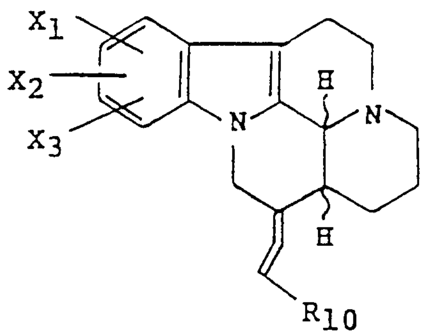

($Ib_2$)

zu erhalten, worin $X_1$, $X_2$, $X_3$ und $R_{10}$ die oben angegebenen Bedeutungen haben, und gewünschtenfalls die Produkte der Formeln ($Ia_1$), ($Ia_3$), ($Ib_1$) und ($Ib_2$) mit einer Mineralsäure oder organischen Säure behandelt, um das entsprechende Salz zu erhalten, wobei diese Produkte der Formel (I) unter allen möglichen isomeren, racemischen oder enantiomeren Formen vorliegen.

**7.** Verfahren zur Herstellung der Produkte der Formel (I), wie in Anspruch 1 definiert, und worin

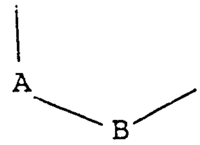

die Gruppe

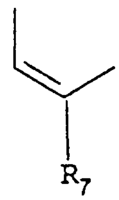

bedeutet, worin $R_7$ einen Methylrest darstellt, der gegebenenfalls durch einen Hydroxyrest, durch ein Halogenatom oder durch einen Rest

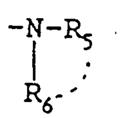

substituiert ist, wobei $R_5$ und $R_6$ die in Anspruch 3 angegebene Bedeutung haben, oder $R_7$ bedeutet einen Carbox-

yrest in freier Form, in Salzform oder verestert, dadurch gekennzeichnet, daß man ein Produkt der Formel (II):

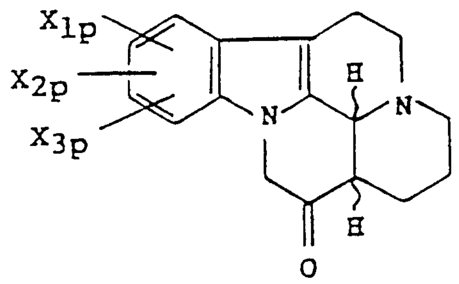

(II)

worin $X_{1p}$, $X_{2p}$ und $X_{3p}$ die in Anspruch 6 angegebene Bedeutung haben, mit einem halogenierten Methyloxosulfoniumderivat der Formel (IX):

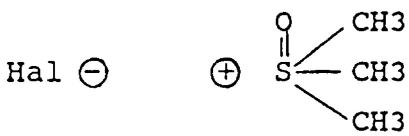

(IX)

in basischem Milieu zur Reaktion bringt, um das entsprechende Reaktionsepoxid der Formel (X)

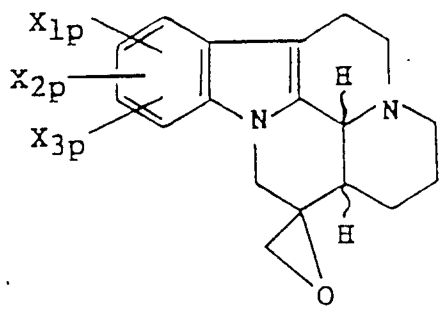

(X)

zu erhalten, worin $X_{1p}$, $X_{2p}$ und $X_{3p}$ die oben angegebenen Bedeutungen haben, das man behandelt:
entweder mit einer Base, um den entsprechenden Alkohol der Formel (X')

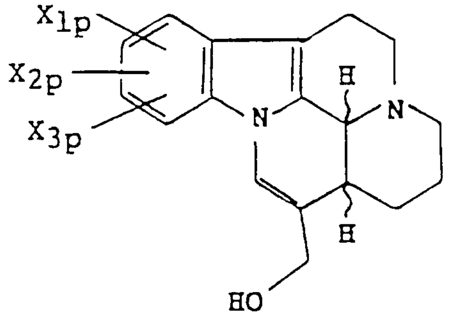

(X')

zu ergeben, worin $X_{1p}$, $X_{2p}$ und $X_{3p}$ die oben angegebenen Bedeutungen haben, und den man, falls notwendig, einer Reaktion zur Entfernung der Schutzgruppen der reaktiven Funktionen, welche $X_{1p}$, $X_{2p}$ und $X_{3p}$ tragen können,

unterwirft, um ein Produkt der Formel ($Ia_4$)

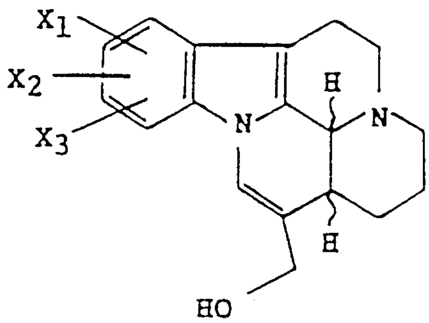

($Ia_4$)

zu ergeben, worin $X_1$, $X_2$ und $X_3$ die in Anspruch 1 angegebenen Bedeutungen haben, dann falls notwendig und gewünscht, das erhaltene Produkt ($Ia_4$) mit Hilfe eines Oxidationsmittels behandelt, um ein Produkt der Formel ($Ia_2$)

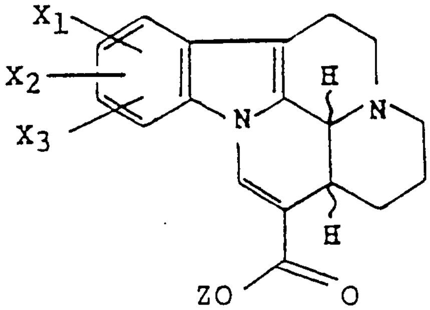

($Ia_2$)

zu erhalten, worin $X_1$, $X_2$, $X_3$ und Z die oben angegebenen Bedeutungen haben, das man, falls notwendig und gewünscht, einer oder beiden der folgenden Reaktionen unterwirft:

- Hydrolyse der Estergruppe,
- Veresterung oder Salzbildung der Carboxylfunktion mit einer Base, oder mit einem Amin der Formel (XI)

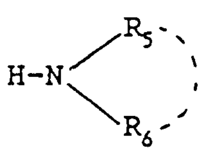

(XI)

worin $R_5$ und $R_6$ die in Anspruch 3 angegebenen Bedeutungen haben, um die Verbindung der Formel (XII)

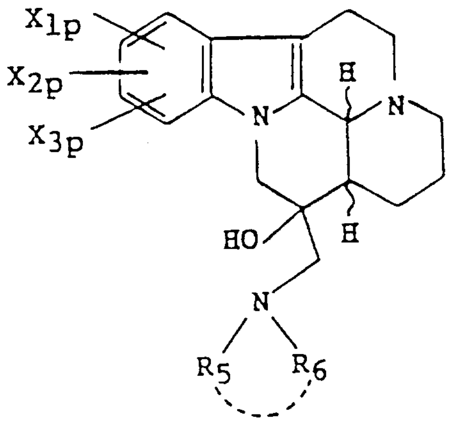

(XII)

zu erhalten, worin $X_{1p}$, $X_{2p}$ und $X_{3p}$ die oben angegebenen Bedeutungen haben, wobei diese Verbindungen der Formeln (X') und (XII) nach Aktivierung des Hydroxylrestes unterworfen werden:

- entweder falls gewünscht im Falle der Verbindung (X'), einer Reaktion mit dem Amin der Formel (XI), wie oben definiert,
- oder im Falle der Verbindung der Formel (XII), einer Dehydratisierungsreaktion, um in beiden Fällen und nach Entfernung, falls notwendig, der Schutzgruppen der reaktiven Funktionen, welche $X_{1p}$, $X_{2p}$ und $X_{3p}$ tragen können, eine Verbindung der Formel ($Ia_5$):

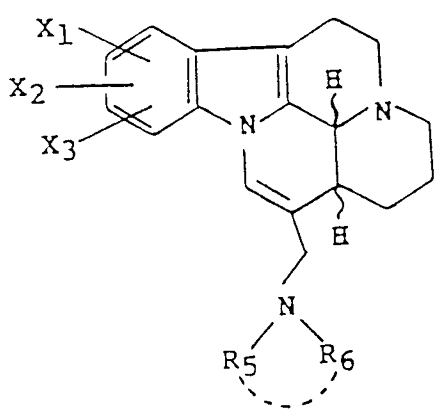

($Ia_5$)

zu erhalten, worin $X_1$, $X_2$, $X_3$, $R_5$ und $R_6$ die in Anspruch 3 angegebenen Bedeutungen haben,

oder aber mit einem Tetrabutylammoniumhalogenid umsetzt, um ein Produkt der Formel (XIII)

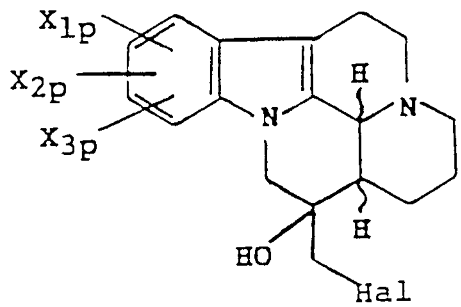

(XIII)

zu erhalten, worin Hal ein Halogenatom darstellt, dann einer Dehydratisierungsreaktion unterwirft, um nach Entfernung, falls notwendig, der Schutzgruppen der reaktiven Funktionen, welche $X_{1p}$, $X_{2p}$ und $X_{3p}$ tragen können, eine Verbindung der Formel ($Ia_6$)

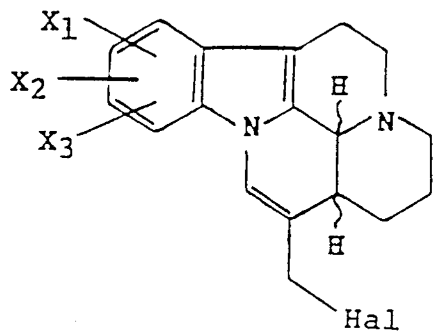

($Ia_6$)

zu erhalten, worin $X_1$, $X_2$, $X_3$ und Hal die oben angegebene Bedeutung haben, und falls gewünscht das Produkt der Formel ($Ia_3$), ($Ia_5$) und ($Ia_6$) mit einer Mineralsäure oder organischen Säure behandelt, um das entsprechende Salz zu erhalten, wobei die Produkte der Formel (I) in allen möglichen isomeren, racemischen oder enantiomeren Formen vorliegen.

**8.** Verfahren zur Herstellung der Produkte der Formel (I) wie in Anspruch 1 definiert, und worin

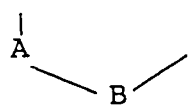

die Gruppe

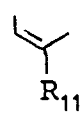

darstellt, worin $R_{11}$ den acetylenischen Rest der Formel

$$R_{10}\text{-}C\equiv C\text{-}$$

bedeutet, worin $R_{10}$ die in Anspruch 1 angegebene Bedeutung hat, dadurch gekennzeichnet, daß man ein Produkt der Formel (VIII)

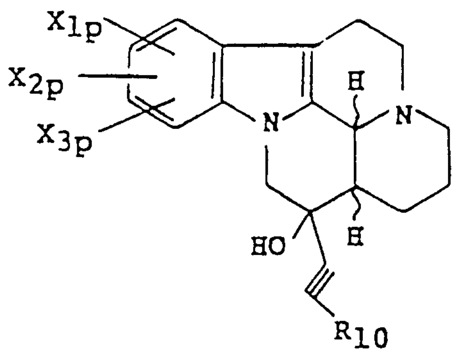

(VIII)

wie sie in Anspruch 6 definiert ist, worin $X_{1p}$, $X_{2p}$, $X_{3p}$ und $R_{10}$ die in Anspruch 6 angegebene Bedeutung haben, nach Aktivierung des Hydroxylrestes einer Dehydratisierungsreaktion unterwirft, um nach Entfernung, falls notwendig und gewünscht, der Schutzgruppen der reaktiven Funktionen, welche $X_{1p}$, $X_{2p}$ und $X_{3p}$ tragen können, eine Verbindung vom Enintyp der Formel ($Ia_7$)

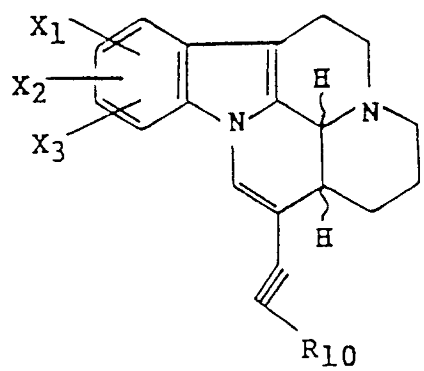

($Ia_7$)

zu erhalten, worin $X_1$, $X_2$, $X_3$ und $R_{10}$ die in Anspruch 1 angegebenen Bedeutungen haben, und falls gewünscht, das Produkt der Formel ($Ia_7$) mit einer Mineralsäure oder organischen Säure behandelt, wobei die genannten Produkte der Formel (I) unter allen möglichen isomeren racemischen oder enantiomeren Formen vorliegen.

**9.** Als Arzneimittel die Produkte, wie sie in der Formel (I) von Anspruch 1 definiert sind, wobei diese Produkte der Formel (I) unter allen möglichen isomeren, racemischen oder enantiomeren Formen vorliegen, sowie die Additionssalze mit pharmazeutisch annehmbaren Mineralsäuren oder organischen Säuren oder mit Basen.

**10.** Als Arzneimittel die Produkte gemäß Formel (I), wie sie in einem der Ansprüche 2, 3, 4 oder 5 definiert sind, sowie deren pharmazeutisch annehmbare Salze.

**11.** Die pharmazeutischen Zusammensetzungen, enthaltend als Wirkstoff mindestens eines der Arzneimittel, wie sie in einem der Ansprüche 9 und 10 definiert sind.

**12.** Als neue industrielle Produkte die Verbindungen der Formel (II")

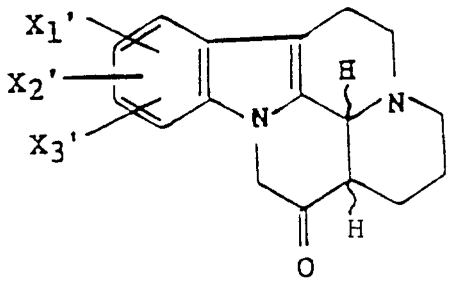

(II'')

worin $X_{1''}$, $X_{2''}$ und $X_{3''}$, die identisch oder verschieden sind, $X_1$, $X_2$ und $X_3$ darstellen, mit Ausnahme der Produkte, worin einer der Substituenten $X_{1''}$, $X_{2''}$ und $X_{3''}$ ein Wasserstoffatom bedeutet und die zwei anderen Reste, die identisch oder verschieden sind, ausgewählt sind unter den Wasserstoff- oder Halogenatomen, den Alkyl- oder Alkyloxyresten mit 1 bis 5 Kohlenstoffatomen, einem Hydroxy-, Trifluoromethyl- oder Nitrorest.

**13.** Als neue industrielle Produkte die Verbindungen der Formeln(V), (VIII), (X), (X'), (XII) und (XIII), wie sie in den Ansprüchen 6 und 7 definiert sind.

**Patentansprüche für folgenden Vertragsstaat : ES**

**1.** Verfahren zur Herstellung der Produkte der Formel (I)

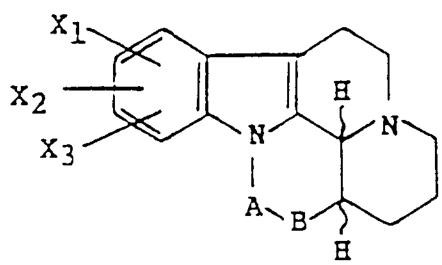

(I)

worin $X_1$, $X_2$ und $X_3$, die identisch oder verschieden sind, ein Wasserstoffatom, ein Halogenatom, einen Alkyl-, Alkenyl- oder Alkinylrest bedeuten, wobei diese Reste gerade oder verzweigt sind und höchstens 18 Kohlenstoffatome enthalten und gegebenenfalls substituiert sind, einen geraden oder verzweigten Alkyloxyrest mit höchstens 7 Kohlenstoffatomen, gegebenenfalls substituiert, einen Hydroxy-, Trifluoromethyl- oder Nitrorest, einen Amino-, Mono- oder Dialkylaminorest, oder einen gegebenenfalls substituierten Phenylrest, und worin

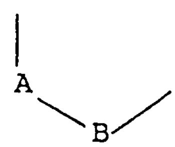

bedeutet:

A) entweder die Gruppe

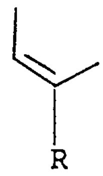

worin R bedeutet:

a) einen geraden oder verzweigten Alkyl-, Alkenyl- oder Alkinylrest mit höchstens 7 Kohlenstoffatomen, wobei dieser Rest gegebenenfalls substituiert ist,

b) einen gegebenenfalls substituierten Phenylrest,

c) eine Carboxylgruppe in freier Form, in Salzform oder verestert,

B) oder die Gruppe

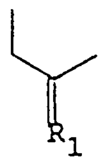

worin $R_1$ eine der beiden Gruppen

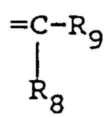

oder

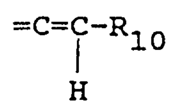

bedeutet, worin:
$R_8$ und $R_9$, die identisch oder verschieden sind, derart sind, daß:

- eines ausgewählt ist aus der Gruppe gebildet durch:

  a) ein Wasserstoffatom,

  b) einen linearen oder verzweigten Alkyl-, Alkenyl- oder Alkinylrest mit höchstens 6 Kohlenstoffatomen, wobei dieser Rest gegebenenfalls substituiert ist,

  c) einen gegebenenfalls substituierten Phenylrest,

  d) einen veresterten Carboxyrest,

  e) einen Cyanorest,

  f) einen Acylrest mit 2 bis 6 Kohlenstoffatomen,

- und der andere ausgewählt ist aus der Gruppe gebildet durch:

  a) ein Wasserstoffatom,

b) einen linearen oder verzweigten Alkyl-, Alkenyl- oder Alkinylrest mit höchstens 6 Kohlenstoffatomen, wobei dieser Rest gegebenenfalls substituiert ist,

c) einen gegebenenfalls substituierten Phenylrest,

und $R_{10}$ bedeutet:

a) ein Wasserstoffatom,

b) einen linearen oder verzweigten Alkyl-, Alkenyl- oder Alkinylrest mit höchstens 5 Kohlenstoffatomen, wobei dieser Rest gegebenenfalls substituiert ist,

c) einen gegebenenfalls substituierten Phenylrest,

wobei diese Produkte der Formel (I) unter allen möglichen isomeren Formen, racemisch oder optisch aktiv vorliegen, sowie die Additionssalze dieser Produkte der Formel (I) mit Mineral- oder organischen Säuren oder mit Basen, dadurch gekennzeichnet, daß man ein Produkt der Formel (II):

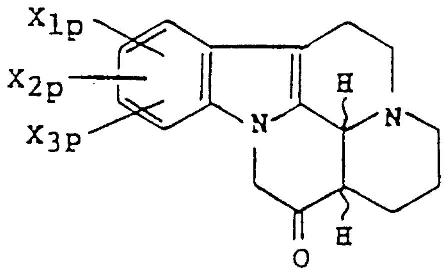

(II)

worin $X_{1p}$, $X_{2p}$ und $X_{3p}$ bedeuten $X_1$, $X_2$ und $X_3$, welche die in Anspruch 1 angegebenen Bedeutungen haben, worin die reaktionsfähigen Funktionen gegebenenfalls geschützt sind,
zur Reaktion bringt

A) <u>entweder</u> mit einem Halogenid der Formel (III)

R'-V-Hal (III)

worin V ein Magnesium- oder Zinkatom darstellt und Hal ein Halogenatom bedeutet, oder einer Organometallverbindung der Formel (IV)

R'-W (IV)

worin W ein Lithium-, Natrium- oder Kaliumatom bedeutet, wobei in den Formeln (III) und (IV) der Rest R' entweder die in Anspruch 1 für R angegebenen Werte bedeutet, außer wenn der Wert Carboxy in freier Form, Salz- oder Esterform vorliegt, oder die genannten Werte von R, worin die reaktiven Funktionen geschützt sind, um eine Verbindung der Formel (V)

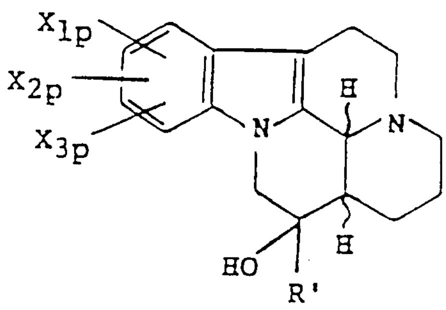

(V)

zu erhalten, worin R' die oben angegebene Bedeutung hat, die man einer Dehydratisierungsreaktion unterwirft, und falls notwendig einer Reaktion zur Entfernung der Schutzgruppen der reaktiven Funktionen, welche $X_{1p}$, $X_{2p}$, $X_{3p}$ und R' tragen können, um eine Verbindung der Formel ($Ia_1$):

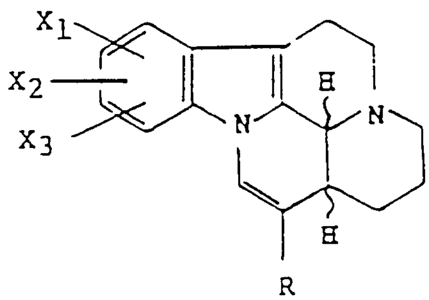

($Ia_1$)

zu erhalten, worin $X_1$, $X_2$, $X_3$ und R die oben angegebenen Bedeutungen haben, daß man das Produkt der Formel ($Ia_1$) gegebenenfalls, falls R einen Rest $-CH_2OH$ darstellt, der Einwirkung eines Oxidationsmittels unterwirft, um ein Produkt der Formel ($Ia_2$)

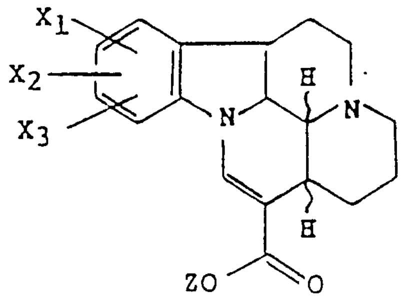

($Ia_2$)

zu erhalten, worin $X_1$, $X_2$ und $X_3$ die oben angegebenen Bedeutungen haben und Z ein Wasserstoffatom oder den Rest einer Estergruppe darstellt, das man falls notwendig oder erwünscht einer oder beiden der folgenden Reaktionen unterwirft:

- Hydrolyse der Estergruppe,
- Veresterung oder Salzbildung der Carboxylfunktion mit einer Base;

B) <u>oder</u> mit einem Derivat des Triphenylphosphorans der Formel (VI):

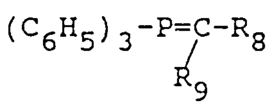

(VI)

oder einem Phosphonat der Formel (VI'):

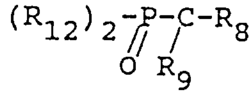

(VI')

umsetzt, worin $R_{12}$ einen Alkyloxyrest bedeutet, worin $R_8$ und $R_9$ in den Formeln (VI) und (VI') die oben angegebenen Bedeutungen haben, um je nach den angewandten Arbeitsbedingungen und gegebenenfalls nach Entfernung der Schutzgruppen der reaktiven Funktionen, welche $X_{1p}$, $X_{2p}$ und $X_{3p}$ tragen können, eine Verbindung

der Formel ($Ib_1$):

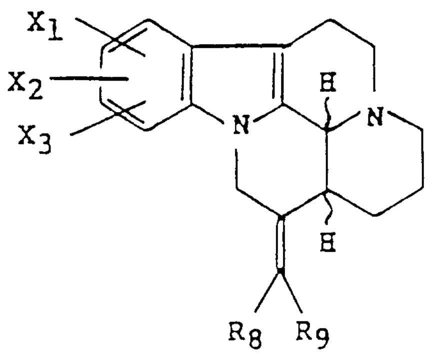

($Ib_1$)

oder eine Verbindung der Formel ($Ia_3$):

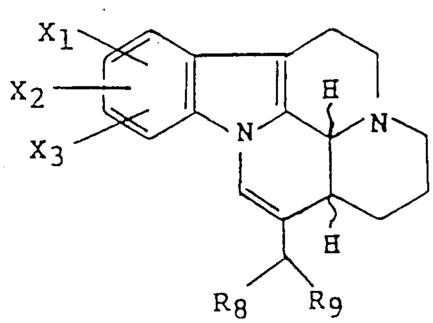

($Ia_3$)

zu ergeben, worin $X_1$, $X_2$, $X_3$, $R_8$ und $R_9$ die in Anspruch 1 angegebenen Bedeutungen haben,
C) <u>oder</u> mit einem aktivierten Derivat des Produkts der Formel (VII):

$$R_{10}\text{-C}\equiv\text{CH} \qquad \text{(VII)}$$

worin $R_{10}$ die in Anspruch 1 angegebene Bedeutung hat, um eine Verbindung der Formel (VIII):

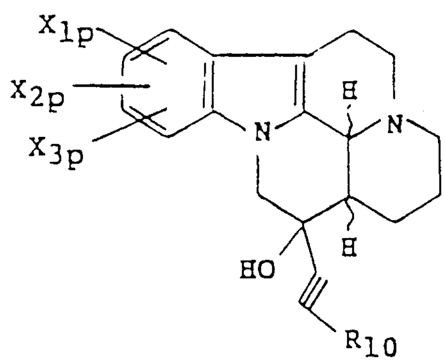

(VIII)

zu erhalten,worin $X_{1p}$, $X_{2p}$, $X_{3p}$ und $R_{10}$ die oben angegebenen Bedeutungen haben, die man nach Aktivierung des Hydroxylrestes und gegebenenfalls Schutz der reaktiven Funktionen, welche $R_{10}$ tragen kann, einer Reduktionsreaktion, gegebenenfalls einer Reaktion zur Entfernung der Schutzgruppen der geschützten reaktiven

Funktionen unterwirft, um eine Verbindung der Formel ($Ib_2$):

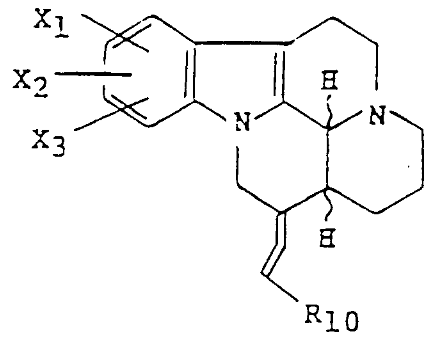

($Ib_2$)

zu erhalten, worin $X_1$, $X_2$, $X_3$ und $R_{10}$ die oben angegebenen Bedeutungen haben, und gewünschtenfalls die Produkte der Formeln ($Ia_1$), ($Ia_3$), ($Ib_1$) und ($Ib_2$) mit einer Mineralsäure oder organischen Säure behandelt, um das entsprechende Salz zu erhalten, wobei diese Produkte der Formel (I) unter allen möglichen isomeren, racemischen oder enantiomeren Formen vorliegen.

**2.** Verfahren zur Herstellung der Produkte der Formel (I), wie in Anspruch 1 definiert, und worin

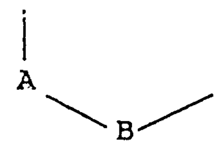

die Gruppe

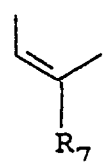

bedeutet, worin $R_7$ einen Methylrest, gegebenenfalls substituiert mit einem Hydroxyrest, mit einem Halogenatom oder durch einen Rest

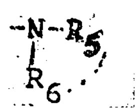

bedeutet, wobei $R_5$ und $R_6$, die identisch oder verschieden sind, bedeuten:

- ein Wasserstoffatom,
- einen geraden oder verzweigten Alkylrest mit höchstens 7 Kohlenstoffatomen, gegebenenfalls substituiert durch einen oder mehrere Rest(e), ausgewählt aus der Gruppe gebildet durch den Hydroxyrest, die geraden oder verzweigten Alkyloxyreste mit höchstens 5 Kohlenstoffatomen, und den Carboxyresten in freier Form oder verestert durch einen geraden oder verzweigten Alkylrest mit höchstens 5 Kohlenstoffatomen,
- einen Aryl- oder Arylalkylrest mit 7 bis 12 Kohlenstoffatomen, wobei diese Reste gegebenenfalls substituiert sind,
  <u>oder</u> $R_5$ und $R_6$ bilden zusammen mit dem Stickstoffatom, an das sie gebunden sind, einen Heterozyklus mit

5 oder 6 Kettengliedern, der gegebenenfalls ein zweites Heteroatom enthält, ausgewählt unter Sauerstoff, Schwefel oder Stickstoff, gegebenenfalls substituiert am nicht an $R_5$ und $R_6$ gebundenen Stickstoff durch:

- einen geraden oder verzweigten Alkylrest mit höchstens 5 Kohlenstoffatomen, gegebenenfalls substituiert durch einen Hydroxyrest, ein Halogenatom oder einen Alkyloxyrest mit 1 bis 4 Kohlenstoffatomen,

- einen Aryl- oder Arylalkylrest mit 7 bis 12 Kohlenstoffatomen, wobei diese Reste gegebenenfalls substituiert sind, oder $R_7$ bedeutet einen Carboxyrest in freier Form, in Salzform oder verestert, dadurch gekennzeichnet, daß man ein Produkt der Formel (II):

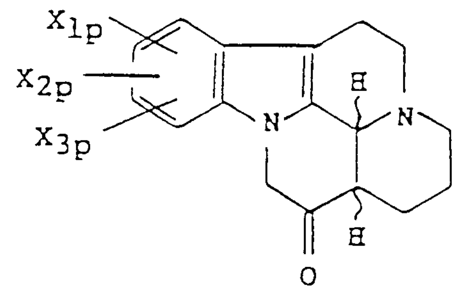

(II)

worin $X_{1p}$, $X_{2p}$ und $X_{3p}$, die in Anspruch 1 angegebene Bedeutung haben, mit einem halogenierten Methyloxosulfoniumderivat der Formel (IX):

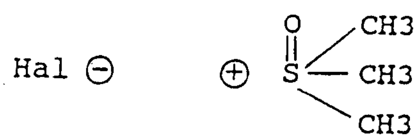

(IX)

in basischem Milieu zur Reaktion bringt, um das entsprechende Reaktionsepoxid der Formel (X)

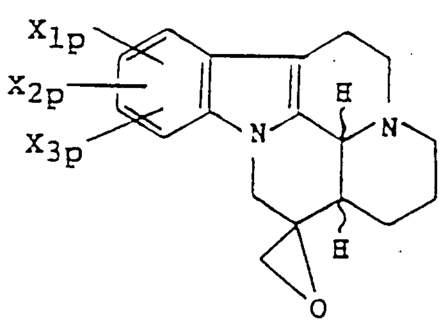

(X)

zu erhalten, worin $X_{1p}$, $X_{2p}$ und $X_{3p}$ die oben angegebenen Bedeutungen haben, das man behandelt:

entweder mit einer Base, um den entsprechenden Alkohol der Formel (X')

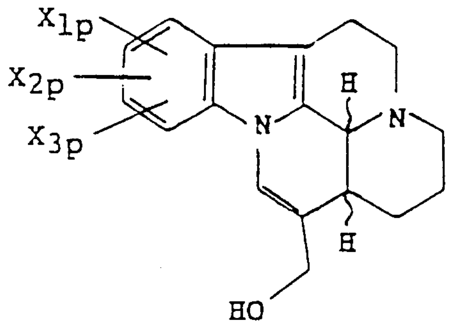

(X')

zu ergeben, worin $X_{1p}$, $X_{2p}$ und $X_{3p}$ die oben angegebenen Bedeutungen haben, und falls notwendig, den man einer Reaktion zur Entfernung der Schutzgruppen der reaktiven Funktionen, welche $X_{1p}$, $X_{2p}$ und $X_{3p}$ tragen können, unterwirft, um ein Produkt der Formel ($Ia_4$)

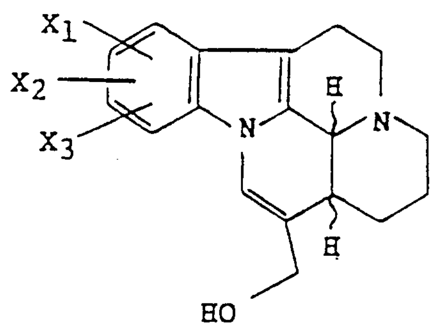

($Ia_4$)

zu ergeben, worin $X_1$, $X_2$ und $X_3$ die in Anspruch 1 angegebenen Bedeutungen haben, dann falls notwendig und gewünscht das erhaltene Produkt ($Ia_4$) mit Hilfe eines Oxidationsmittels behandelt, um ein Produkt der Formel ($Ia_2$)

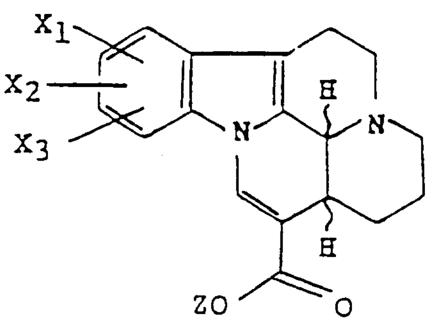

($Ia_2$)

zu erhalten, worin $X_1$, $X_2$, $X_3$ und Z die oben angegebenen Bedeutungen haben, das man, falls notwendig und gewünscht, einer oder beiden der folgenden beiden Reaktionen unterwirft:

- Hydrolyse der Estergruppe,
- Veresterung der Carboxylfunktion oder Salzbildung mit einer Base,

oder mit einem Amin der Formel (XI)

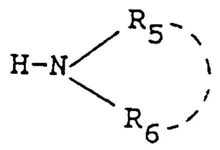

(XI)

worin $R_5$ und $R_6$ die oben angegebene Bedeutung haben, um die Verbindung der Formel (XII)

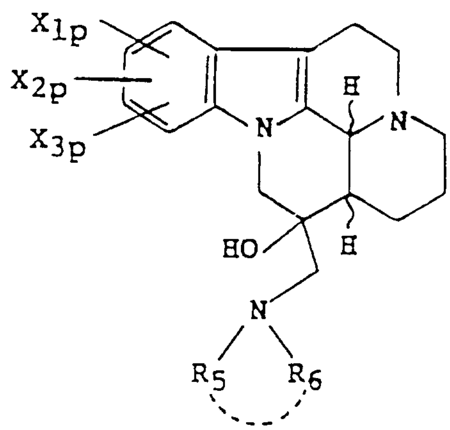

(XII)

zu erhalten, worin $X_{1p}$, $X_{2p}$ und $X_{3p}$ die oben angegebenen Bedeutungen haben, wobei diese Verbindungen der Formeln (X') und (XII) nach Aktivierung des Hydroxylrestes unterworfen werden:

- entweder falls gewünscht im Falle der Verbindung (X') einer Reaktion mit dem Amin der Formel (XI), wie oben definiert,
- oder im Falle der Verbindung der Formel (XII) einer Dehydratisierungsreaktion, um in beiden Fällen und falls notwendig nach Entfernung der Schutzgruppen der reaktiven Funktionen, welche $X_{1p}$, $X_{2p}$ und $X_{3p}$ tragen können, eine Verbindung der Formel ($Ia_5$):

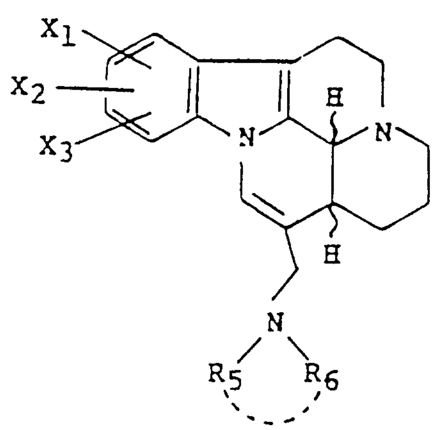

($Ia_5$)

zu erhalten, Worin $X_1$, $X_2$, $X_3$, $R_5$ und $R_6$ die oben angegebenen Bedeutungen haben,

- oder aber mit einem Tetrabutylammoniumhalogenid umsetzt, um ein Produkt der Formel (XIII)

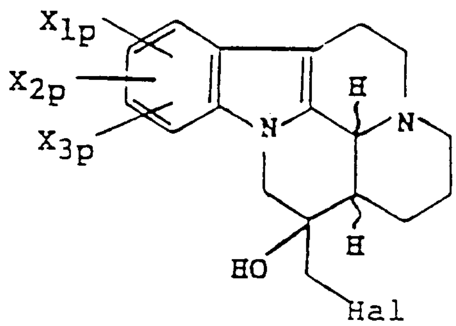

(XIII)

zu erhalten, worin Hal ein Halogenatom bedeutet, dann einer Dehydratisierungsreaktion unterwirft, um falls notwendig nach Entfernung der Schutzgruppen der reaktiven Funktionen, welche $X_{1p}$, $X_{2p}$ und $X_{3p}$ tragen können, eine Verbindung der Formel ($Ia_6$)

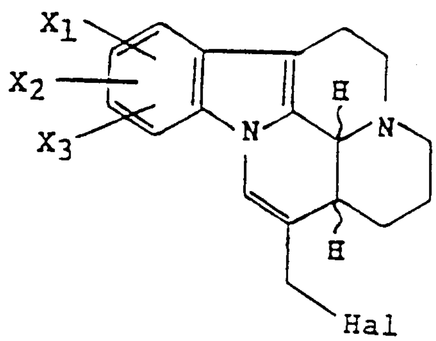

($Ia_6$)

zu erhalten, worin $X_1$, $X_2$, $X_3$ und Hal die oben angegebenen Bedeutungen haben, und falls gewünscht das Produkt der Formel ($Ia_3$), ($Ia_5$) und ($Ia_6$) mit einer Mineralsäure oder organischen Säure behandelt, um das entsprechende Salz zu erhalten, wobei diese Produkte der Formel (I) in allen möglichen isomeren Formen, racemischen oder enantiomeren, vorliegen.

**3.** Verfahren zur Herstellung der Produkte der Formel (I) wie in Anspruch 1 definiert, und worin

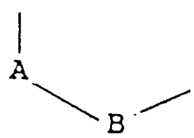

die Gruppe

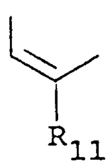

darstellt, worin $R_{11}$ den acetylenischen Rest der Formel

$R_{10}$-C≡C-

bedeutet, worin $R_{10}$ die in Anspruch 1 angegebene Bedeutung hat, dadurch gekennzeichnet, daß man ein Produkt der Formel (VIII)

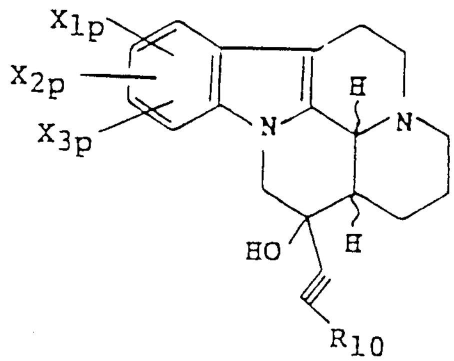

(VIII)

wie in Anspruch 1 definiert ist, worin $X_{1p}$, $X_{2p}$, $X_{3p}$ und $R_{10}$ die in Anspruch 1 angegebene Bedeutung haben, nach Aktivierung des Hydroxylrestes einer Dehydratisierungsreaktion unterwirft, um nach Entfernung, falls notwendig und gewünscht, der Schutzgruppen der reaktiven Funktionen, welche $X_{1p}$, $X_{2p}$ und $X_{3p}$ tragen können, eine Verbindung vom Enintyp der Formel ($Ia_7$)

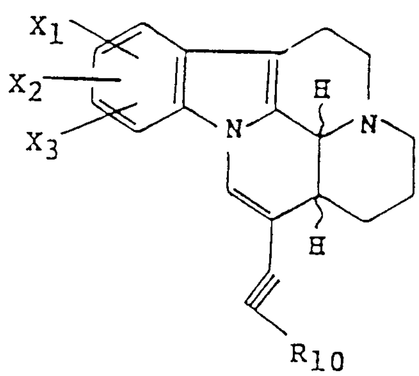

($Ia_7$)

zu erhalten, worin $X_1$, $X_2$, $X_3$ und $R_{10}$ die in Anspruch 1 angegebenen Bedeutungen haben, und falls gewünscht das Produkt der Formel ($Ia_7$) mit einer Mineralsäure oder organischen Säure behandelt, wobei diese Produkte der Formel (I) unter allen möglichen isomeren Formen, racemischen oder enantiomeren Formen, vorliegen.

4. Verfahren gemäß Anspruch 1 zur Herstellung der Produkte der Formel (I), wie in Anspruch 1 definiert, entsprechend der Formel ($I_A$):

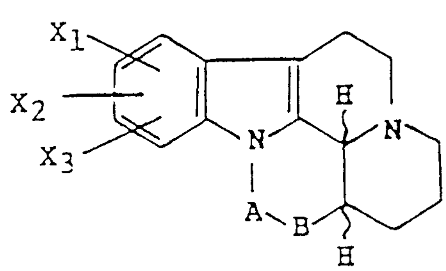

($I_A$)

worin $X_1$, $X_2$ und $X_3$ die in Anspruch 1 angegebene Bedeutung haben und

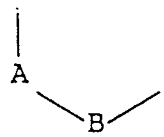

bedeutet entweder die Gruppe

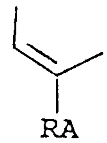

worin RA die in Anspruch 1 für R angegebene Bedeutung hat mit Ausnahme des Wertes Carboxyl in freier Form, in Salzform oder verestert, oder die Gruppe

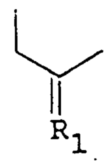

bedeutet, worin $R_1$ die in Anspruch 1 angegebene Bedeutung hat, dadurch gekennzeichnet, daß man Produkte der Formel ($Ia_1$), ($Ib_1$), ($Ib_2$), ($Ia_3$) gemäß dem in Anspruch 1 beschriebenen Verfahren herstellt.

5. Verfahren gemäß Anspruch 2 zur Herstellung der Produkte der Formel ($I_A$), wie in Anspruch 4 definiert, und worin RA einen Methylrest darstellt, der gegebenenfalls durch einen Hydroxyrest oder durch einen Rest

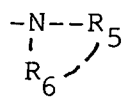

substituiert ist, worin $R_5$ und $R_6$ die in Anspruch 2 angegebene Bedeutung haben, dadurch gekennzeichnet, daß man Produkte der Formel ($Ia_4$) oder ($Ia_5$) nach dem in Anspruch 2 beschriebenen Verfahren herstellt.

6. Verfahren gemäß einem der Ansprüche 1 bis 5, dadurch gekennzeichnet, daß man zu Beginn ein Produkt der Formel (II) verwendet, worin $X_1$, $X_2$ und $X_3$ ein Wasserstoffatom bedeuten.

7. Verfahren gemäß einem der Ansprüche 1 bis 5, dadurch gekennzeichnet, daß man zu Beginn ein Produkt der Formel (II), (III), (IV), (VI), (VI') oder (VII) verwendet, worin der oder die Substituent(en), welche die Alkyl-, Alkyloxy-, Alkenyl- oder Alkinylreste tragen können, ausgewählt sind aus der Gruppe gebildet durch:

- den Hydroxyrest,
- die Halogenatome,
- den Alkyloxyrest mit 1 bis 6 Kohlenstoffatomen,
- den Formylrest und Acylreste mit 2 bis 6 Kohlenstoffatomen und dem Benzoylrest,
- den Carboxyresten in freier Form oder verestert mit einem linearen oder verzweigten Alkylrest mit höchstens 5 Kohlenstoffatomen,

- den Cyanorest,
- den Carbamoylrest, gegebenenfalls substituiert,
- den Phenylrest, gegebenenfalls substituiert,
- den Rest

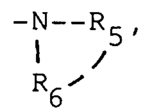

worin:
entweder $R_5$ und $R_6$, die identisch oder verschieden sind, bedeuten:

- ein Wasserstoffatom,
- einen geraden oder verzweigten Alkylrest mit höchstens 7 Kohlenstoffatomen, gegebenenfalls substituiert durch einen oder mehrere Rest(e), ausgewählt aus der Gruppe gebildet durch den Hydroxyrest, die geraden oder verzweigten Alkyloxyreste mit höchstens 5 Kohlenstoffatomen und den Carboxyresten in freier Form oder verestert durch einen geraden oder verzweigten Alkylrest mit höchstens 5 Kohlenstoffatomen,
- einen Aryl- oder Arylalkylrest mit 7 bis 12 Kohlenstoffatomen, wobei diese Reste gegebenenfalls substituiert sind,
oder $R_5$ und $R_6$ bilden zusammen mit dem Stickstoffatom, an das sie gebunden sind, einen Heterozyklus von 5 bis 6 Kettengliedern, der gegebenenfalls ein zweites Heteroatom enthält, ausgewählt aus Sauerstoff, Schwefel oder Stickstoff, gegebenenfalls substituiert am nicht an $R_5$ und $R_6$ gebundenen Stickstoff durch:
- einen geraden oder verzweigten Alkylrest mit höchstens 5 Kohlenstoffatomen, gegebenenfalls substituiert durch einen Hydroxyrest, ein Halogenatom oder einen Alkyloxyrest mit 1 bis 4 Kohlenstoffatomen,
- einen Aryl- oder Arylalkylrest mit 7 bis 12 Kohlenstoffatomen, wobei diese Reste gegebenenfalls substituiert sind.

8. Verfahren gemäß einem der Ansprüche 1 bis 5, dadurch gekennzeichnet, daß man zu Beginn ein Produkt der Formel (II), (III), (IV), (VI), (VI'), (VII) oder (XI) verwendet, worin der oder die Substituent(en), welche die Reste Phenyl, Aryl und Arylalkyl tragen können, ausgewählt sind aus der Gruppe gebildet durch die Halogenatome, den Hydroxyrest, die geraden oder verzweigten Alkyl- und Alkyloxyreste mit höchstens 5 Kohlenstoffatomen,den Resten Methylthio, Nitro, Amino, Monoalkyl- und Dialkylamino.

**Patentansprüche für folgenden Vertragsstaat : GR**

1. Verfahren zur Herstellung der Produkte der Formel (I)

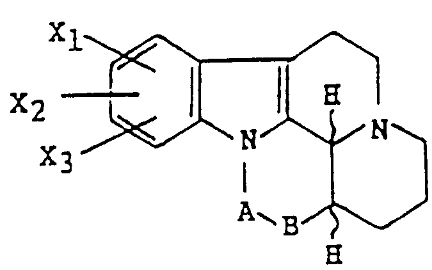

(I)

worin $X_1$, $X_2$ und $X_3$, die identisch oder verschieden sind, ein Wasserstoffatom, ein Halogenatom, einen Alkyl-, Alkenyl- oder Alkinylrest darstellen, wobei diese Reste gerade oder verzweigt sind und höchstens 18 Kohlenstoffatome enthalten und gegebenenfalls substituiert sind, einen geraden oder verzweigten Alkyloxyrest mit höchstens

7 Kohlenstoffatomen, gegebenenfalls substituiert, einen Hydroxy-, Trifluoromethyl- oder Nitrorest, einen Amino-, Mono- oder Dialkylaminorest, oder einen gegebenenfalls substituierten Phenylrest bedeuten, und worin

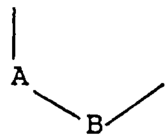

bedeutet:

A) entweder die Gruppe

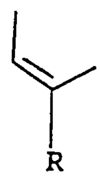

worin R bedeutet:

a) einen linearen oder verzweigten Alkyl-, Alkenyl- oder Alkinylrest mit höchstens 7 Kohlenstoffatomen, wobei dieser Rest gegebenenfalls substituiert ist,

b) einen gegebenenfalls substituierten Phenylrest,

c) eine Carboxylgruppe in freier Form, in Salzform oder verestert,

B) oder die Gruppe

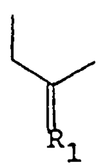

worin $R_1$ eine der beiden Gruppen

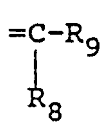

oder

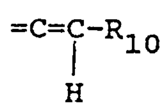

bedeutet, worin:
$R_8$ und $R_9$, die identisch oder verschieden sind, derart sind, daß:

- einer ausgewählt ist aus der Gruppe gebildet durch:

  a) ein Wasserstoffatom,

  b) einen linearen oder verzweigten Alkyl-, Alkenyl- oder Alkinylrest mit höchstens 6 Kohlenstoffatomen, wobei dieser Rest gegebenenfalls substituiert ist,

c) einen gegebenenfalls substituierten Phenylrest,

d) einen veresterten Carboxyrest,

e) einen Cyanorest,

f) einen Acylrest mit 2 bis 6 Kohlenstoffatomen,

- und der andere ausgewählt ist aus der Gruppe gebildet durch:

a) ein Wasserstoffatom,

b) einen linearen oder verzweigten Alkyl-, Alkenyl- oder Alkinylrest mit höchstens 6 Kohlenstoffatomen, wobei dieser Rest gegebenenfalls substituiert sein kann,

c) einen gegebenenfalls substituierten Phenylrest,

und $R_{10}$ bedeutet:

a) ein Wasserstoffatom,

b) einen linearen oder verzweigten Alkyl-, Alkenyl- oder Alkinylrest mit höchstens 5 Kohlenstoffatomen, wobei dieser Rest gegebenenfalls substituiert ist,

c) einen gegebenenfalls substituierten Phenylrest,

wobei diese Produkte der Formel (I) in allen möglichen racemischen oder optisch aktiven isomeren Formen vorliegen, sowie die Additionssalze dieser Produkte der Formel (I) mit Mineralsäuren oder organischen Säuren oder mit Basen, dadurch gekennzeichnet, daß man ein Produkt der Formel (II):

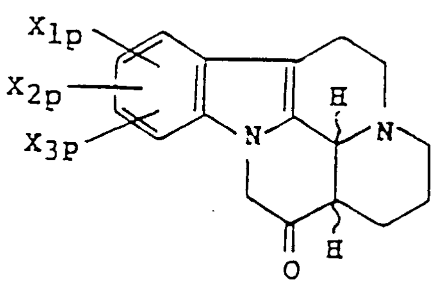

(II)

worin $X_{1p}$, $X_{2p}$ und $X_{3p}$ bedeuten $X_1$, $X_2$ und $X_3$, welche die in Anspruch 1 angegebenen Bedeutungen haben, worin die reaktiven Funktionen gegebenenfalls geschützt sind, zur Reaktion bringt

A) entweder mit einem Halogenid der Formel (III)

R'-V-Hal (III)

worin V ein Magnesium- oder Zinkatom darstellt und Hal ein Halogenatom bedeutet, oder einer metallorganischen Verbindung der Formel (IV)

R'-W (IV)

worin W ein Lithium-, Natrium- oder Kaliumatom bedeutet, wobei in den Formeln (III) und (IV) der Rest R' entweder die in Anspruch 1 für R angegebenen Bedeutungen hat, außer den Wert Carboxy in freier Form, in Salzform oder verestert, oder die genannten Werte von R, worin die reaktiven Funktionen geschützt sind, um

eine Verbindung der Formel (V)

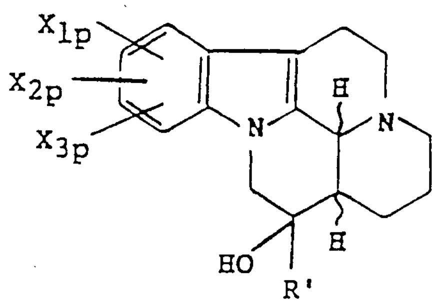

(V)

zu erhalten, worin R' die oben angegebene Bedeutung hat, die man einer Dehydratisierungsreaktion unterwirft, und falls notwendig einer Reaktion zur Entfernung der Schutzgruppen der reaktiven Funktionen, welche $X_{1p}$, $X_{2p}$, $X_{3p}$ und R' tragen können, um eine Verbindung der Formel ($Ia_1$):

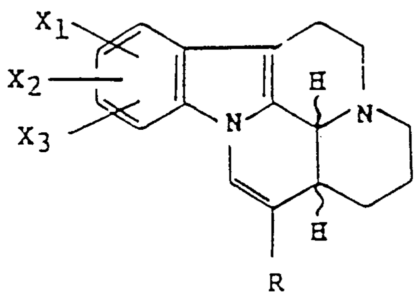

($Ia_1$)

zu erhalten, worin $X_1$, $X_2$, $X_3$ und R die oben angegebenen Bedeutungen haben, wobei man das Produkt der Formel ($Ia_1$) gegebenenfalls, wenn R einen Rest $-CH_2OH$ darstellt, der Einwirkung eines Oxidationsmittels unterwirft, um ein Produkt der Formel ($Ia_2$)

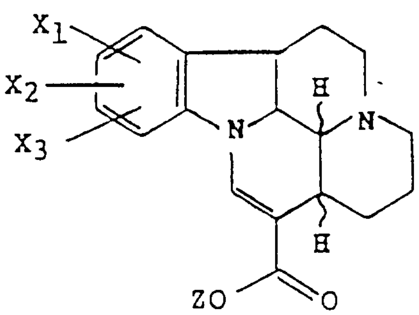

($Ia_2$)

zu erhalten, worin $X_1$, $X_2$ und $X_3$ die oben angegebenen Bedeutungen haben und Z ein Wasserstoffatom oder den Rest einer Estergruppe darstellt, das man falls notwendig oder erwünscht, einer oder beiden der folgenden Reaktion unterwirft:

- Hydrolyse der Estergruppe,
- Veresterung der Carboxylfunktion oder Salzbildung mit einer Base;

B) <u>oder</u> mit einem Triphenylphosphoranderivat der Formel (VI):

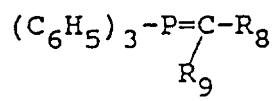

(VI)

oder einem Phosphonat der Formel (VI'):

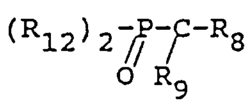

(VI')

umsetzt, worin $R_{12}$ einen Alkyloxyrest bedeutet, worin $R_8$ und $R_9$ in den Formeln (VI) und (VI') die oben angegebenen Bedeutungen haben, um gemäß den angewandten Reaktionsbedingungen und gegebenenfalls nach Entfernung der Schutzgruppen der reaktiven Funktionen, welche $X_{1p}$, $X_{2p}$ und $X_{3p}$ tragen können, eine Verbindung der Formel ($Ib_1$):

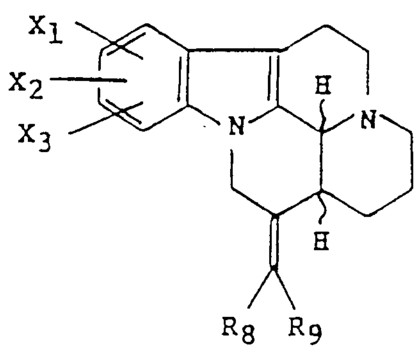

($Ib_1$)

oder eine Verbindung der Formel ($Ia_3$):

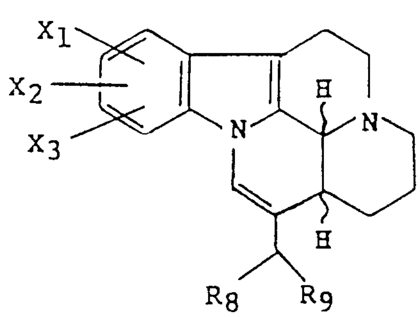

($Ia_3$)

zu ergeben, worin $X_1$, $X_2$, $X_3$, $R_8$ und $R_9$ die in Anspruch 1 angegebenen Bedeutungen haben,

C) <u>oder</u> mit einem aktivierten Derivat des Produkts der Formel (VII):

$$R_{10}\text{-}C{\equiv}CH \qquad \text{(VII)}$$

worin $R_{10}$ die in Anspruch 1 angegebene Bedeutung hat, um eine Verbindung der Formel (VIII):

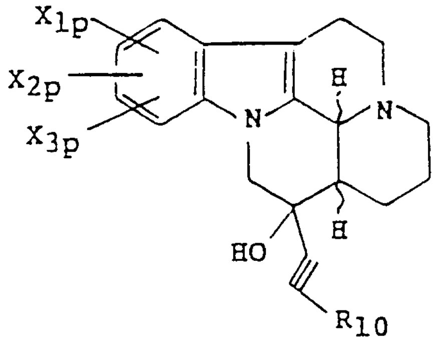

(VIII)

zu ergeben, worin $X_{1p}$, $X_{2p}$, $X_{3p}$ und $R_{10}$ die oben angegebenen Bedeutungen haben, die man nach Aktivierung des Hydroxylrestes und gegebenenfalls Schutz der reaktiven Funktionen, welche $R_{10}$ tragen kann, einer Reduktionsreaktion, gegebenenfalls einer Reaktion zur Entfernung der Schutzgruppen der geschützten reaktiven Funktionen unterwirft, um eine Verbindung der Formel ($Ib_2$):

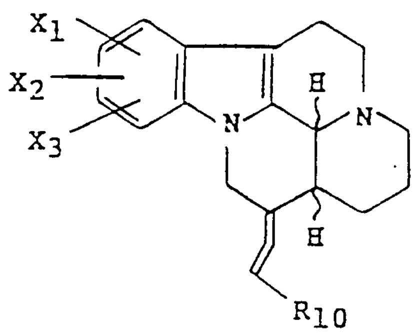

($Ib_2$)

zu erhalten, worin $X_1$, $X_2$, $X_3$ und $R_{10}$ die oben angegebenen Bedeutungen haben, und gewünschtenfalls die Produkte der Formeln($Ia_1$), ($Ia_3$), ($Ib_1$) und ($Ib_2$) mit einer Mineralsäure oder organischen Säure behandelt, um das entsprechende Salz zu erhalten, wobei diese Produkte der Formel (I) in allen möglichen isomeren, racemischen oder enantiomeren Formen vorliegen.

**2.** Verfahren zur Herstellung der Produkte der Formel (I), wie in Anspruch 1 definiert, und worin

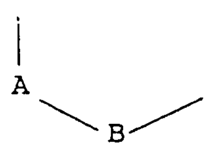

die Gruppe

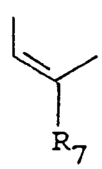

bedeutet, worin $R_7$ einen Methylrest, gegebenenfalls substituiert mit einem Hydroxyrest, durch ein Halogenatom

oder durch einen Rest

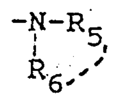

bedeutet, wobei $R_5$ und $R_6$, die identisch oder verschieden sind, bedeuten:

- ein Wasserstoffatom,
- einen linearen oder verzweigten Alkylrest mit höchstens 7 Kohlenstoffatomen, gegebenenfalls substituiert durch einen oder mehrere Rest(e), ausgewählt aus der Gruppe gebildet durch den Hydroxyrest, die linearen oder verzweigten Alkyloxyreste mit höchstens 5 Kohlenstoffatomen, und den Carboxyresten in freier Form oder verestert durch einen geraden oder verzweigten Alkylrest mit höchstens 5 Kohlenstoffatomen,
- einen Aryl- oder Arylalkylrest mit 7 bis 12 Kohlenstoffatomen, wobei diese Reste gegebenenfalls substituiert sind,
  oder $R_5$ und $R_6$ bilden zusammen mit dem Stickstoffatom, an das sie gebunden sind, einen Heterozyklus mit 5 bis 6 Kettengliedern, der gegebenenfalls ein zweites Heteroatom enthält, ausgewählt unter Sauerstoff, Schwefel oder Stickstoff, gegebenenfalls substituiert am nicht an $R_5$ und $R_6$ gebundenen Stickstoff durch:
- einen linearen oder verzweigten Alkylrest mit höchstens 5 Kohlenstoffatomen, gegebenenfalls substituiert durch einen Hydroxyrest, ein Halogenatom oder einen Alkyloxyrest mit 1 bis 4 Kohlenstoffatomen,
- einen Aryl- oder Arylalkylrest mit 7 bis 12 Kohlenstoffatomen, wobei diese Reste gegebenenfalls substituiert sind, oder $R_7$ bedeutet einen Carboxyrest in freier Form, in Salzform oder verestert, dadurch gekennzeichnet, daß man ein Produkt der Formel (II):

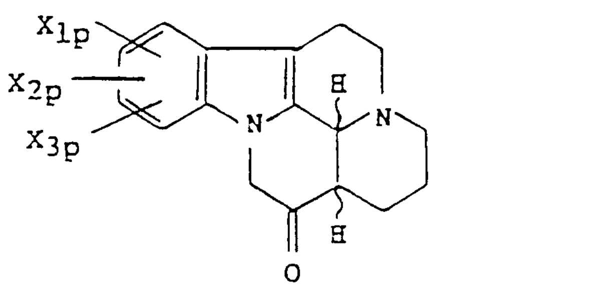

(II)

worin $X_{1p}$, $X_{2p}$ und $X_{3p}$, die in Anspruch 1 angegebene Bedeutung haben, mit einem halogenierten Methyloxosulfoniumderivat der Formel (IX):

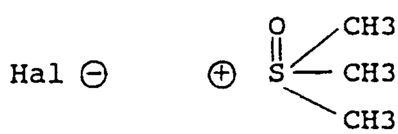

(IX)

in basischem Milieu zur Reaktion bringt, um das entsprechende Reaktionsepoxid der Formel (X)

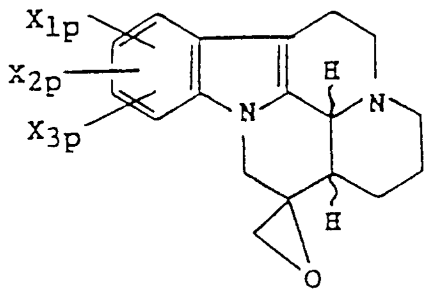

(X)

zu erhalten, worin $X_{1p}$, $X_{2p}$ und $X_{3p}$ die oben angegebenen Bedeutungen haben, das man behandelt:

<u>entweder</u> mit einer Base, um den entsprechenden Alkohol der Formel (X')

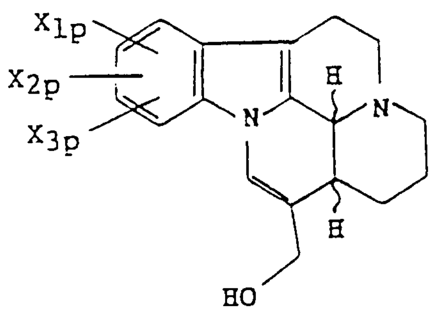

(X')

zu ergeben, worin $X_{1p}$, $X_{2p}$ und $X_{3p}$ die oben angegebenen Bedeutungen haben, und falls notwendig, den man einer Reaktion zur Entfernung der Schutzgruppen der reaktiven Funktionen, welche $X_{1p}$, $X_{2p}$ und $X_{3p}$ tragen können, unterwirft, um ein Produkt der Formel ($Ia_4$)

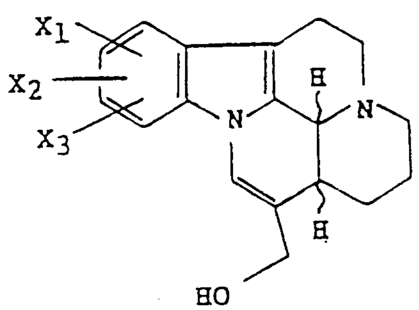

($Ia_4$)

zu ergeben, worin $X_1$, $X_2$ und $X_3$ die in Anspruch 1 angegebenen Bedeutungen haben, dann falls notwendig und

erwünscht das erhaltene Produkt ($Ia_4$) mit Hilfe eines Oxidationsmittels behandelt, um ein Produkt der Formel ($Ia_2$)

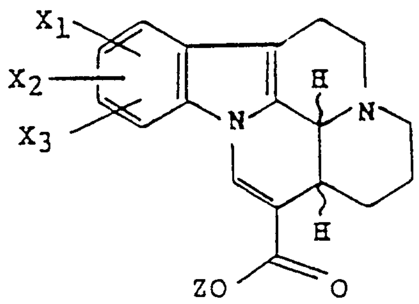

($Ia_2$)

zu erhalten, worin $X_1$, $X_2$, $X_3$ und Z die oben angegebenen Bedeutungen haben, das man, falls notwendig und erwünscht, einer oder beiden der folgenden beiden Reaktionen unterwirft:

- Hydrolyse der Estergruppe,
- Veresterung der Carboxylfunktion oder Salzbildung mit einer Base,

oder mit einem Amin der Formel (XI)

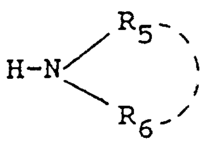

(XI)

worin $R_5$ und $R_6$ die oben angegebenen Bedeutungen haben, um die Verbindung der Formel (XII)

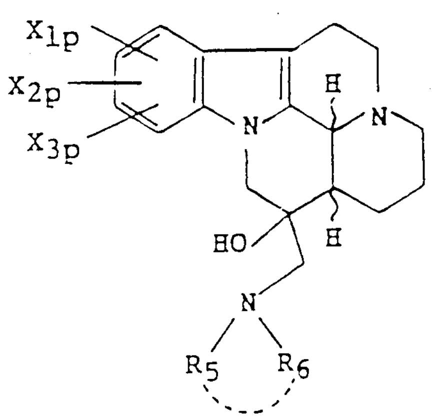

(XII)

zu erhalten, worin $X_{1p}$, $X_{2p}$ und $X_{3p}$ die oben angegebenen Bedeutungen haben, wobei diese Verbindungen der Formeln (X') und (XII) nach Aktivierung des Hydroxylrestes unterworfen werden:

- entweder falls gewünscht im Falle der Verbindung (X') einer Reaktion mit dem Amin der Formel (XI), wie oben definiert,
- oder im Falle der Verbindung der Formel (XII) einer Dehydratisierungsreaktion, um in beiden Fällen und falls notwendig nach Entfernung der Schutzgruppen der reaktiven Funktionen, welche $X_{1p}$, $X_{2p}$ und $X_{3p}$ tragen kön-

nen, eine Verbindung der Formel ($Ia_5$):

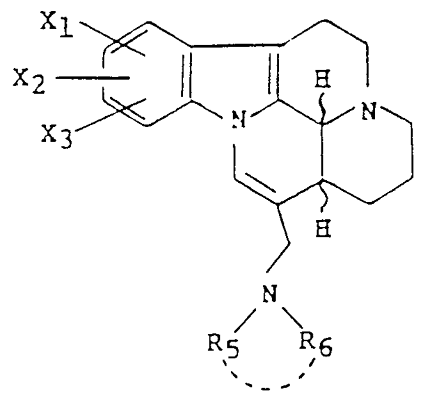

($Ia_5$)

zu erhalten, worin $X_1$, $X_2$, $X_3$, $R_5$ und $R_6$ die oben angegebenen Bedeutungen haben,

- oder mit einem Tetrabutylammoniumhalogenid umsetzt, um ein Produkt der Formel (XIII)

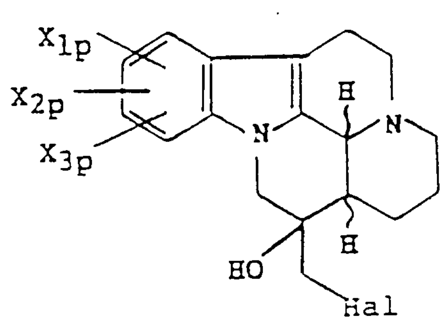

(XIII)

zu erhalten, worin Hal ein Halogenatom bedeutet, dann einer Dehydratisierungsreaktion unterwirft, um falls notwendig nach Entfernung der Schutzgruppen der reaktiven Funktionen, welche $X_{1p}$, $X_{2p}$ und $X_{3p}$ tragen können, eine Verbindung der Formel ($Ia_6$)

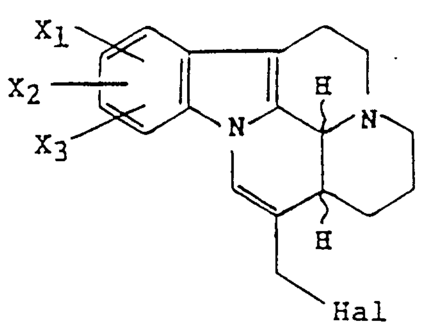

($Ia_6$)

zu erhalten, worin $X_1$, $X_2$, $X_3$ und Hal die oben angegebenen Bedeutungen haben, und falls erwünscht das Produkt der Formel ($Ia_3$), ($Ia_5$) und ($Ia_6$) mit einer Mineralsäure oder organischen Säure behandelt, um das entsprechende Salz zu erhalten, wobei diese Produkte der Formel (I) in allen möglichen isomeren Formen, racemisch oder enantiomer, vorliegen.

**3.** Verfahren zur Herstellung der Produkte der Formel (I) wie in Anspruch 1 definiert, und worin

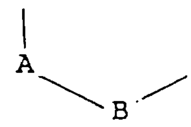

die Gruppe

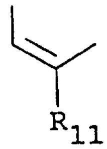

darstellt, worin $R_{11}$ den acetylenischen Rest der Formel

$$R_{10}\text{-C}\equiv\text{C-}$$

bedeutet, worin $R_{10}$ die in Anspruch 1 angegebene Bedeutung hat, dadurch gekennzeichnet, daß man ein Produkt der Formel (VIII)

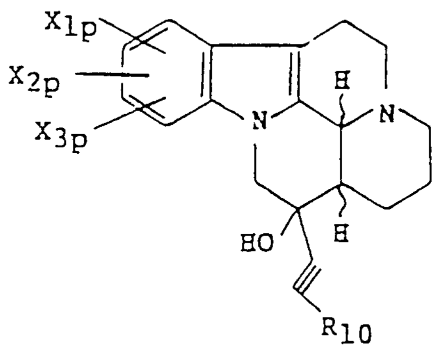

(VIII)

wie in Anspruch 1 definiert, worin $X_{1p}$, $X_{2p}$, $X_{3p}$ und $R_{10}$ die in Anspruch 1 angegebenen Bedeutungen haben, nach Aktivierung des Hydroxylrestes einer Dehydratisierungsreaktion unterwirft, um falls notwendig und gewünscht, nach Entfernung der Schutzgruppen der reaktiven Funktionen, welche $X_{1p}$, $X_{2p}$ und $X_{3p}$ tragen können, eine Verbindung vom Enintyp der Formel ($Ia_7$)

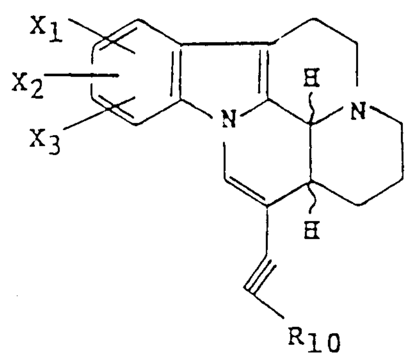

($Ia_7$)

zu erhalten, worin $X_1$, $X_2$, $X_3$ und $R_{10}$ die in Anspruch 1 angegebenen Bedeutungen haben, und falls gewünscht

das Produkt der Formel ($Ia_7$) mit einer Mineralsäure oder organischen Säure behandelt, wobei diese Produkte der Formel (I) in allen möglichen isomeren Formen, racemisch oder enantiomer, vorliegen können.

4. Verfahren gemäß Anspruch 1 zur Herstellung der Produkte der Formel (I), wie in Anspruch 1 definiert, entsprechend der Formel ($I_A$):

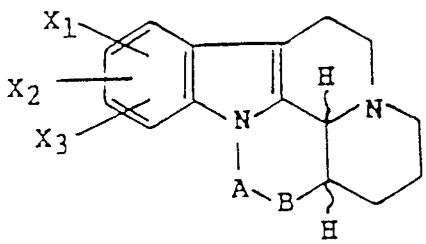

($I_A$)

worin $X_1$, $X_2$ und $X_3$ die in Anspruch 1 angegebene Bedeutung haben und

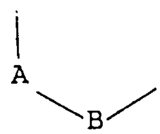

bedeutet entweder die Gruppe

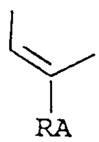

worin RA die in Anspruch 1 für R angegebene Bedeutung hat, mit der Ausnahme des Wertes Carboxyl in freier Form, in Salzform oder verestert, oder die Gruppe

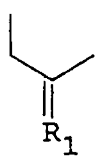

bedeutet, worin $R_1$ die in Anspruch 1 angegebene Bedeutung hat, dadurch gekennzeichnet, daß man Produkte der Formel ($Ia_1$), ($Ib_1$), ($Ib_2$), ($Ia_3$) gemäß dem in Anspruch 1 beschriebenen Verfahren herstellt.

5. Verfahren gemäß Anspruch 2 zur Herstellung der Produkte der Formel ($I_A$), wie in Anspruch 4 definiert, und worin RA einen Methylrest darstellt, gegebenenfalls substituiert durch einen Hydroxyrest oder durch einen Rest

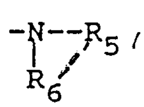

worin $R_5$ und $R_6$ die in Anspruch 2 angegebenen Bedeutungen haben, dadurch gekennzeichnet, daß man Produkte der Formel ($Ia_4$) oder ($Ia_5$) nach dem in Anspruch 2 beschriebenen Verfahren herstellt.

6. Verfahren gemäß einem der Ansprüche 1 bis 5, dadurch gekennzeichnet, daß man zu Beginn ein Produkt der Formel (II) verwendet, worin $X_1$, $X_2$ und $X_3$ ein Wasserstoffatom bedeuten.

7. Verfahren gemäß einem der Ansprüche 1 bis 5, dadurch gekennzeichnet, daß man zu Beginn ein Produkt der Formel (II), (III), (IV), (VI), (VI') oder (VII) verwendet, worin der oder die Substituent(en), welche die Alkyl-, Alkyloxy-, Alkenyl- oder Alkinylreste tragen können, ausgewählt sind aus der Gruppe gebildet durch:

- den Hydroxyrest,
- die Halogenatome,
- die Alkyloxyreste mit 1 bis 6 Kohlenstoffatomen,
- die Formyl- und Acylreste mit 2 bis 6 Kohlenstoffatomen und dem Benzoylrest,
- die Carboxyreste in freier Form oder verestert mit einem linearen oder verzweigten Alkylrest mit höchstens 5 Kohlenstoffatomen,
- den Cyanorest,
- den gegebenenfalls substituierten Carbamoylrest,
- den gegebenenfalls substituierten Phenylrest,
- den Rest

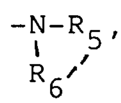

worin:
entweder $R_5$ und $R_6$, die identisch oder verschieden sind, bedeuten:

- ein Wasserstoffatom,
- einen linearen oder verzweigten Alkylrest mit höchstens 7 Kohlenstoffatomen, gegebenenfalls substituiert durch einen oder mehrere Rest(e), ausgewählt aus der Gruppe gebildet durch den Hydroxyrest, die linearen oder verzweigten Alkyloxyreste mit höchstens 5 Kohlenstoffatomen und die freien Carboxyreste oder verestert durch einen linearen oder verzweigten Alkylrest mit höchstens 5 Kohlenstoffatomen,
- einen Aryl- oder Arylalkylrest mit 7 bis 12 Kohlenstoffatomen, wobei diese Reste gegebenenfalls substituiert sind,
oder $R_5$ und $R_6$ bilden zusammen mit dem Stickstoffatom, an das sie gebunden sind, einen Heterozyklus von 5 bis 6 Kettengliedern, der gegebenenfalls ein zweites Heteroatom enthält, ausgewählt aus Sauerstoff, Schwefel oder Stickstoff, gegebenenfalls substituiert am nicht an $R_5$ und $R_6$ gebundenen Stickstoff durch:
- einen linearen oder verzweigten Alkylrest mit höchstens 5 Kohlenstoffatomen, gegebenenfalls substituiert durch einen Hydroxyrest, ein Halogenatom oder einen Alkyloxyrest mit 1 bis 4 Kohlenstoffatomen,
- einen Aryl- oder Arylalkylrest mit 7 bis 12 Kohlenstoffatomen, wobei diese Reste gegebenenfalls substituiert sind.

8. Verfahren gemäß einem der Ansprüche 1 bis 5, dadurch gekennzeichnet, daß man zu Beginn ein Produkt der Formel (II), (III), (IV), (VI), (VI'), (VII) oder (XI) verwendet, worin der oder die Substituent(en), welche die Reste Phenyl, Aryl und Arylalkyl tragen können, ausgewählt sind aus der Gruppe gebildet durch die Halogenatome, den Hydroxyrest, die linearen oder verzweigten Alkyl- und Alkyloxyreste mit höchstens 5 Kohlenstoffatomen,die Methylthio-, Nitro-, Amino-, Monoalkyl- und Dialkylaminoreste.

9. Verfahren gemäß Anspruch 4, dadurch gekennzeichnet, daß man eines der Produkte der Formel (I) mit den folgenden Bezeichnungen herstellt:

[16α,(±)]-15-(1-Propinyl)-20,21-Dinoreburnameninoxalat,
[16α,(±)]-15-Methyl-20,21-dinoreburnamenin,
das saure Maleat von [16α,(±)]-14,15-dihydro-15-methylen-20,21-dinoreburnamenin,
das Maleat von [16α,(±)]-20,21-dinoreburnamenin-15-ethylacetat,
das saure Maleat von [16α,(±)]-20,21-dinoreburnamenin-15-methanol,
das saure Maleat von [16α,(±)]-N,N-Dimethyl-20,21-dinoreburnamenin-15-methanamin.

**10.** Verfahren zur Herstellung von pharmazeutischen Zusammensetzungen, dadurch gekennzeichnet, daß man als Wirkstoff mindestens eines der Produkte der Formel (I), wie in Anspruch 1 definiert, oder mindestens eines seiner Salze mit pharmazeutisch annehmbaren Säuren oder Basen in einer für diesen Verwendungszweck bestimmten Form verwendet.

**11.** Verfahren zur Herstellung von pharmazeutischen Zusammensetzungen, dadurch gekennzeichnet, daß man als Wirkstoff mindestens eines der Produkte der Formel ($I_A$), wie in Anspruch 4 definiert, oder mindestens eines seiner Salze mit pharmazeutisch annehmbaren Säuren oder Basen in einer für diesen Verwendungszweck bestimmten Form einsetzt.

**12.** Verfahren zur Herstellung von pharmazeutischen Zusammensetzungen, dadurch gekennzeichnet, daß man als Wirkstoff mindestens eines der Produkte der Formel (I), welche in Anspruch 9 genannt sind, oder gegebenenfalls mindestens eines seiner Salze mit pharmazeutisch annehmbaren Säuren oder Basen in einer für diesen Verwendungszweck bestimmten Form einsetzt.

**13.** Als neue industrielle Produkte die Verbindungen der Formeln (V), (VIII), (X), (X'), (XII) und (XIII), wie sie in den Ansprüchen 1 und 2 definiert sind.